# EUROPEAN PATENT APPLICATION

(11) **EP 4 019 037 A1**
(43) Date of publication of application: **29.06.2022**
(21) Application number: 22151267.6
(22) Date of filing: 29.03.2018
(51) Int. Cl.: A61K 38/21, A61K 31/7105, A61K 31/7115, C12N 15/67

(54) **PREVENTION AND TREATMENT OF NON-MELANOMA SKIN CANCER (NMSC)**

(30) Priority: 31.03.2017 EP 17164257; 06.11.2017 EP 17200162
(62) Divisional of application: 18712919.2
(71) Applicant: Accanis Biotech F&E GmbH & Co KG, 1030 Vienna (AT)
(72) Inventor: MANDLER, Markus, 1030 Wien (AT); SCHNEEBERGER, Achim, 1030 Wien (AT); SCHMIDT, Walter, 1030 Wien (AT); MATTNER, Frank, 1030 Wien (AT)
(74) Representative: SONN Patentanwälte OG

(57) **Abstract**

The invention discloses Interferon alpha (IFN-α) messenger-RNA (mRNA), wherein the mRNA has a 5' CAP region, a 5' untranslated region (5'-UTR), a coding region, a 3' untranslated region (3'-UTR) and a poly-adenosine tail (poly-A tail), for use in the prevention and treatment of non-melanoma skin cancer (NMSC) and kits for administrating this IFN-α mRNA to a human patient.

## Description

The present invention relates to compositions and methods for the prevention and treatment of non-melanoma skin cancer (NMSC).

Non-melanoma skin cancer (NMSC) is the most common form of cancer worldwide. NMSC includes all forms of skin cancer that do not start in melanocytes, most notably basal cell carcinoma (BCC) (around 75-80% of all NMSCs) and squamous cell carcinoma (SCC) (around 20-25% of all NMSCs).

Actinic Keratosis (AK) is an important NMSC entity. Historically, it was considered to be a precursor lesion of SCC. Nowadays, the scientific community understands it to be a carcinoma in situ that may progress to an invasive stage. In contrast to the other NMSCs, AK is highly abundant.

AK is a skin disease that typically develops on areas of chronic sun exposed skin. The change in the pathophysiological concept of AK, which is now considered a carcinoma in situ, is driven by the facts that at the level of cytology AK is indistinguishable from SCC, and at the level of molecular biology, it shares multiple similarities with SCC.

AK is classified histopathologically in 3 grades (AK I-III) based on the extent of atypical keratinocytes in the epidermis. Clinically, AKs typically presents as scaly patches on an erythematous base. Palpation reveals a sand paper-like texture. The surrounding skin may show signs of chronic sun damage including telangiectasias, dyschromia and elastosis. Dermoscopy supports both differential diagnosis (e.g., of pigmented lesions on the face) and clinical grading. The anatomical distribution (face, bald head, neck, back of the hand) further highlights the importance of chronic sun exposure as major risk factor for the development of AKs. Other risk factors include advanced age, male gender and fair skin type. Immunosuppression (e.g. transplant recipients) also confers a higher risk for AKs. UVB radiation can lead to direct DNA damage. As a result, the function of tumour suppressor proteins such as p53 is suppressed. In fact, dysregulation of the p53 pathway seems to play the most important role in the development of AK lesions and their further progression to SCC. Some evidence suggests infections with human papilloma viruses act as cofactors.

AKs may occur as single lesions (≤5) as multiple lesions (≥6) or multiple lesions (≥6) in the context of field cancerization (i.e. at least 6 AK lesions in one body region or field, and contiguous areas of chronic sun damage). The common classification recommends a fourth subgroup, immunosuppressed patients with AKs.

Data available on the natural history of AK indicate that the presence of AK without adequate treatment is a dynamic but chronic condition. The risk of progression from AK to invasive SCC is estimated at 10% for individual lesions. As it is currently impossible to predict which AK lesions will progress to SCC, it is important to intervene as soon as possible in all cases.

Current AK therapies can be divided into two main classes: lesion directed and field directed. The most common lesion directed therapy is cryosurgery followed by surgical removal. The major drawback of both lesion-directed methods is that they are only able to address visible lesions but not the damaged field. Moreover, their cosmetic results are judged to be inferior to those of field-directed treatments. For these reasons, field-directed therapies are increasingly being used to eliminate not only clinically visible lesions, but also subclinical lesions to prevent their transition to SCC.

Current field directed therapies include topical agents such as Imiquimod (sold as Aldara^{®}, Zyclara^{®}), Diclofenac (sold as Solaraze^{®}), 5-fluorouracil (sold as Efudix^{®} or Actikerall^{®}), ingenol mebutate (sold as Picato^{®}) and photodynamic therapy (Ameluz^{®}, Metvix^{®}). Field therapies have the potential to affect subclinical lesions.

These current state of the art treatments often require an extensive treatment period with frequent home applications and a substantial down time. This is decreasing patient compliance severely and limiting efficacy of NMSC treatments in clinical practice. Additionally, responder rates to existing treatments vary, with histological clearance rates of AK following standard treatment reported as low as 67% following fluorouracil and 73% following Imiquimod. Information on the long-term effectiveness and recurrence rates of field-directed therapies is scarce and unimpressive. Recurrence rates at the 1-year time point range from 10 to 56%.

These unimpressively low success rates warrant the development of novel treatment paradigms to meet the medical need in the treatment of NMSCs.

Interferon alpha (Interferon α, IFN-α, IFNa), a type I interferon, exhibits antiviral, antiangiogenic as well as pro-apoptotic properties and is a key immunostimulatory molecule with proven therapeutic anti-tumoural efficacy. Interferons were first described in 1957. Cloning of genes for IFNa, and later, Interferon beta (IFN-β; IFNb) and Interferon gamma (IFN-γ; IFNg) was only reported in the early 1980s.

In the 1980s, recombinant IFNa protein has been suggested for the treatment of AKs and other forms of NMSC. At that time, multiple reports claimed the safety and efficacy of recombinant IFNa protein for the treatment of BCC and SCC. Repeated perilesional administration at a dose of 1.5×10⁶ IU IFNa 3× a week for 3 weeks was found adequate for most BCCs and SCCs (large tumours required higher total doses).

US 5 002 764 A and US 5 028 422 A disclose an intralesional administration treatment of AKs, with recombinant human IFNa-2 protein.

Various BCC subtypes appear to respond differently with superficial and ulcerative types being more amenable to IFNa therapy than nodular types. Intralesional administration of recombinant human IFNa-2b three times over a period of three weeks for the treatment of human BCC is also discussed in EP 0 248 583 B1.

SCC is generally more aggressive than BCC and potentially life-threatening. The overall 5-year recurrence rate of primary skin SCC is 8% (compared to BCC <0.1%).

There are a few reports on studies assessing intralesional IFNa in squamous cell carcinomas.

Frequent and long lasting application of high dose intralesional IFNa is effective in the treatment of small squamous cell carcinomas. For AK, Edwards and colleagues reported 6 perilesional injections of high dose, 5×10⁵ IU per dose to clear AKs (Arch. Dermatol., 1986. 122(7): p. 779-82). Lower doses of IFNa protein (1×10⁵, 1×10⁴ IU per injection) proved less effective in AK clearance in situ, respectively.

The mechanism of action of IFNa in the treatment of the various types of NMSC is partially known. The antitumor effect appears to be due to a combination of direct antiproliferative, as well as indirect effects, relying on the tumour stroma and microenvironment. The latter includes effects on both the innate and adaptive immune system as well as on the tumour vessels. Effectiveness of IFNa in Kaposi's sarcoma and hemangiomas demonstrates the clinical relevance of its antiangiogenic activity.

While IFNa has been shown to be effective in NMSC, in particular in AK, BCC and SCC, it never became a therapeutic option for clinical routine. The reasons are obvious. Treatment requires frequent (daily or three times weekly) perilesional injections over several weeks. Also, recombinant IFNa protein was expensive and the quality and importantly bioactivity of the preparations differed. IFNa was produced by recombinant DNA technology using a genetically engineered E. coli strain. Although expression by E. coli lead to high protein yield, the product had to be extensively purified and tested for potency and bioactivity in order to provide a comparable level of bioactivity for treatment with varying degree of non-active and active protein in the product.

The reason why there is a desire in modern medicine to avoid Interferons, especially IFNa, is that these proteins have a number of characteristic toxic effects. Four major groups of side-effects occur: constitutional, neuropsychiatric, hematological/immunological and hepatic. The severity of many of these side-effects appears to be directly related to the dose and duration of IFN protein therapy (Borden et al., Semin Oncol, 1998. 25 (1 Suppl 1): p. 3-8).

Constitutional symptoms include influenza-like symptoms such as fatigue, fever, chills and rigor. Constitutional symptoms occur early, often within 3-6 hours after dosing. Patients may also experience headaches, myalgias, and malaise. Transaminitis and neutropenia may occur within the first few days of treatment and can be controlled by reducing the dose. Transaminitis, if not handled appropriately, can result in fatal hepatotoxicity. The most frequent chronic symptoms experienced by patients on IFN include fatigue (70-100%), anorexia (40-70%) and neuropsychiatric disorders (up to 30%). These symptoms are dose related and cumulative, worsening over time. Auto-immune toxicities include triggering of lupus erythematosus, psoriasis and thyroid dysfunction. Thyroid dysfunction, ranging from overt to subclinical hyper- or hypothyroidism, occurs in 8-20% of patients receiving IFNa therapy. The pattern followed by most patients is one of an autoimmune thyroiditis, with a period of hyper- followed by hypothyroidism.

The need to replace protein Interferons, especially IFNa protein has i.a. led to the enormous market success of the combination of ledipasvir/sofosbuvir (sold under the trade name Harvoni) in the treatment of hepatitis C (replacing IFNa (in combination with ribavirin)).

However, also these commercially successful treatment options for NMSCs, especially AKs show significant adverse effects and limited efficacy in the course of treatment: Of note, in special individuals, local IFN inducers like the TLR7 agonist Imiquimod may lead to more concerning types of autoimmune toxicity such as the development of subacute cutaneous lupus erythematosus. Furthermore, certain individuals also carry single nucleotide polymorphisms in TLR7 which makes them non-responders to TLR7 agonists like Imiquimod (Pharmacogenomics. 2015. Nov.; 16 (17): p. 1913-17).

Accordingly, there is still a significant medical need for improved prevention and treatment schemes for NMSC providing improved patient's compliance and improved adverse reaction events.

Current field directed therapies represent a heterogenic class of topical agents including Imiquimod, Ingenol mebutate, Diclofenac and photosensitizers for photodynamic therapy (PDT) and fluorouracil. These current state-of-the-art treatments show histological clearance rates of AK following standard treatment reported as low as 67% following fluorouracil and 73% following Imiquimod. All of these current state of the art agents rely on an either immediately direct (e.g.:fluorouracil, ingenol mebutate, PDT) or indirect mechanism (imiquimod) for NMSC/AK therapy.

Imiquimod, one of the most widely used therapeutic agents, acts through activation of the innate immune system which in turn exerts anti-tumor activity.

Imiquimod application activates Toll-like receptor 7 (TLR7) signalling, which is commonly involved in pathogen recognition. Importantly, epidermal keratinocytes, as target cells for Imiquimod activity in NMSC, constitutively express several members of the TLR family. However, TLR7 expression is either absent or only very scarcely detectable. Thus, a direct effect of Imiquimod on keratinocytes is rather unlikely.

In contrast, there is compelling evidence for an indirect anti-tumor-function of Imiquimod: Imiquimod activates innate immune cells via TLR-7 which leads to extensive secretion of cytokines, primarily interferon-α (IFNa), interleukin-6 (IL-6), and tumour necrosis factor-α (TNF-α), among others, and to the rapid recruitment of plasmacytoid dendritic cells (pDCs) to the skin appplication side. Imiquimod induces pDC maturation and their conversion into cytolytic killer cells, which are capable of eliminating tumours independently of the adaptive immune system. Other cell types activated by Imiquimod include natural killer cells, and macrophages. In addition, there is also compelling evidence that Imiquimod, when applied to skin topically, leads to the activation of Langerhans cells, which subsequently migrate to local lymph nodes to activate the adaptive immune system including B-lymphocytes.

In contrast to the mere indirect effects of Imiquimod or the mere direct effects of fluorouracil, ingenol mebutate and PDT on aberrant cells in NMSC, BCC, SCC and especially AK, IVT mRNA mediated IFNa protein expression is exerting specific direct and indirect effects on affected cells (e.g.: keratinocytes which have been successfully transfected and are expressing IFNa). IF-Na is known to directly affect apoptosis, proliferation or cellular differentiation of tumour cells resulting from induction of a subset of genes, called IFN stimulated genes (ISGs). In addition to the ISGs implicated in anti-angiogenic, immunomodulatory and cell cycle inhibitory effects, oligonucleotide microarray studies have identified ISGs with apoptotic functions. These include TNF-α related apoptosis inducing ligand (TRAIL/Apo2L), Fas/FasL, XIAP associated factor-1 (XAF-1), caspase-4, caspase-8, dsRNA activated protein kinase (PKR), 2'5'A oligoadenylate synthetase (OAS), death activating protein kinases (DAP kinase), phospholipid scramblase, galectin 9, IFN regulatory factors (IRFs), promyelocytic leukaemia gene (PML) and regulators of IFN induced death (RIDs). Of note, IFNa will also exploit indirect anti tumor activity as excerted by immune modulators as it is currently expected to constitute one of the major factors involved in excerting anti tumor activity of Imiquimod.

IVT mRNA based IFNa expression is thus showing the potential to change the current state-of-the-art in treating NMSC, BCC, SCC and especially AK by exploiting several anti-tumour strategies (direct and indirect) simultaneously. Specifically, the direct IFNa activity in target cells, and, as the current state-of-the-art approach using immune modulators, activation of cells of the innate immune system and subsequent anti-tumour activity. By doing so one can readily imagine that such a dual strategy enhances overall activity of the treatment as it may enhance efficacy and/or enlarge the subset of patients responding to therapy.

A further object of the present invention is to provide methods which are easily reversible and do not have severe impact on the patient's body as a whole (i.e. (adverse) systemic consequences due to the treatment) . Moreover, it is a desire to provide cytokine treatment without the normally accompanied burden for the patients and to increase treatment efficiency, responder rates and patient compliance.

Therefore, to overcome the limitations of current treatement options summarized above, the present invention provides Interferon alpha (IFN-α) messenger-RNA (mRNA), wherein the mRNA has a 5' CAP region, a 5' untranslated region (5'-UTR), a coding region, a 3' untranslated region (3'-UTR) and a poly-adenosine Tail (poly-A Tail), for use in the prevention and treatment of non-melanoma skin cancer (NMSC).

Surprisingly, it was possible to apply IFN-α mRNA to patients suffering from NMSC, without the undue consequences known to be associated with administration of (recombinant) IFN-α protein. The present invention allows e.g. local administration on or into the skin of NMSC patients, especially patients suffering from actinic keratosis (AK), without the systemic adverse effects normally connected with IFN-α treatment.

Importantly, the present invention uses the full length IFNa precursor molecules instead of the recombinant molecule: e.g.: recombinant human IFNa2a used for clinical applications so far, is a 165aa long protein of bacterially manufactured origin, whereas the construct used in this invention is producing a fully human 188aa precursor protein in the transfected cells. This full-length precursor is intracellularly processed to allow for formation of the secreted bioactive protein of 165aas. Importantly, and in contrast to recombinant proteins like the bacterially produced Roferon A, this naturally processed protein is also including naturally occurring posttranslational modifications required for full bioactivity in mammals, especially humans. Thus, in contrast to recombinant, purified IFNas, all protein produced from IVT mRNA as presented in this invention is expected to have 100% bioactivity locally, in the intended target organ without major systemic exposure.

This is in contrast to recombinant IFNa, where only a portion of the recombinant material is bioactive and due to the high protein doses and injection volumes as well as mode of application, is also systemically active. E.g. after intramuscular injection of Roferon A, the bioavailability was greater than 80%. After intramuscular and subcutaneous administration of 36 million IU, peak serum concentrations ranged from 1,500 to 2,580 pg/ml (mean: 2,020 pg/ml) at a mean time to peak of 3.8 hours, and from 1,250 to 2,320 pg/ml (mean: 1,730 pg/ml) at a mean time to peak of 7.3 hours, respectively (Israeli ministry of health (MOH) approved prescribing information December 2001). Interestingly, recombinant IFNa is eliminated quickly following bolus applications with an average elimination half-life of 5.1h, explaining the need for the frequent and high dose application required for NMSC treatment in patients.

With the present invention, the drawbacks of the IFNa protein therapies known in the art were surprisingly overcome. On the other hand, instead of further developing the protein administration further (by providing better IFNa products, developing better routes of administration, etc.), the present invention provides a significant change in the direction of NMSC treatments: administering of IFNa-mRNA. IFNa-mRNA surprisingly does not lead to most of the adverse reaction observed for recombinant IFNa. The mRNA format of IFNa therefore provides a significant advantage over prior art therapy using IFNa protein. According to the present invention, following e.g. local and/or field-directed administration, IFNa mRNA will remain in the target cells and be expressed locally for several days at levels sufficient for the elimination of atypical keratinocytes without the need for bolus like application schedules and excessive dosing. This is impressively shown in the example section of the present invention. IFNa in vitro transcribed (IVT) mRNA according to the present invention is suited for the treatment of both, singular and multiple AK lesions depending primarily on the mode of administration, i.e. local versus field directed. Likewise, e.g. all 3 stages of AKs (Grade I-III) are targets for treatment with IFNa IVT mRNA according to the present invention.

The present invention also allows the use IFNa IVT mRNA as a minimally invasive, efficient local or field-directed therapy, with ideally a single application, building upon the sustainable expression of IVT mRNA in vivo. It is also evident that this strategy renders patient compliance irrelevant and increase treatment efficacy with higher responder rates than the current standard of care for AK and potentially for the direct treatment of SCC and BCC, respectively, especially those with IFNa protein that have been suggested more than 30 years ago. Other parameters including long-term recurrence rate and cosmetic outcome deserve consideration in the context of AK and NMSC and are also superior compared to current standard of care as well.

The mRNA used in the present invention contains (at least) five essential elements which are all known and available to a person skilled in the art (in this order from 5' to 3'): a 5' CAP region, a 5' untranslated region (5'-UTR), a coding region for IFN-α, a 3' untranslated region (3'-UTR) and a poly-adenosine tail (poly-A tail). The coding region should, of course encode a (human) IFN-α, the other components may be the (native) IFN-α UTRs or, preferably, other UTRs. Specifically preferred UTRs according to the present invention are UTRs which improve the properties of the mRNA molecule according to the present invention, i.e. by effecting better and/or longer and/or more effective translation of the mRNA into IFN-α protein at the site of administration.

A "CAP region" ("5'CAP") refers to a structure found on the 5' end of an mRNA molecule and generally consists of a guanosine nucleotide connected to the mRNA via an unusual 5' to 5' triphosphate linkage. This guanosine nucleotide is methylated on the 7-position directly after capping in vivo by a methyl transferase ("7-methylguanylate cap" ("m7G"), "cap-0"). Further modifications include the possible methylation of the 2' hydroxy-groups of the first two ribose sugars of the 5' end of the mRNA (i.e. "CAP1" and "CAP2"): "CAP1" has a methylated 2'-hydroxy group on the first ribose sugar, while "CAP2" has methylated 2'-hydroxy groups on the first two ribose sugars. The 5' cap is chemically similar to the 3' end of an RNA molecule (the 5' carbon of the cap ribose is bonded, and the 3' unbonded). This provides significant resistance to 5' exonucleases and is therefore also providing stability in vivo. For generation of mRNAs according to the present invention also CAP analogues may be used including but not limited to: monomethylated CAP analogue (mCAP), Anti-Reverse Cap Analog (ARCA CAP), m7G(5')ppp(5')A RNA CAP structure analog, G(5')ppp(5')A RNA CAP structure analog, and G(5')ppp(5')G RNA CAP structure analog.

The term "(5'- or 3'-)UTR" refers to the well-established concept of untranslated region of a mRNA in molecular genetics. There is one UTR on each side of a coding sequence on a strand of mRNA. The UTR on the 5' side, is the 5'-UTR (or leader sequence), the UTR on the 3' side, is the 3'-UTR (or trailer sequence). The 5'-UTR is upstream from the coding sequence. Within the 5'-UTR is a sequence that is recognized by the ribosome which allows the ribosome to bind and initiate translation. The mechanism of translation initiation differs in prokaryotes and eukaryotes. The 3'-UTR is found immediately following the translation stop codon. The 3'-UTR plays a critical role in translation termination as well as post-transcriptional gene expression. The UTRs as used in the present invention are usually delivering beneficial stability and expression (translation) properties to the mRNA molecules according to the present invention. The 3' end of the 3'-UTR also contains a tract of multiple adenosine monophosphates important for the nuclear export, translation, and stability of mRNA. This so-called poly-Adenosine (poly-A) tail consists of at least 60 adenosine monophosphates, preferably 100 and most preferably 120 adenosine monophosphates.

The "poly-A tail" consists of multiple adenosine monophosphates; it is a part of naturally occurring mRNA that has only adenine bases. This process called "polyadenylation" is part of the process that produces mature messenger RNA (mRNA) for translation in the course of gene expression. The natural process of polyadenylation begins as the transcription of a gene terminates. The 3'-most segment of the newly made pre-mRNA is first cleaved off by a set of proteins; these proteins then synthesize the poly(A) tail at the RNA's 3' end. In some genes these proteins add a poly(A) tail at one of several possible sites. Therefore, polyadenylation can produce more than one transcript from a single gene (alternative polyadenylation), similar to alternative splicing. The poly(A) tail is important for the nuclear export, translation, and stability of mRNA. For the present invention it is therefore mainly the translation and stability properties that are important for a sufficient polyadenylation of the mRNA molecules according to the present invention. During the protein generation, the tail is shortened over time, and, when it is short enough, the mRNA is enzymatically degraded. The poly-A tail according to the present invention is provided in the manner currently used and applied in the art of administering mRNA molecules in human therapy. For example, the poly-A tail may be at least 60 adenosine monophosphates long. According to a preferred embodiment, the poly-A tail is at least 100 adenosine monophosphates long, especially at least 120 adenosine monophosphates. This allows excellent stability and protein generation; however, as for the other features, the action and activity of the mRNA molecule according to the present invention can also be regulated by the poly-A tail feature.

The sequences used in the mRNA molecules according to the present invention can either be native or not. This holds true for the IFN-α coding region as well as for the UTRs.

The term "native" relates to the human IFN-α mRNA in its natural environment.

Preferably, the sequences are not native but are improved to increase various parameters of the mRNA molecule, such as efficacy, stability, deliverability, producibility, translation initiation and translation.

For example, instead of using the native IFN-α coding sequence, sequences optimised with respect to GC-content or codon adaption index (CAI) may be used according to preferred embodiments of the present invention (see below).

The present invention, due to its mechanism, targets treatment and prevention of NMSC in general; actinic keratosis (AK), basal cell carcinoma (BCC) and squamous cell carcinoma (SCC) are, however, preferred indications addressed with the present invention, especially AK. The present invention allows administration of a powerful molecule (IFN-α encoding mRNA) in a very diligent manner so as to obtain a successful clinical outcome for the patients and at least a significant amelioration of disease, especially for AK. In case of AK, amelioration of disease is measured by assessing the number of lesion in a pre-defined area, typically the field of skin exposed to a comparable degree of carcinogen (mainly UV radiation). Lesion counts are done at baseline and at defined time points after the treatment, typically 1 and 3 months later. Response of individual lesions is assessed visually and by palpation. Parameters to be reported include mean reduction of lesion counts from baseline to assessment, rate of participants with a complete clearance of all lesions within a predefined field, rate of participants with at least a 75% reduction in AK lesion counts within a predefined field.

According to a preferred embodiment, the IFN-α mRNA according to the present invention is IFN-α type 1 mRNA (IFNal), IFN-α type 2a mRNA (IFNa2a), or IFN-α type 2b mRNA (IFNa2b). These three types are the most straightforward IFN-α entities pursued by the present invention.

Since the major treatment/prevention area of the present invention is human medicine, the most preferred embodiment is, of course, a mRNA wherein the coding region encodes human IFN-α., especially human IFNal, human IFNa2a, or human IFNa2b (as encoded by the various SEQ ID NOs disclosed in the example section of the present invention encoding IFN-α).

According to a preferred embodiment of the present invention, the present mRNA comprises in the 5'-UTR and/or 3'-UTR (preferably in the 3'UTR) one or more stabilisation sequences that are capable of increasing the half-life of the mRNA intracellularly. These stabilization sequences may exhibit a 100% sequence homology with naturally occurring sequences that are present in viruses, bacteria and eukaryotic cells, but may however also be partly or completely synthetic. Examples for such stabilizing sequences are described in: Nucleic Acids Res. 2010; 38 (Database issue): D75-D80. UTRdb and UTRsite (RELEASE 2010): a collection of sequences and regulatory motifs of the untranslated regions of eukaryotic mRNAs and under http://utrdb.ba.itb.cnr.it/.

As a further example of stabilising sequences that may be used in the present invention, the non-translated sequences (UTR) of the β-globin gene, for example of Homo sapiens or Xenopus laevis, may be mentioned.

Another example of a stabilisation sequence has been described in Holcik et al. (Proc. Natl. Acad. Sci. USA 1997, 94: 2410 to 2414) and has the general formula:
(C/U)CCANₓCCC(U/A)PyₓUC(C/U)CC (SEQ ID NO:38),
which is contained in the 3'UTR of the very stable mRNAs that code for example for alpha(1)-collagen or for alpha globin, and ALOX15 or for tyrosine hydroxylase (and wherein "x" is (independently in Nₓ and Pyₓ) an integer of 0 to 10, preferably of 0 to 5 (Holcik et al., 1997), especially 0, 1, 2, 4 and/or 5).

Such stabilisation sequences may be used individually or in combination with one another for stabilizing the inventive mRNA as well as in combination with other stabilisation sequences known to the person skilled in the art. E.g.: The stabilizing effect of human β-globin 3'-UTR sequences is further augmented by using two human β-globin 3'-UTRs arranged in a head-to-tail orientation.

Accordingly, a preferred embodiment of the IFN-α mRNA according to the present invention is an mRNA molecule, wherein the 5'-UTR or 3'-UTR or the 5'-UTR and the 3'-UTR are different from the native IFN-α mRNA, preferably wherein the 5'-UTR or 3'-UTR or the 5'-UTR and the 3'-UTR contain at least one a stabilisation sequence, preferably a stabilisation sequence with the general formula (C/U)CCANₓCCC(U/A)PyₓUC(C/U)CC (SEQ ID NO:38).

Preferably, the 5'-UTR and/or 3'-UTR are the 5'-UTR and/or 3'-UTR of a different human mRNA than IFN-α, preferably selected from alpha Globin, beta Globin, Albumin, Lipoxygenase, ALOX15, alpha(1) Collagen, Tyrosine Hydroxylase, ribosomal protein 32L, eukaryotic elongation factor 1a (EEF1A1), 5'-UTR element present in orthopoxvirus, and mixtures thereof, especially selected from alpha Globin, beta Globin, alpha(1) Collagen, and mixtures thereof.

Accordingly, the present invention preferably relates to an mRNA which comprises in the 3'-UTR one or more stabilisation sequences that are capable of increasing the half-life of the mRNA in the cytosol. These stabilisation sequences may exhibit a 100% sequence homology with naturally occurring sequences that are present in viruses, bacteria and eukaryotic cells, but may, however, also be partly or completely synthetic. As an example of stabilising sequences that may be used in the present invention, the non-translated sequences (UTR) of the β-globin gene, for example of homo sapiens or xenopus laevis, may be mentioned. As already stated, another example of a stabilisation sequence has the general formula (C/U)CCANₓCCC(U/A)PyₓUC(C/U)CC, which is contained in the 3'-UTR of the very stable mRNA that codes for alpha-globin, alpha-(1)-collagen, 15-lipoxygenase or for tyrosine hydroxylase (c.f. Holcik et al., Proc. Natl. Acad. Sci. USA 1997, 94: 2410 to 2414). Such stabilisation sequences may be used individually or in combination with one another for stabilizing the inventive modified mRNA as well as in combination with other stabilisation sequences known to the person skilled in the art.

Another preferred embodiment of the present invention is the 5'-TOP-UTR derived from the ribosomal protein 32L, followed by a stabilizing sequence derived from the albumin-3'-UTR.

Accordingly, a preferred embodiment of the IFN-α mRNA according to the present invention is an mRNA molecule containing a tract of multiple adenosine monophosphates at the 3' end of the 3'-UTR. This so-called poly-adenosine (poly-A) tail consists of at least 60 adenosine monophosphates, preferably at least 100 and most preferably at least 120 adenosine monophosphates. Further stabilizing and translation efficient mRNAs are disclosed e.g. in WO 02/098443 A2 and EP 3 112 469 A1.

In certain cases, destabilizing the mRNA might be desirable as well to limit the duration of protein production. This effect can be achieved by incorporating destabilizing sequence elements (DSE) like AU-rich elements into 3'-UTRs, thus ensuring rapid mRNA degradation and a short duration of protein expression.

Although it may be desired for certain embodiments to provide an mRNA which is devoid of destabilizing sequence elements (DSE) in the 3' and/or 5' UTR, there may be other embodiments, wherein the presence or introduction of such DSEs is advantageous. In general, a "DSE" refers to a sequence, which reduces the half-life of a transcript, e.g. the half-life of the mRNA according to the present invention inside a cell and/or organism, e.g. a human patient. Accordingly, a DSE comprises a sequence of nucleotides, which reduces the intracellular half-life of an RNA transcript.

DSE sequences are found in short-lived mRNAs such as, for example: c-fos, c-jun, c-myc, GM-CSF, IL-3, TNF-alpha, IL-2, IL-6, IL-8, IL-10, Urokinase, bcl-2, SGL T1 (Na(+)-coupled glucose transporter), Cox-2 (cyclooxygenase 2), PAI-2 (plasminogen activator inhibitor type 2), beta(1)-adrenergic receptor or GAP43 (5'-UTR and 3'-UTR).

Further DSEs are AU-rich elements (AREs) and/or U-rich elements (UREs), including single, tandem or multiple or overlapping copies of the nonamer UUAUUUA(U/A) (U/A) (where U/A is either an A or a U) and/or the pentamer AUUUA and/or the tetramer AUUU. Further DSEs are described in Nucleic Acids Res. 2010; 38 (Database issue): D75-D80. UTRdb and UTRsite (RELEASE 2010) : a collection of sequences and regulatory motifs of the untranslated regions of eukaryotic mRNAs and under http://utrdb.ba.itb.cnr.it/.

Accordingly, it may also be preferred if the 5'-UTR or 3'-UTR or the 5'-UTR and the 3'-UTR contain at least one destabilisation sequence element (DSE), preferably AU-rich elements (AREs) and/or U-rich elements (UREs), especially a single, tandem or multiple or overlapping copies of the nonamer UUAUUUA(U/A)(U/A), such as the pentamer AUUUA and/or the tetramer AUUU (the term "U/A" meaning either A or U).

These stabilizing and destabilizing elements can be used alone or in combination to aim at a given duration of protein production and to individualize the treatment of the present invention to the specific NMSC type, the specific stage of disease, the specific group of patients or even to the specific patient in a specific state of disease in this patient.

Although also nucleic acids encoding IFNa have been suggested for therapeutic applications, these proposals have either been suggested considerable time ago, were mostly related to DNA (not RNA or specifically mRNA) and were related to completely different fields and modes of administration. Moreover, the advantages revealed in the context of the present invention were not observed for these prior art uses of IFNa-mRNA. Treatment regimens comprising IFNa nucleic acids have primarily been used on liver diseases and viral diseases such as hepatitis C. WO 98/17801 A1 discloses a pharmaceutical composition for intravesical hepatoma treatment, whereby the composition comprises a recombinant adenoviral vector comprising a liver-specific promoter sequence and an IFNa-b encoding sequence. In a different approach described in WO 2006/134195 A2, IFNa DNA is administered as combination therapy together with either an IL-6 family, Gpl30 family or ADN sequence for treating viral disease. Similar, WO 00/69913 A1 discusses a nucleic acid sequence or viral expression vector comprising a signal sequence, an immunoglobulin Fc region and a target protein sequence comprising IFNa, which is used for the treatment of hepatitis.

In the context of dermatological diseases, IFNa-DNA treatments have been applied to patients suffering from Condylomata acuminata (genital warts). In one approach the application WO/9000406 A1 described the combination of topical podophyllin treatment or an active constituent thereof together with intralesional injection of either recombinant human DNA IFN-2a or 2b in patients with Condylomata acuminata. In WO/9004977 A2 a combination therapy for the same disease disclosed intralesional injection of either recombinant human DNA IFN-2a or 2b following cryosurgical treatment with liquid nitrogen.

The use of immunostimulatory compositions comprising adjuvant mRNA complexed with a cationic or polycationic compound in combination with free mRNA encoding a tumour antigen has previously been described in WO 2010/037408 A1 for prophylaxis, treatment and/or amelioration of tumour diseases, autoimmune, infectious and allergic diseases. This approach allows efficient translation of the administered free mRNA into the protein of interest, while the mRNA complexed with the adjuvant component induces an immune response. WO 99/47678 A2 discloses the use of IFN- α plasmids for cancer treatment. Another approach to stabilize nucleic acid for in vivo application is the modification of nucleic acid sequence such as the addition of a Kunitz domain, a protease inhibitor (WO 2009/030464 A2).

RNA-based therapies for the treatment of rare dermatological diseases and treatments for use in medical dermatology, including AK, and aesthetic medicine have been suggested: WO 2015/117021 A1 discloses the use of a pharmaceutical composition comprising an RNA composed of one or more non-canonical nucleotides for the treatment of AK, whereby the nucleic acid encodes either for a protein of interest of the group of skin-specific structural or growth factor proteins, or for gene-editing protein targets. Similar, WO 2016/131052 A1 discusses the administration of RNA comprising non-canonical nucleotides encoding for either a protein of the family of interleukins, LIF, FGF growth factors, SERPINB1, caspase-1 or BMPs for treating diseases of the integumentary system including actinic keratosis. In both patent applications the administration of the pharmaceutical composition comprising the synthetic RNA can occur on multiple ways such as subcutaneous, intradermal, subdermal or intramuscular injection, as well as topical.

However, cytokines, such as interferons, especially IFNa, have not been suggested to be applied in this context.

General concepts for improved mRNA-based therapeutics (see e.g. Sahin et al., Nat. Rev. Drug Disc. 2014. 13(10): 759-780) are also applicable for the present invention.

For example, according to another preferred embodiment, the IFN-α mRNA according to the present invention may contain other residues than cytidine (C), uridine (U), adenosine (A) or guanosine (G) residues. There are a significant number of naturally occurring analogs of these nucleosides and also (even more) synthetic variants of these mRNA residues. Preferred embodiments of such variants can be found e.g. in WO 2014/153052 A2 and WO 2015/062738 A1.

According to a preferred embodiment, in the present IFN-α mRNA, at least 5%, preferably at least 10%, preferably at least 30%, especially at least 50% of all
- cytidine residues are replaced by 5-methyl-cytidine residues, and/or
- cytidine residues are replaced by 2-amino-2-deoxy-cytidine residues, and/or
- cytidine residues are replaced by 2-fluoro-2-deoxy-cytidine residues, and/or
- cytidine residues are replaced by 2-thio-cytidine residues, and/or
- cytidine residues are replaced by 5-iodo-cytidine residues, and/or
- uridine residues are replaced by pseudo-uridine residues, and/or
- uridine residues are replaced by 1-methyl-pseudo-uridine residues, and/or
- uridine residues are replaced by 2-thio-uridine residues, and/or
- uridine residues are replaced by 5-methyl-uridine residues, and/or
- adenosine residues are replaced by N6-methyl-adenosine residues.

Especially preferred embodiments are IFN-α mRNAs, wherein in the IFN-α mRNA, at least 5%, preferably at least 10%, preferably at least 30%, especially at least 50% of all
- cytidine residues are replaced by 5-methyl-cytidine residues, and/or
- uridine residues are replaced by pseudo-uridine residues, and/or
- uridine residues are replaced by 2-thio-uridine residues.

In the course of the present invention it has been surprisingly found out that even more improved results can be obtained if the GC-content (or GC to AU ratio) of the mRNA is further increased. The reason why this was specifically surprising was that the native IFNa sequences were already regarded as being optimal with respect to translation/expression efficiency. However, if the IFN-α mRNA according to the present invention is designed with a GC to AU ratio of at least 49.5% or more preferred at least 49.6% (e.g. at least 49.7, at least 49.8, at least 49.9), the performance according to the present invention further increases. Accordingly, the GC to AU ratio is preferably of at least 50%, more preferred, at least 55%, especially at least 60%.

In connection with the nucleoside variants disclosed above, it is important to note that the specific preferred variants described above do not influence the GC content, i.e. the variant is conservative in this respect (e.g. a cytidine variant still counts as a cytidine for the calculation of the GC content).

Another surprising observation revealed in the course of the present invention was the fact that IFNa-mRNAs with increased Codon Adaptation Index (CAI) also showed improved performance in the present invention, especially with respect to expression capacity within the cell.

The CAI is a measurement of the relative adaptiveness of the codon usage of a gene towards the codon usage of highly expressed genes. The relative adaptiveness (w) of each codon is the ratio of the usage of each codon, to that of the most abundant codon for the same amino acid. The CAI index is defined as the geometric mean of these relative adaptiveness values. Non-synonymous codons and termination codons (dependent on genetic code) are excluded. CAI values range from 0 to 1, with higher values indicating a higher proportion of the most abundant codons (Sharp et al., Nucleic Acids Res. 15 (1987) : 1281-1295., Jansen et al., Nucleic Acids Res. 31 (2003): 2242-2251).

Therefore, a preferred embodiment of the present invention relates to an IFN-α mRNA, wherein the IFN-α mRNA has a codon adaption index (CAI) of at least 0.8, preferably at least 0.81, at least 0.82, at least 0.83, at least 0.84, at least 0.85, at least 0.86, at least 0.87, at least 0.88, at least 0.89. Even a more preferred CAI of the mRNAs according to the present invention is at least 0.9, especially at least 0.91.

For comparison: native GC to AU ratio of IFNa1 is 49.5%, of IFNa2a is 48.7%, and of IFNa2b is 48.9%; native CAIs of IFNa1 is 0.8, of IFNa2a is 0.8, and of IFNa2b is 0.8.

Even more preferred, the mRNAs according to the present invention have a CAI of at least 0.8 AND a GC content of at least 49.5% (or an even more preferred higher CAI/GC content).

Preferred consensus sequences of the mRNA according to the present invention for IFNa2a are SEQ ID NOs:12, 13 and 14; most preferred SEQ ID NO:12.

SEQ ID NO:12 comprises GC rich sequences only; SEQ ID NO:13 is also optimised and includes AU rich sequences; SEQ ID NO:14 is an optimised sequence.

Accordingly, the coding region of the IFN-α mRNA encoding human IFNa2a is preferably SEQ ID NO:12, especially SEQ ID NOs: 2, 3, 5, 6, 7, 8, 9, 10, or 11; the coding region of the IFN-α mRNA encoding human IFNa2b is preferably SEQ ID NO:26, especially SEQ ID NOs: 19, 20, 22, or 25; the coding region of the IFN-α mRNA encoding human IFNa1 is preferably SEQ ID NO:36, especially SEQ ID NOs: 29, 30, 31, 32, 34, or 35.

Preferred embodiments of the present invention are IFN-α mRNAs which showed improved performance within the course of the present invention, specifically those molecules,
- wherein the IFN-α mRNA has a CAI of 0.8 or more and a GC content of 48.7% or more;
- wherein the IFN-α mRNA has a CAI of 0.8 to 0.99, preferably of 0.81 to 0.97, especially of 0.83 to 0.85 (such as e.g. SEQ ID NO:3);
- wherein the IFN-α mRNA has a GC content of 48.7% to 63.7%, preferably of 48.7% to 60%, especially of 48.7% to 58.0%;
- wherein the IFN-α mRNA has a CAI of 0.80 to 0.97 and a GC content of 48.7% to 63.7%;
- wherein the IFN-α mRNA has a CAI of 0.6 to 0.8 and an AU content of 48.7% to 65.0%;
- wherein the IFN-α mRNA has a CAI of 0.6 to 0.8, preferably of 0.6 to 0.7, especially of 0.65 to 0.75 (such as e.g. SEQ ID NO:4); and
- wherein the IFN-α mRNA has an AU content of 48.7% to 65.0%, preferably of 51.3 to 60.0%.

The present invention also relates to the mRNA molecules provided with the present invention (excluding the native RNA sequences and all mRNA sequences which comprise the native coding region of IFN-α).

According to a preferred embodiment, the IFN-α mRNA according to the present invention is administered subcutaneously, intradermally, transdermally, epidermally, or topically, especially epidermally.

Administration of the present IFN-α mRNA can be performed according to optimised expression aims, depending on the amount of mRNA applied, the stability of the mRNA molecule and the status of disease. For example, the IFN-α mRNA may be administered at least once, at least twice, at least twice within one month, preferably weekly. For example, if the IFN-α mRNA may be administered at least twice, at least twice within one month, preferably weekly doses applied may vary.

The amount of mRNA delivered per dose may also be made dependent on the stability of the molecule, etc.. Preferably the IFN-α mRNA according to the present invention is administered in an amount of 0.001µg to 100 mg per dose, preferably of 0.01µg to 100mg per dose, more preferably of 0.1µg to 10mg per dose, especially of 1µg to 1mg per dose.

Suitable formulations for mRNA therapeutics are well available in the art (see e.g. Sahin et al., 2014; WO 2014/153052 A2 (paragraphs 122 to 136), etc.).

The present invention therefore also relates to a pharmaceutical formulation comprising an IFN-α mRNA according to the present invention. The present formulation comprises the mRNA in a pharmaceutically acceptable environment, e.g. with suitable components usually provided in mRNA therapeutics (excipients, carriers, buffers, auxiliary substances (e.g. stabilizers), etc.)

The mRNA formulations according to the present invention preferably contain a suitable carrier. Suitable carriers include polymer based carriers, such as cationic polymers including linear and branched PEI and viromers, lipid nanoparticles and liposomes, nanoliposomes, ceramide-containing nanoliposomes, proteoliposomes, cationic amphiphilic lipids e.g: SAINT^{®}-Lipids, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, dry powders, poly(D-arginine), nanodendrimers, starch-based delivery systems, micelles, emulsions, sol-gels, niosomes, plasmids, viruses, calcium phosphate nucleotides, aptamers, peptides, peptide conjugates, small-molecule targeted conjugates, and other vectorial tags. Also contemplated is the use of bionanocapsules and other viral capsid proteins assemblies as a suitable carrier. (Hum. Gene Ther. 2008 Sep;19(9):887-95).

Preferred carriers are cationic polymers including linear and branched PEI and viromers, lipid nanoparticles and liposomes, transfersomes, and nanoparticulates including calcium phosphate nanoparticulates (i.e. naked RNA precipitated with CaCl₂ and then administered).

A preferred embodiment of the present invention related to the use of non-complexed mRNA, i.e. non-complexed mRNA in a suitable aqueous buffer solution, preferably a physiological glucose buffered aqueous solution (physiological). For example, a 1xHEPES buffered solution; a 1xPhosphate buffered solution, Na-Citrate buffered solution; Na-Acetate buffered solution; preferred with Glucose (e.g.: 5% Glucose); physiologic solutions can be preferably applied.

Preferably the present invention applies liposomes, especially liposomes which are based on DOTAP, DOTMA, Dotap-DOPE, DOTAP-DSPE, Dotap-DSPE-PEG, Dotap-DOPE-PEG, Dotap-DSPE-PEG-Na-Cholate, Dotap-DOPE-PEG-Na-Cholate, DOTAP with cationic amphiphilic macromolecules (CAM) as complexes, and combinations thereof.

According to another aspect, the present invention relates to a kit for administering the IFN-α mRNA according to the present invention to a patient comprising
- the IFN-α mRNA as defined herein, and
- a skin delivery device.

Preferably, the skin delivery device is
- an intradermal delivery device, preferably selected from the group consisting of needle based injection systems, and needle-free injecton systems,
- a transdermal delivery device, preferably selected from the group consisting of transdermal patches, hollow and solid microneedle systems, microstructured transdermal systems, electrophoresis systems, and iontophoresis systems, or
- an epidermal delivery device, preferably selected from the group consisting of needle free injection systems, laser based systems, especially Erbium YAG laser systems, and gene gun systems.

These administration devices are in principle available in the art; adaption to the administration of the mRNA according to the present invention is easily possible for a person skilled in the art.

The present invention also relates to a method for treating and preventing NMSC, preferably AK, BCC and SCC, wherein the mRNA according to the present invention is administered in an effective amount to a patient in need thereof.

According to a further aspect, the present invention also relates to the use of the mRNAs according to the present invention for the presvention or treatment of Condyloma und vascular deformities. Also for these aspects, the present invention is suitable.

In addition to NMSC (esp.: AK, BCC and SCC) also treatment of other skin conditions/diseases may be subject of the present invention. These conditions/diseases include but are not limited to vascular tumors and/or malformations such as hemangioma, and port-wine stains (nevus flammeus, firemark).

Superficial hemangiomas are situated higher in the skin and have a bright red, erythematous to reddish-purple appearance. Superficial lesions can be flat and telangiectatic, composed of a macule or patch of small, varied branching capillary blood vessels. They can also be raised and elevated from the skin, forming papules and confluent bright red plaques like raised islands. Superficial hemangiomas in certain locations, such as the posterior scalp, neck folds and groin/perianal areas are at potential risk of ulceration. Ulcerated hemangiomas can present as black crusted papules or plaques, or painful erosions or ulcers. Ulcerations are prone to secondary bacterial infections which can present with yellow crusting, drainage, pain or odor. Ulcerations are also at risk for bleeding, particularly deep lesions or in areas of friction. Multiple superficial hemangiomas, more than 5 can be associated with extracutaneous hemangiomas, the most common being a liver (hepatic) hemangioma and these warrant ultrasound examination.

Deep hemangiomas initially often present as poorly defined, bluish macules that can proliferate into papules, nodules or larger tumors. Proliferating lesions are often compressible, but fairly firm. Many deep hemangiomas may have a few superficial capillaries visible evident over the primary deep component or surrounding venous prominence. Deep hemangiomas have a tendency to develop a little later than superficial hemangiomas and may have longer and later proliferative phases as well. Deep hemangiomas rarely ulcerate, but can cause issues depending on their location, size and growth. Deep hemangiomas near sensitive structures can cause compression of softer surrounding structures during the proliferative phase, such as the external ear canal and the eyelid. Mixed hemangiomas are simply a combination of superficial and deep hemangiomas, and may not be evident for several months. Patients may have any combination of superficial, deep or mixed hemangiomas.

Treatment options for hemangiomas include medical therapies (systemic,intralesional and topical), surgery, and laser therapy. Prior to 2008, the mainstay of therapy for problematic hemangiomas was oral corticosteroids, which are effective and remain an option for patients in whom beta-blocker therapy is contraindicated or poorly tolerated. Following the serendipitous observation that propranolol, a non-selective beta blocker, is well tolerated and effective for treatment of hemangiomas, the agent was studied in a large, randomized controlled trial and was approved by the U.S. Food and Drug Administration for this indication in 2014. Propranolol has subsequently become the first-line systemic medical therapy for treatment of these lesions. Other systemic therapies which may be effective for hemangioma treatment include vincristine, interferon- and other agents with antiangiogenic properties. Vincristine, which requires central venous access for administration, is traditionally used as a chemotherapy agent, but has been demonstrated to have efficacy against hemangiomas and other childhood vascular tumors, such as Kaposiform hemangioendothelioma and tufted angioma. Interferon-alpha 2a and 2b, given via subcutaneous injection, has shown efficacy against hemangiomas (Wilson et al., Ophthalmology 2007; 114 (5): 1007-11), but may result in spastic diplegia in up to 20% of treated children (Barlow et al., J. Pediatr. 1998;132(3 pt 1):527-30; and Worle et al., Eur. J. Pediatr. 1999;158(4):344). Therefore, IFNa agents are rarely utilized now in the era of beta blocker therapy for hemangiomas.

### Sequences:

SEQ ID NO:1 IFNa2a 
auggccuuga ccuuugcuuu acugguggcc cuccuggugc ucagcugcaa gucaagcugc
ucugugggcu gugaucugcc ucaaacccac agccugggua gcaggaggac cuugaugcuc
cuggcacaga ugaggaaaau cucucuuuuc uccugcuuga aggacagaca ugacuuugga
uuuccccagg aggaguuugg caaccaguuc caaaaggcug aaaccauccc uguccuccau
gagaugaucc agcagaucuu caaucucuuc agcacaaagg acucaucugc ugcuugggau
gagacccucc uagacaaauu cuacacugaa cucuaccagc agcugaauga ccuggaagcc
ugugugauac aggggguggg ggugacagag acuccccuga ugaaggagga cuccauucug
gcugugagga aauacuucca aagaaucacu cucuaucuga aagagaagaa auacagcccu
ugugccuggg agguugucag agcagaaauc augagaucuu uuucuuuguc aacaaacuug
caagaaaguu uaagaaguaa ggaauga
SEQ ID NO:2 IFNa2a 
auggcacuga cauuugcccu gcucguugcu cuuuuggucc uuuccugcaa gaguagcugc
ucuguuggcu gugauuugcc ccaaacccac ucucucgguu caaggagaac ucugaugcug
cuugcccaaa ugcggaagau uagccuguuc ucaugccuga aagaccggca ugauuucggc
uuuccucagg aggaauuugg gaaccaguuc cagaaagcgg aaaccauucc cguccuucac
gagaugaucc agcagaucuu caaccuguuu ucuaccaagg auuccagugc ugcuugggau
gagacacugc uggacaaguu cuacacugag cucuaucagc agcugaauga ccuggaagcc
ugugugaucc aaggaguagg agugacugag acaccacuca ugaaagagga cuccauacuc
gcagugcgca aguacuucca gaggauuacc cuguaucuga aggagaagaa auacaguccg
ugugcauggg aaguggugag agccgagauc augcguagcu uuucccuguc aacgaaucug
caggaaagcu ugcgaagcaa agaauga
SEQ ID NO:3 IFNa2a 
auggcacuga cauucgcccu gcucgucgcu cuccucgucc ucuccugcaa gaguagcugc
ucugucggcu gugauuugcc ccaaacccac ucccucgguu ccaggcgcac ucugaugcug
cucgcccaga ugcggaagau uagccuguuc ucaugccuga aggaccggca ugauuucggc
uucccucagg aggaauucgg gaaccaguuc cagaaggcgg agaccauccc cguccuccac
 
gagaugaucc agcagaucuu caaccuguuc ucuaccaagg acuccagugc ugcuugggac
gagacccugc ucgacaaguu cuacacugag cucuaccagc agcugaacga ccuggaggcc
ugugugaucc aaggagucgg agugacugag acaccacuca ugaaggagga cuccauccuc
gcaguccgca aguacuucca gaggaucacc cuguaccuga aggagaagaa guacaguccg
ugugcauggg agguggugag agccgagauc augcguagcu ucucccuguc aacgaaccug
caggagagcc uccgaagcaa ggaguga
SEQ ID NO:4 IFNa2a 
auggcacuga cauuugcccu gcucguugcu cuuuuggucc uuucuugcaa gaguagcugu
ucuguuggau gugauuugcc ucaaacucau ucuuugguuu caagaagaac uuugaugcuu
cuugcacaaa ugagaaagau uagccuuuuc ucauguuuga aagaucgaca ugauuucgga
uuuccucaag aagaauuugg uaaccaauuc caaaaagcug aaaccauucc uguccuucau
gaaaugaucc aacaaaucuu caaucuuuuu ucuacuaagg auucuagugc ugcuugggau
gaaacacuuc uugauaaguu cuacacugaa cucuaucaac agcugaauga cuuggaagcc
uguguuaucc aaggaguugg aguuacugag acaccacuca ugaaagaaga uuccauacuc
gcaguucgca aguacuucca aaggauuacc uuguaucuga aggaaaagaa auacaguccu
ugugcauggg aagugguuag agcugaaauc augcguagcu uuucccuguc aacgaauuug
caggaaagcu ugcgaagcaa agaauga
SEQ ID NO:5 IFNa2a 
auggcccuga cauucgcucu gcugguggcc cugcuggugc ugagcugcaa gagcagcugu
agcgugggcu gcgaccugcc ucagacacac agccugggca gcagacggac ccugaugcug
cuggcccaga ugcggaagau cagccuguuc agcugccuga aggaccggca cgacuucggc
uucccucagg aagaguucgg caaccaguuc cagaaggccg agacaauccc cgugcugcac
gagaugaucc agcagaucuu caaccuguuc uccaccaagg acagcagcgc cgccugggac
gagacacugc uggacaaguu cuacaccgag cuguaccagc agcugaauga ccuggaagcc
ugcgugaucc agggcguggg cgugacagag acaccccuga ugaaggaaga uagcauccug
gccgugcgca aguacuucca gcggaucacc cuguaccuga aagagaagaa guacagcccc
ugcgccuggg aggucgugcg ggccgagauc augagaagcu ucagccugag caccaaccug
caggaaagcc ugcggagcaa agaguga
SEQ ID NO:6 IFNa2a 
auggcucuca ccuucgcccu gcugguggca cuucuugucc ucucauguaa aucuagcugu
agcguuggcu gcgaucugcc ucagacucau agucugggau cucggaggac gcuuaugcug
uuggcccaga ugaggaagau cucccuguuc uccugucuca aagaccggca cgauuuuggc
uucccacagg aggaguuugg gaaccaguuc cagaaagcug agaccauccc ggugcuucau
gaaaugaucc agcagaucuu caaucuguuc aguacaaagg auaguucugc ugcuugggac
gagacacucc ucgacaaauu uuacacugaa cuguaucagc aguugaacga ccuugaggcu
ugcguuauuc agggagucgg ugugacagaa acuccccuca ugaaggagga cagcauccuc
gccguucgaa aguauuucca acgaaucaca cuguaucuua aggagaaaaa guacagccca
 
ugugccuggg agguuguccg ggcugagaua augcgaaguu ucucacugag uacaaacuug
caggagagcc uccgaucaaa ggaguga
SEQ ID NO:7 IFNa2a 
auggcucuua cguucgcucu uuugguugcc cucuuggugc ugaguuguaa auccucaugu
uccgugggau gcgaucugcc gcagacucac ucucuuggca guagaaggac ccugaugcug
cuggcucaaa ugcggaagau uagucuguuc uccugccuga aggaccggca ugacuucggu
uuuccacagg aggaauucgg aaaccaguuu cagaaggcug agacaauccc ugugcugcac
gaaaugaucc aacagauuuu caaccuuuuc ucaaccaagg acuccucagc cgccugggac
gaaacacugc uggauaaauu cuauaccgag cucuaccaac agcugaacga uuuggaggca
ugugucaucc agggggucgg ggucacugag acuccacuga ugaaggaaga cuccauucuc
gcgguaagga aauacuucca gaggaucacg cuguaccuga aggaaaagaa auacagcccu
ugcgcauggg aggugguucg cgcugagauc augcggagcu ucagucugag cacuaaucug
caggaaagcc ucaggucaaa ggaauag
SEQ ID NO:8 IFNa2a 
auggcuuuga ccuucgcccu ccugguggcc cuguuggugc ucucaugcaa auccagcugc
ucagugggcu gcgauuugcc ccagacccac ucacugggaa guaggaggac uuugaugcug
uuggcucaga ugcggaaaau cucucuguuc uccugccuga aggauaggca ugacuuuggu
uuuccucaag aggaguuugg aaaccaguuc caaaaggccg aaaccauccc ugugcuccac
gaaaugaucc agcagauauu uaaccuuuuc ucuacuaaag auuccagcgc cgcauggac
gagacccugc uugacaaguu cuacacugag cucuaccagc agcucaacga ccuggaggcg
ugugugaucc agggugucgg ugugacggag acuccccuua ugaaagagga uucuauccug
gcagugcgga aauauuuuca gagaaucacg cuguauuuga aggagaagaa guauagcccc
ugcgccuggg aaguggugag ggcugaaauu augcgcaguu ucagcuugag caccaaccug
caggaauccc uucgguccaa ggaauaa
SEQ ID NO:9 IFNa2a 
auggcccuga ccuucgcacu gcugguggca cugcugguuc uuuccuguaa gagcaguugc
agcguuggau gugaccugcc acagacucau ucucugggua gccggcgcac ucuuaugcuc
cucgcacaaa ugaggaagau uagucuguuc agcugucuca aagauagaca cgauuuuggc
uucccucagg aagaauucgg aaaccaguuu cagaaggccg agaccauccc cguguugcau
gagaugauuc agcagaucuu caaccuguuu ucuaccaagg acagcuccgc ugcuugggac
gagacucugc uggacaaguu cuacacugag cucuaccagc agcugaacga ccuugaagcc
ugcguuauuc agggcgucgg cgugacagag acuccacuga ugaaagagga caguauccug
gccgugcgga aguauuuuca gaggauuaca cuuuauuuga aggaaaagaa guacuccccc
ugcgcauggg aagugguaag ggcugaaauc augcggagcu uuucccuguc uaccaaccug
caggaauccc ugagauccaa agaauaa
SEQ ID NO:10 IFNa2a 
auggcacuga cauuugccuu gcucgucgcc cugcuuguuc uguccuguaa gagcuccugc
ucaguuggcu gcgaucuucc ucaaacccau agccucggua gccgccgaac ccugaugcug
cuggcccaga ugcgcaagau uagucuguuc ucuugucuga aagacaggca cgauuucgga
uucccacagg aggaguucgg caaucaauuc cagaaagcgg agaccauccc cgugcuucac
gagaugauuc agcaaauuuu caaccuguuc aguacuaaag auucuagcgc ugcguggac
gagacccugc uggacaaauu cuauacagaa cucuaucagc agcugaacga ucuggaggcc
uguguuaucc aggggguagg ugugaccgaa accccucuua ugaaggaaga uuccauccug
gccguuagga aauacuucca gcgcaucaca uuguaccuga aggagaaaaa guacagcccu
ugcgcauggg agguggucag agcugaaauc augcgaucau uuagucucag uacuaaucuc
caagagucac ugcgcuccaa ggaguaa
SEQ ID NO:11 IFNa2a 
auggcccuga ccuucgcccu gcugguggcc cugcuggugc ugagcugcaa gagcagcugc
agcgugggcu gcgaccugcc ccagacccac agccugggca gccgccgcac ccugaugcug
cuggcccaga ugcgcaagau cagccuguuc agcugccuga aggaccgcca cgacuucggc
uucccccagg aggaguucgg caaccaguuc cagaaggccg agaccauccc cgugcugcac
gagaugaucc agcagaucuu caaccuguuc agcaccaagg acagcagcgc cgccugggac
gagacccugc uggacaaguu cuacaccgag cuguaccagc agcugaacga ccuggaggcc
ugcgugaucc agggcguggg cgugaccgag accccccuga ugaaggagga cagcauccug
gccgugcgca aguacuucca gcgcaucacc cuguaccuga aggagaagaa guacagcccc
ugcgccuggg agguggugcg cgccgagauc augcgcagcu ucagccugag caccaaccug
caggagagcc ugcgcagcaa ggaguaa
SEQ ID NO:12 IFNa2a consensus (GC rich sequences only) 
auggchyuba cvuuygchyu byusgubgch cubyubgubc ubwshugyaa rwsywshugy
wshgubggmu gygayyukcc ncarachcay wshcubgghw shmgvmgvac byukaugcus
yubgchcara ugmgsaarau ywsycuguuc wshugycusa argaymgvca ygayuuyggh
uuycchcarg argaruuygg vaaycaruuy caraargcbg aracmauycc bgusyubcay
garaugauyc arcarauhuu yaaycukuuy wshachaarg aywsywshgc ygcnugggay
garachcusc ubgayaaruu yuayachgar cusuaycarc agyusaayga yyukgargcn
ugygubauyc arggnguvgg ngusacngar achcchcuba ugaargarga ywsyauhcus
gcvgunmgva aruayuuyca rmgvauyacv yukuayyuka argaraaraa ruaywsyccn
ugygcmuggg argubgunmg vgcygarauh augmgnwshu uywshyusws hacnaayyus
cargarwsmy ubmgvwsmaa rgarurr
SEQ ID NO:13 IFNa2a consensus (GC rich plus AT rich sequences) 
auggchyuba cvuuygchyu byusgubgch cubyubgubc ubwshugyaa rwsywshugy
wshgubggmu gygayyukcc ncarachcay wshyubgghw shmgvmgvac byukaugcub
yubgchcara ugmgvaarau ywsycukuuc wshugyyusa argaymgvca ygayuuyggh
uuycchcarg argaruuygg naaycaruuy caraargcbg aracmauycc bgusyubcay
 
garaugauyc arcarauhuu yaaycukuuy wshachaarg aywsywshgc ygcnugggay
garachcubc ubgayaaruu yuayachgar cusuaycarc agyusaayga yyukgargcn
ugygubauyc arggngungg ngubacngar achcchcuba ugaargarga ywsyauhcus
gcvgunmgva aruayuuyca rmgvauyacv yukuayyuka argaraaraa ruaywsyccn
ugygcmuggg argubgunmg vgcygarauh augmgnwshu uywshyusws hacnaayyus
cargarwsmy ubmgvwsmaa rgarurr
SEQ ID NO:14 IFNa2a consensus (all sequences including native sequences 
auggchyuba cvuuygchyu nyusgubgch cubyubgubc ubwshugyaa rwshwshugy
wshgubggmu gygayyukcc ncarachcay wshyubgghw shmgvmgvac byukaugcub
yubgchcara ugmgvaarau ywsycukuuc wshugyyusa argaymgvca ygayuuyggh
uuycchcarg argaruuygg naaycaruuy caraargcbg aracmauycc bgusyubcay
garaugauyc arcarauhuu yaaycubuuy wshachaarg aywshwshgc ygcnugggay
garachcubc ungayaaruu yuayachgar cusuaycarc agyusaayga yyukgargcn
ugygubauhc arggngungg ngubacngar achcchcuba ugaargarga ywsyauhcus
gcngunmgva aruayuuyca rmgvauyacn yubuayyuka argaraaraa ruaywsyccn
ugygcmuggg argubgunmg vgchgarauh augmgnwshu uywshyusws hacnaayyus
cargarwshy unmgvwshaa rgarurr
SEQ ID NO:15 IFNa2a protein
SEQ ID NO:16 Model Seq including the 5'UTR (aGlobin, human+ Koszak) 
gggagacata aaccctggcg cgctcgcggc ccggcactct tctggtcccc acagactcag
agagaaccca cc
SEQ ID NO:17 Model Seq including the 3'UTR (aGlobin, human+ Poly A site and first A(of 120)) 
gctggagcct cggtggccat gcttcttgcc ccttgggcct ccccccagcc cctcctcccc
ttcctgcacc cgtacccccg tggtctttga ataaagtctg agtgggcggc aaaaaaaaaa
aaaaaaaaaa aaaaaaaaaa aaaaaaaaaa aaaaaaaaaa aaaaaaaaaa aaaaaaaaaa
aaaaaaaaaa aaaaaaaaaa aaaaaaaaaa aaaaaaaaaa aaaaaaaaaa
SEQ ID NO:18 IFNa2b 
auggccuuga ccuuugcuuu acugguggcc cuccuggugc ucagcugcaa gucaagcugc
ucugugggcu gugaucugcc ucaaacccac agccugggua gcaggaggac cuugaugcuc
 
cuggcacaga ugaggagaau cucucuuuuc uccugcuuga aggacagaca ugacuuugga
uuuccccagg aggaguuugg caaccaguuc caaaaggcug aaaccauccc uguccuccau
gagaugaucc agcagaucuu caaucucuuc agcacaaagg acucaucugc ugcuugggau
gagacccucc uagacaaauu cuacacugaa cucuaccagc agcugaauga ccuggaagcc
ugugugauac aggggguggg ggugacagag acuccccuga ugaaggagga cuccauucug
gcugugagga aauacuucca aagaaucacu cucuaucuga aagagaagaa auacagcccu
ugugccuggg agguugucag agcagaaauc augagaucuu uuucuuuguc aacaaacuug
caagaaaguu uaagaaguaa ggaauga
SEQ ID NO:19 IFNa2b 
auggcccuga ccuucgcccu gcugguggcc cugcuggugc ugagcugcaa gagcagcugc
agcgugggcu gcgaccugcc ccagacccac agccugggca gccgccgcac ccugaugcug
cuggcccaga ugcgccgcau cagccuguuc agcugccuga aggaccgcca cgacuucggc
uucccccagg aggaguucgg caaccaguuc cagaaggccg agaccauccc cgugcugcac
gagaugaucc agcagaucuu caaccuguuc agcaccaagg acagcagcgc cgccugggac
gagacccugc uggacaaguu cuacaccgag cuguaccagc agcugaacga ccuggaggcc
ugcgugaucc agggcguggg cgugaccgag accccccuga ugaaggagga cagcauccug
gccgugcgca aguacuucca gcgcaucacc cuguaccuga aggagaagaa guacagcccc
ugcgccuggg agguggugcg cgccgagauc augcgcagcu ucagccugag caccaaccug
caggagagcc ugcgcagcaa ggaguaa
SEQ ID NO:20 IFNa2b 
auggcccuga cauucgcucu gcugguggcc cugcuggugc ugagcugcaa gagcagcugu
agcgugggcu gcgaccugcc ucagacacac agccugggca gcagacggac ccugaugcug
cuggcccaga ugcggagaau cagccuguuc agcugccuga aggaccggca cgacuucggc
uucccucagg aagaguucgg caaccaguuc cagaaggccg agacaauccc cgugcugcac
gagaugaucc agcagaucuu caaccuguuc uccaccaagg acagcagcgc cgccugggac
gagacacugc uggacaaguu cuacaccgag cuguaccagc agcugaauga ccuggaagcc
ugcgugaucc agggcguggg cgugacagag acaccccuga ugaaggaaga uagcauccug
gccgugcgca aguacuucca gcggaucacc cuguaccuga aagagaagaa guacagcccc
ugcgccuggg aggucgugcg ggccgagauc augagaagcu ucagccugag caccaaccug
caggaaagcc ugcggagcaa agaguga
SEQ ID NO:21 IFNa2b 
auggcuuuga cauucgcacu guuggucgcc cugcuugugc ucucaugcaa aagcaguugu
uccguggguu gcgauuugcc acagacucac agucugggau cucgccgcac ucugaugcuu
cucgcgcaaa ugcgccggau uagucuuuuc uccugucuga aggauagaca cgacuuuggg
uuuccccagg aggaguuugg gaaucaguuc caaaaggcgg aaacuauucc uguccuucac
gaaaugaucc agcagauauu uaauuuguuc ucaacaaaag auucaucagc ugcauggac
gaaacccugc uggauaaguu cuacacggag cucuaccagc agcugaauga ucucgaagcc
 
ugugucaucc agggcguagg agugacagaa accccacuga ugaaggagga uucaauccug
gcggugagga aguauuucca gcggaucacc cuguaucuga aggaaaagaa guauucccca
ugugcuuggg aggugguccg agcagagauc augaggagcu ucucucucuc aacaaaucug
caggagaguc uuagguccaa ggaguga
SEQ ID NO:22 IFNa2b 
auggcucuca cuuucgcacu ccucguggca cugcuugugc uguccugcaa aaguucuugc
agcguggggu gcgaccuccc acagacccac ucacuggggu caaggcgcac ccugaugcug
cuggcccaga ugagacgaau uucccuguuu uccugccuca aggaucggca ugacuucggc
uuuccccaag aggaguucgg caaccaguuc cagaaagccg agaccauccc ugugcugcau
gagaugaucc agcaaauauu caaucucuuu uccaccaagg acagcuccgc cgccugggau
gagacauugc ucgauaaguu uuauacugaa cuguaccagc agcugaacga ucuugaggcc
uguguaauac aggguguagg cgugacagag accccccuua ugaaggagga cucaauucug
gcagucagga aauauuucca gcggauaacu cuguaccuga aggagaaaaa guauagucca
ugugcuuggg aaguggugcg ggccgagauc augcgcagcu uuucacuuag uacuaaucuc
caggagucuu ugaggucaaa ggaguga
SEQ ID NO:23 IFNa2b 
auggcacuua ccuucgcccu uuugguggca cugcugguac ucucaugcaa gaguaguugc
aguguggggu gcgaucuccc acagacucac uccuugggau caaggcggac gcucaugcuc
cuggcucaaa ugagaagaau uuccuuguuc ucaugcuuga aagauagaca cgacuuuggc
uucccacagg aggaauuugg gaaccaguuc caaaaagcug agacgauccc aguccugcac
gagaugauac agcagaucuu uaaucuuuuu uccaccaagg acagcagugc cgccugggac
gagacucucc uugauaaauu cuacacugaa cucuaucaac aguugaauga cuuggaagcc
ugugugaucc aaggagucgg cgugaccgaa acaccacuua ugaaggagga cagcauccuu
gccguuagaa aguacuuuca acggaucacu cucuaucuca aagagaaaaa guacaguccc
ugugcuuggg aggugguccg cgccgaaauc augaggaguu ucagccucuc cacuaaucuu
caagaauccc uccgaagcaa agaauga
SEQ ID NO:24 IFNa2b 
auggcucuca ccuucgcacu guugguggcu cuccuugugc ugagcuguaa aucuuccugu
ucugucgguu gcgauuugcc gcagacacac agccugggga gucggagaac ccugauguug
cuggcucaga ugcggagaau uucucuguuc aguugccuua aggaccgcca ugauuuuggg
uucccccagg aggaauuugg aaaucaguuu cagaaggcgg agacgauccc gguucugcac
gaaaugaucc agcagaucuu caauuuguuu ucaaccaagg acuccucugc ugccugggau
gaaacacugc uggacaaguu cuauaccgag cuguaccaac agcugaacga ucuugaggca
ugugugauuc aaggagucgg ggucaccgaa accccacuga ugaaagaaga uucuauucug
gcugugagaa aauauuucca aagaauaacu cucuaccuga aggagaagaa auauucacca
ugugccuggg aagucgugcg cgcugagaua augcgcagcu uuagucuuag uacaaaccug
caggagucuc ugcgcucuaa ggaguga
SEQ ID NO:25 IFNa2b 
auggcccuua cuuuugcccu gcugguggca cuucuggucc ucucuugcaa gucaagcugc
aguguuggau gcgaucuucc ucagacacac ucccugggga guagaagaac ucugauguug
cucgcucaga ugcgcagaau aagucuguuu aguugucuga aggaucgcca cgauuucggg
uuuccacagg aagaguucgg caaccaguuc cagaaagcug agacuauccc uguacuucac
gaaaugauuc agcagaucuu uaaucuguuc uccaccaagg acuccucugc cgcuugggau
gagacucucc uggacaaauu uuacacugag cuguaucaac agcugaauga ucuggaagcc
ugcguaaucc aggggguggg ugugacagaa acaccguuga ugaaggaaga cuccauacuu
gcugugcgca aguacuuuca gcggaucacu cuguaucuga aagagaagaa auauucuccu
ugcgcuuggg aggucgucag ggcggaaaua augcggucuu ucagccucuc uaccaaucug
caggaguccc ugagaucuaa ggaauga
SEQ ID NO:26 IFNa2b consensus sequence 
auggcycuba chuuygchcu scusguggcm cukcukgusc uswsyugcaa rwshwsyugy
agygukggvu gcgaycubcc hcagacmcac wsmcugggsw shmgvmgvac ycugaugyug
cusgcycaga ugmgvmgmau hwsycuguuy wsyugycusa aggaycgsca ygayuucggs
uuycchcarg argaguucgg caaccaguuc cagaargcyg agachauccc ygurcukcay
garaugauyc agcaraumuu yaaycusuuy wscaccaagg acwscwsygc cgcyugggay
gagachyusc usgayaaruu yuayacygar cuguaycarc agcugaayga ycukgargcc
ugyguraumc agggbgurgg ygugacmgar acmccsyuka ugaaggarga ywsmauhcuk
gchgusmgsa aruayuuyca gcgsaumacy cuguaycuga argagaaraa ruaywsycch
ugygcyuggg argusgusmg sgcsgaraum augmgvwsyu uywsmcubws yacyaaycus
caggarwsyy ugmgvwshaa rgarura
SEQ ID NO:27 IFNa2b protein 
MALTFALLVA LLVLSCKSSC SVGCDLPQTH SLGSRRTLML LAQMRRISLF SCLKDRHDFG FPQEEFGNQF
QKAETIPVLH EMIQQIFNLF STKDSSAAWD ETLLDKFYTE LYQQLNDLEA CVIQGVGVTE TPLMKEDSIL
AVRKYFQRIT LYLKEKKYSP CAWEVVRAEI MRSFSLSTNL QESLRSKE
SEQ ID NO:28 IFNa1 
auggccucgc ccuuugcuuu acugaugguc cugguggugc ucagcugcaa gucaagcugc
ucucugggcu gugaucuccc ugagacccac agccuggaua acaggaggac cuugaugcuc
cuggcacaaa ugagcagaau cucuccuucc uccugucuga uggacagaca ugacuuugga
uuuccccagg aggaguuuga uggcaaccag uuccagaagg cuccagccau cucuguccuc
caugagcuga uccagcagau cuucaaccuc uuuaccacaa aagauucauc ugcugcuugg
gaugaggacc uccuagacaa auucugcacc gaacucuacc agcagcugaa ugacuuggaa
gccuguguga ugcaggagga gaggguggga gaaacucccc ugaugaaugc ggacuccauc
uuggcuguga agaaauacuu ccgaagaauc acucucuauc ugacagagaa gaaauacagc
ccuugugccu gggagguugu cagagcagaa aucaugagau cccucucuuu aucaacaaac
 uugcaagaaa gauuaaggag gaaggaauaa
SEQ ID NO:29 IFNa1 
auggccucuc cauucgcccu gcugauggug cugguggugc ugagcugcaa gagcagcugc
agccugggcu gcgaccugcc ugagacacac agccuggaca accggcggac ccugaugcug
cuggcccaga ugagcagaau cagccccagc agcugccuga uggaccggca cgauuucggc
uucccucagg aagaguucga cggcaaccag uuccagaagg ccccugccau cagcgugcug
cacgagcuga uccagcagau cuucaaccug uucaccacca aggacagcag cgccgccugg
gacgaggacc ugcuggauaa guucugcacc gaacuguauc agcagcugaa cgaccuggaa
gccugcguga ugcaggaaga gagagugggc gagacacccc ugaugaacgc cgacucuauc
cuggccguga agaaguacuu ucggcggauc acccuguacc ugaccgagaa aaaguacagc
cccugcgccu gggaagucgu gcgggccgag aucaugagaa gccugagccu gagcaccaac
cugcaggaac ggcugcggcg gaaagaguaa
SEQ ID NO:30 IFNa1 
auggccagcc ccuucgcccu gcugauggug cugguggugc ugagcugcaa gagcagcugc
agccugggcu gcgaccugcc cgagacccac agccuggaca accgccgcac ccugaugcug
cuggcccaga ugagccgcau cagccccagc agcugccuga uggaccgcca cgacuucggc
uucccccagg aggaguucga cggcaaccag uuccagaagg cccccgccau cagcgugcug
cacgagcuga uccagcagau cuucaaccug uucaccacca aggacagcag cgccgccugg
gacgaggacc ugcuggacaa guucugcacc gagcuguacc agcagcugaa cgaccuggag
gccugcguga ugcaggagga gcgcgugggc gagacccccc ugaugaacgc cgacagcauc
cuggccguga agaaguacuu ccgccgcauc acccuguacc ugaccgagaa gaaguacagc
cccugcgccu gggagguggu gcgcgccgag aucaugcgca gccugagccu gagcaccaac
cugcaggagc gccugcgccg caaggaguaa
SEQ ID NO:31 IFNa1 
auggccucuc ccuucgcuuu gcugaugguu cucgugguuc ucagcugcaa guccuccugu
agucuggggu gugaccuucc cgagacucac agccuugaca accgacggac ucugaugcug
cuggcccaga ugagucgcau auccccuucu ucuugcuuga uggauagaca cgauuucggc
uucccucagg aggaguucga ugggaaccaa uuccagaaag cgccugcgau cagcguacuc
caugagcuga uccagcagau cuuuaauuug uucacaacga aggacagcag ugcugcuugg
gacgaagacc ugcuggacaa guucuguaca gaauuguacc agcagcugaa ugaccucgag
gccuguguga ugcaggagga aagagucggc gagacuccuc ucaugaacgc cgacagcauc
cucgccguga agaaguauuu ccggcggauc acccucuauc ugacagagaa gaaguacucc
cccugcgccu gggagguggu gcgagccgaa auaaugcgca gccucucucu gucaacuaau
cuccaggagc ggcuucggcg aaaggaguga
SEQ ID NO:32 IFNa1 auggcaucuc cauucgcucu gcugauggug cugguggucc ugucauguaa gagcagcugc 
ucccuggggu gcgaucugcc agagacccac agccuugaca acagaagaac cuugaugcuc
uuggcccaaa ugucaagaau aagcccuagc ucaugucuga uggaccggca ugauuucggc
uuuccgcagg aggaguucga cggaaaucag uuucagaaag caccagcaau aagcgugcuc
cacgaacuca uucagcagau uuucaaucug uuuacuacaa aagauucauc cgcugcuugg
gaugaagacu ugcuugacaa guucugcacu gagcucuacc aacaacugaa cgaucuggag
gcaugcguca ugcaagagga gagagugggg gaaacccccc ugaugaacgc ugauuccaua
cuggccguga agaaauauuu ccgcagaauc acucuguacu ugacugagaa gaaguauucu
cccugcgcuu gggagguggu gcgcgcugag auaaugcggu ccuugucacu cagcaccaau
cuucaagagc ggcugaggcg caaggaauga
SEQ ID NO:33 IFNa1 
auggccucuc ccuucgcucu ucucaugguc cuugucguuc ugaguuguaa gagcaguugu
uccuugggcu gugacuugcc cgaaacucac agucuggaca accgccgcac gcucaugcuc
cucgcccaga ugucacggau cagcccguca aguugccuga uggacaggca cgauuucggg
uucccacagg aagaguucga ugguaaucaa uuccaaaaag cuccugcaau uucuguacug
caugaacuua uucagcagau cuucaaccuc uuuaccacua aagauuccuc ugccgccugg
gaugaggauu ugcucgauaa guucugcacc gaacucuacc agcagcugaa cgaccucgag
gcuuguguua ugcaggaaga gcgggugggg gagacacccc ucaugaacgc cgacagcauu
cuggccguga aaaaguacuu uagaagaaua acgcuguacc ucaccgaaaa gaaauacucc
cccugcgcuu gggaggucgu gcgcgccgag auaaugcgcu cacucucuuu gagcacaaau
cuccaagaac ggcugaggag aaaagaguga
SEQ ID NO:34 IFNa1 
auggcaucuc cuuucgcucu gcuuauggug cugguugugc ucucuugcaa guccagcugu
agccuuggau gcgaccuucc ugagacccau ucucuggaua accggaggac gcucaugcug
cuggcacaga ugagucggau cagcccgucc agcugucuca uggacaggca cgacuuuggu
uuuccccagg aggaguucga ugguaaccag uuccagaagg cgccagcaau aagcgugcug
cacgagcuga uucagcagau cuuuaaccuc uucacuacua aggacaguag cgcugcaugg
gacgaggauc uguuggauaa guucuguacg gaacuguauc agcagcucaa ugaucucgaa
gcauguguga ugcaagaaga gcgcgucggu gaaacuccac ugaugaacgc ugauagcauc
cuggccguua agaaauacuu uaggcgcauu acucuguauc ugacugagaa aaaguauagc
ccaugcgcau gggagguagu gcgagccgag aucaugcgca gccugucauu gucaacuaac
cuccaggaac gccuccgacg aaaggaguga
SEQ ID NO:35 IFNa1 
auggcaucac ccuucgcucu gcugaugguc cugguggugc ugucauguaa auccagcugc
agcuugggau gcgaccugcc ggaaacacau ucccucgaca auaggcggac gcugaugcug
cucgcucaga ugucccgcau aagcccauca agcugccuca uggaccggca cgauuuuggu
uucccacagg aggaguucga uggaaaccag uuccagaagg cucccgcuau cuccguucug
caugagcuua uucagcagau uuucaaccuc uuuacgacaa aggauucauc cgccgccugg
 
gaugaagacc ugcuggauaa guucuguacc gaguuguacc aacagcugaa cgaucucgaa
gccuguguca ugcaggagga gcgcgugggg gagacucccu ugaugaacgc agacucaauu
cuugcaguga agaaauacuu ucgccggauu acucuuuauc ucaccgagaa gaaguacagu
cccugcgcau gggaagucgu ucgggccgaa aucaugcggu cccugucccu uuccaccaac
cugcaggaac gccuucgcag aaaagaguaa
SEQ ID NO:36 IFNa1 consensus sequence 
auggcmwshc chuucgcyyu gcukauggub cusgukgubc uswshugyaa rwscwscugy
wsyyukggvu gygaycukcc ngarachcay wsycubgaya aymgvmgvac byusaugcus
yusgchcara ugwshmgvau mwscccnwsh wshugyyusa uggaymgvca ygayuuyggy
uuyccncagg argaguucga yggnaaycar uuycagaarg cncchgcnau mwscgudcus
caygarcuba uycagcagau yuuyaayyus uuyacnacna argaywshws ygcygchugg
gaygargayy ugyukgayaa guucugyacn garyusuayc arcarcusaa ygaycusgar
gcmugygusa ugcargarga rmgmgusggb garachcchy usaugaacgc hgaywshauh
cubgcmguka agaaruayuu ymgsmgvauy acycubuayy usachgagaa raaguaywsy
ccmugcgchu gggarguvgu kcgvgcygar aumaugmgvw scyuswshyu bwsmacyaay
cubcargarc gscubmgvmg vaargarura
SEQ ID NO:37 IFNa1 protein 
MASPFALLMV LVVLSCKSSC SLGCDLPETH SLDNRRTLM LLAQMSRISP SSCLMDRHDF
GFPQEEFDGN QFQKAPAISV LHELIQQIFN LFTTKDSSAA WDEDLLDKFC TELYQQLNDL
EACVMQEERV GETPLMNADS ILAVKKYFRR ITLYLTEKKY SPCAWEVVRA EIMRSLSLST
NLQERLRRKE
SEQ ID NO:38 general formula stabilisation sequence yccancccwn ucycc
SEQ ID NO:39
   agcgtggctg tctcctctc
SEQ ID NO:40
   gagccttgaa tacagcaggc
SEQ ID NO:41
   gcttgggatg agaccctcct a
SEQ ID NO:42
   cccaccccct gtatcacac
SEQ ID NO:43
   cacgagatga tccagcagat
SEQ ID NO:44
   cttgtccagc agtgtctcgt
SEQ ID NO:45
   ctgctctgtt ggctgtgatt
SEQ ID NO:46
   caggcatgag aacaggctaa
SEQ ID NO:47
   tgatgcttct tgcacaaatg
SEQ ID NO:48
   aggacaggaa tggtttcagc
SEQ ID NO:49
   caaggagtcg gagtgactga
SEQ ID NO:50
   cagggtgat cctctggaag t
SEQ ID NO:51
   ggctgtattc ccctccatcg
SEQ ID NO:52
   ccagttggta acaatgccatg t
Seq ID NO:53 native IFNa2a including native IFNa2a UTRs at 5' and 3' ends; first A of Poly A tail included (poly A 120nts)

gggagaugag ccuaaaccuu aggcucaccc auuucaacca gucuagcagc aucugcaaca
ucuacaaugg ccuugaccuu ugcuuuacug guggcccucc uggugcucag cugcaaguca
agcugcucug ugggcuguga ucugccucaa acccacagcc uggguagcag gaggaccuug
augcuccugg cacagaugag gaaaaucucu cuuuucuccu gcuugaagga cagacaugac
uuuggauuuc cccaggagga guuuggcaac caguuccaaa aggcugaaac caucccuguc
cuccaugaga ugauccagca gaucuucaau cucuucagca caaaggacuc aucugcugcu
ugggaugaga cccuccuaga caaauucuac acugaacucu accagcagcu gaaugaccug
gaagccugug ugauacaggg ggugggggug acagagacuc cccugaugaa ggaggacucc
auucuggcug ugaggaaaua cuuccaaaga aucacucucu aucugaaaga gaagaaauac

agcccuugug ccugggaggu ugucagagca gaaaucauga gaucuuuuuc uuugucaaca
aacuugcaag aaaguuuaag aaguaaggaa ugaaaacugg uucaacaugg aaaugauuuu
cauuaauucg uaugccagcu caccuuuuua ugaucugcca uuucaaagac ucauguuucu
gcuaugacca ugacacgauu uaaaucuuuu ucaaauguuu uuaggaguau uaaucaacau
uguauucagc ucuuaaggca cuagucccuu acagaggacc augcugacug auccauuauc
uauuuaaaua uuuuuaaaau auuauuuauu uaacuauuua uaaaacaacu uauuuuuguu
cauauuacgu caugugcacc uuugcacagu gguuaaugua auaaaaua

The consensus sequences SEQ ID NOs:12, 13, 14, 26 and 36 are given with the following IUPAC nomenclature:

**IUPAC Nomenclature:**

| **Symbol** | **Description** | **Bases represented** | | | | |
|---|---|---|---|---|---|---|
| **A** | **A**denine | A | | | | |
| **C** | **C**ytosine | | C | | | |
| **G** | **G**uanine | | | G | | 1 |
| **T** | **T**hymine | | | | T | |
| **U** | **U**racil | | | | U | |
| **W** | **W**eak | A | | | T | |
| **S** | **S**trong | | C | G | | |
| **M** | a**M**ino | A | C | | | |
| **K** | **K**eto | | | G | T | 2 |
| **R** | pu**R**ine | A | | G | | |
| **Y** | p**Y**rimidine | | C | | T | |
| **B** | not A (**B** comes after A) | | C | G | T | |
| **D** | not C (**D** comes after C) | A | | G | T | |
| **H** | not G (**H** comes after G) | A | C | | T | 3 |
| **V** | not T (**V** comes after T and U) | A | C | G | | |
| **N** | any **N**ucleotide (not a gap) | A | C | G | T | 4 |
| **Z** | **Z**ero | | | | | 0 |

**Table 1: Codon Adaptation Index and GC content of IFNa2a variants (SEQ ID NO:1 is the standard for expression; SEQ ID NOs:2, 3 and 5: higher; SEQ ID NO:4: lower; it is therefore preferred to have a CAI ≥ 0,8 and a GC content ≥49,5%; compared to the native sequences SEQ ID NOs:1, 18 and 28)**

| Sequence ID | CAI (Human/HEK) | GC Content % |
|---|---|---|
| SEQ ID NO:1 | 0.8 | 48.7 |
| SEQ ID NO:2 | 0.8 | 49.9 |
| SEQ ID NO:3 | 0.84 | 56.8 |
| SEQ ID NO:4 | 0.69 | 40.4 |
| SEQ ID NO:5 | 0.97 | 60 |
| SEQ ID NO:6 | 0.78 | 50.1 |
| SEQ ID NO:7 | 0.8 | 51 |
| SEQ ID NO:8 | 0.81 | 51,3 |
| SEQ ID NO:9 | 0.84 | 50.8 |
| SEQ ID NO:10 | 0.8 | 50.6 |
| SEQ ID NO:11 | 0.99 | 63.7 |

**Table 2: Codon Adaptation Index and GC content of IFNa2b variants (preferred CAI and GC as for table 1 also applies here)**

| Sequence ID | CAI (Human/HEK) | GC Content % |
|---|---|---|
| SEQ ID NO:18 | 0.8 | 48.9 |
| SEQ ID NO:19 | 0.99 | 64 |
| SEQ ID NO:20 | 0.97 | 60 |
| SEQ ID NO:21 | 0.78 | 49.7 |
| SEQ ID NO:22 | 0.82 | 51.9 |
| SEQ ID NO:23 | 0.75 | 48.7 |
| SEQ ID NO:24 | 0.79 | 49.2 |
| SEQ ID NO:25 | 0.8 | 49.2 |

**Table 3: Codon Adaptation Index and GC content of IFNa1 variants (preferred CAI and GC as for table 1 also applies here)**

| Sequence ID | CAI (Human/HEK) | GC Content % |
|---|---|---|
| SEQ ID NO:28 | 0,8 | 49,5 |
| SEQ ID NO:29 | 0,97 | 60,3 |
| SEQ ID NO:30 | 0,99 | 64,9 |
| SEQ ID NO:31 | 0,81 | 54,4 |
| SEQ ID NO:32 | 0,8 | 50,9 |
| SEQ ID NO:33 | 0,78 | 51,4 |
| SEQ ID NO:34 | 0,8 | 51,9 |
| SEQ ID NO:35 | 0,81 | 53,2 |

The invention is further explained by way of the following examples and the figures, yet without being limited thereto.

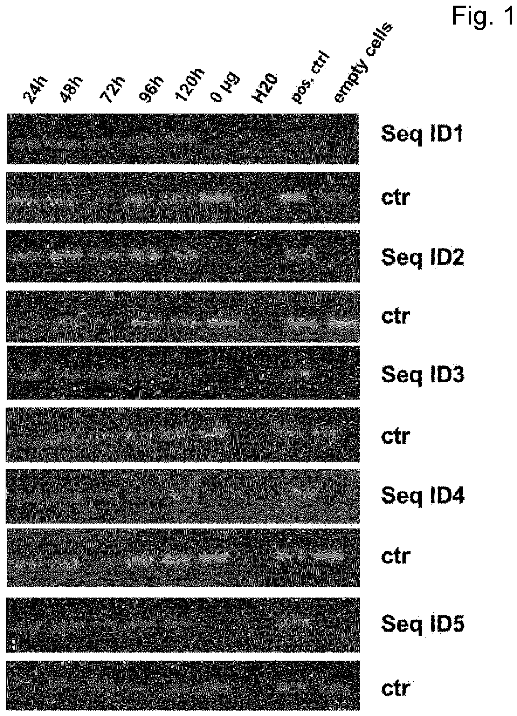

Figure 1 shows the RT-PCR based detection of IVT mRNA 24-120h post transfection of human BJ cells (24-120h: samples taken 24-120h post transfection; 0µg: cells were treated with TransIT only and harvested 24h post transfection; H2O: negative control containing no cDNA.; pos. Control: cDNA from cellular RNA and IVT mRNA variants; empty cells: non-transfected BJ fibroblasts).

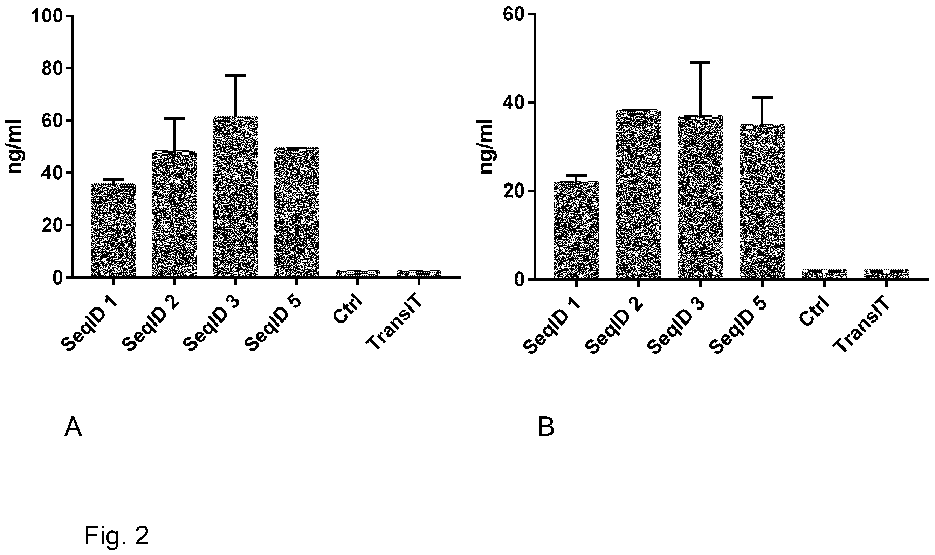

Figure 2 shows that IVT mRNA transfection of codon and GC content optimized mRNA variants induces increased human IFNa2a protein expression in human BJ fibroblasts (SEQ ID NO: respective IFNa2 mRNA sequence complexed with TransIT; TransIT: TransIT mRNA transfection reagent only; ctrl: buffer only; A: analysis at 24h post transfection; B: analysis at 72h post transfection) .

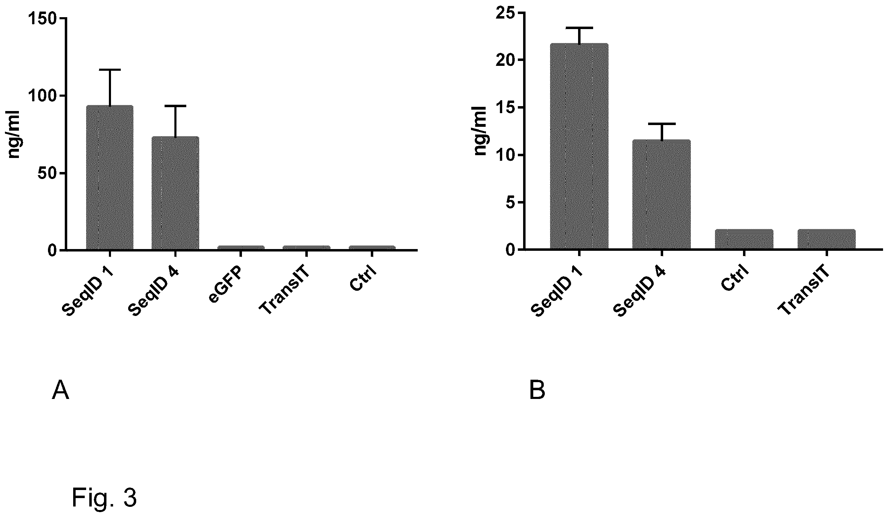

Figure 3 shows that IVT mRNA transfection of codon and AU content optimized mRNA variants induces low human IFNa2a protein expression in human BJ fibroblasts (SeqID: respective IFNa2 mRNA sequence complexed with TransIT; TransIT: TransIT mRNA transfection reagent only; ctrl: buffer only; A: analysis at 24h post transfection; B: analysis at 72h post transfection).

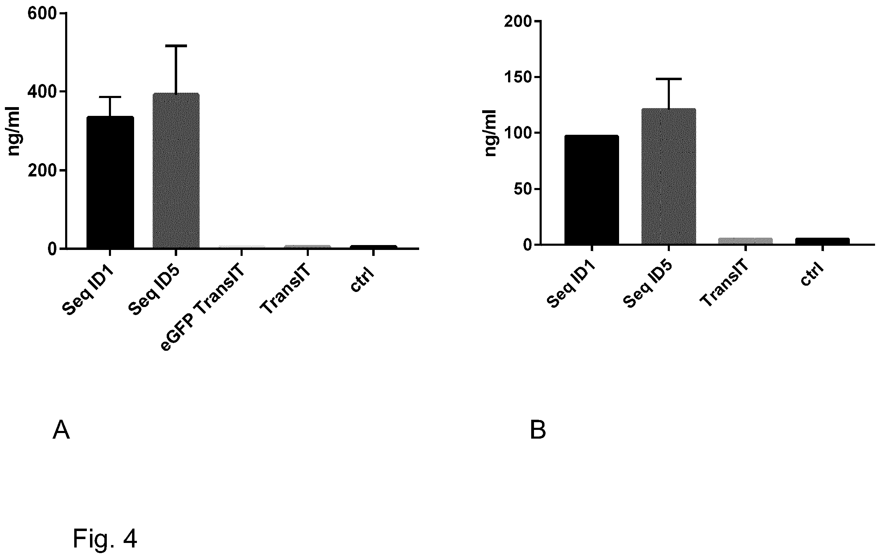

Figure 4 shows that IVT mRNA transfection of codon and GC content optimized mRNA variants induces increased human IFNa2a protein expression in porcine skin epithelial sheets (SeqID: respective IFNa2 mRNA sequence complexed with TransIT; TransIT: TransIT mRNA transfection reagent only; ctrl: buffer only; A: analysis at 24h post transfection; B: analysis at 48h post transfection).

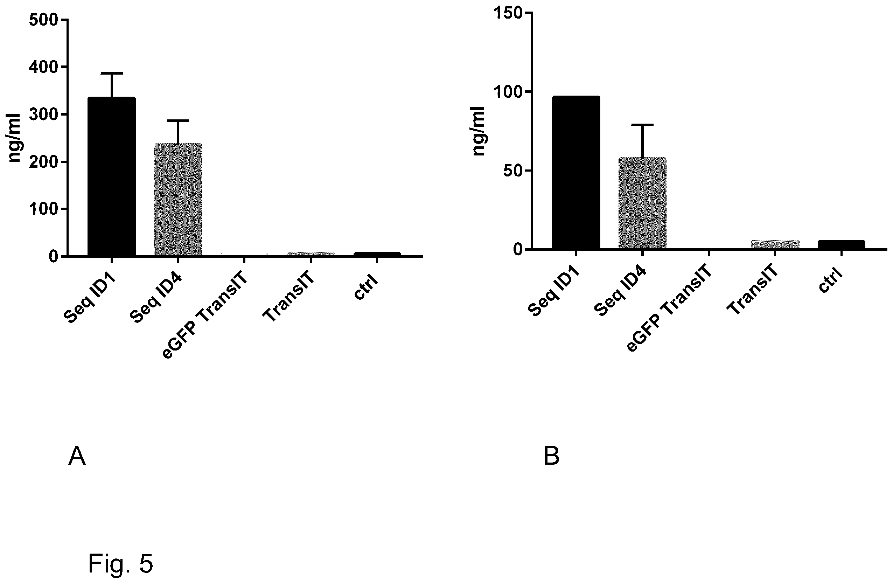

Figure 5 shows that IVT mRNA transfection of codon and AU content optimized mRNA variants induces low human IFNa2a protein expression in porcine skin epithelial sheets (SeqID: respective IFNa2 mRNA sequence complexed with TransIT; TransIT: TransIT mRNA transfection reagent only; ctrl: buffer only; A: analysis at 24h post transfection; B: analysis at 48h post transfection).

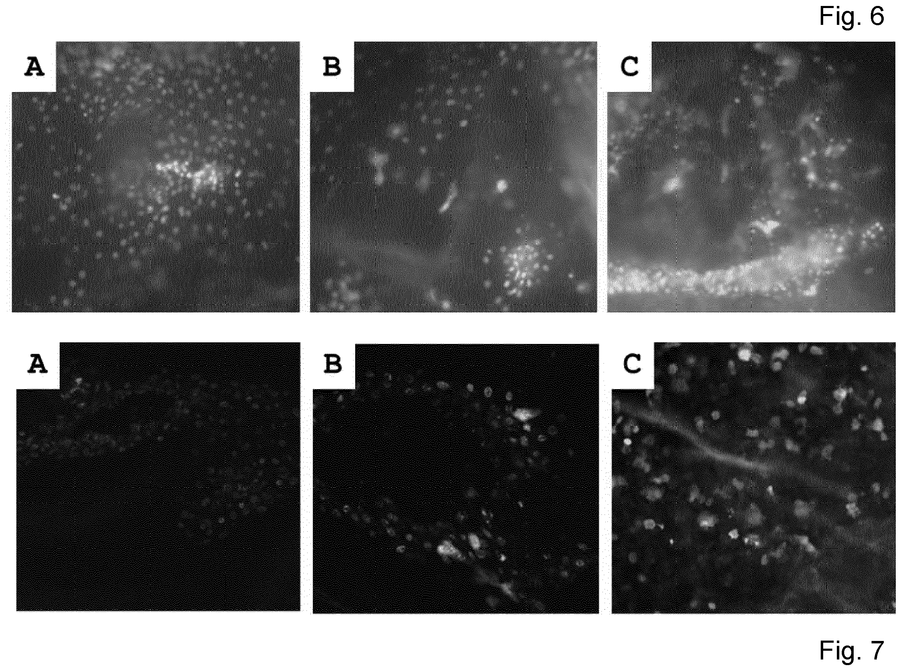

Figure 6 shows that EGFP mRNA transfection of porcine epithelial sheets using TransIT mRNA transfection reagent induces eGFP expression in porcine skin epithelial sheets (A: porcine skin transfected with liposomes only; B: porcine skin transfected with 0,5µg/ml eGFP IVTm RNA, formulated in TransIT; C: porcine skin transfected with 1µg/ml eGFP IVTm RNA, formulated in TransIT).
Figure 7 shows that EGFP mRNA transfection of porcine epithelial sheets using mRNA/Liposome complexes induces eGFP expression in porcine skin epithelial sheets (A: porcine skin transfected with liposomes only; B: porcine skin transfected with 2µg/ml eGFP IVTm RNA, formulated in liposomes; C: porcine skin transfected with 10µg/ml eGFP IVTm RNA, formulated in liposomes) .

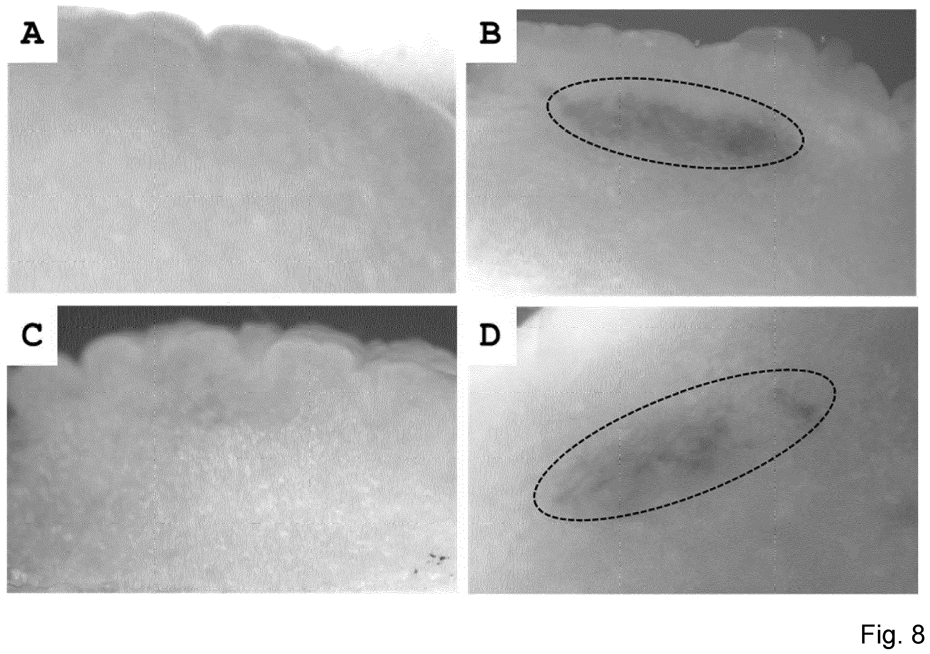

Figure 8 shows the detection of whole mount β-Galactosidase (bGal) activity in porcine skin explants 24h after transfection with LacZ IVT mRNA (A: porcine skin transfected with DOTAP-liposomes only w/o Rnase inhibitor; B: porcine skin transfected with 5µg LacZ IVTm RNA, formulated in DOTAP-liposomes w/o Rnase inhibitor; C: porcine skin transfected with DOTAP-liposomes only + Rnase inhibitor; D: porcine skin transfected with 5µg LacZ IVTm RNA, formulated in DOTAP-liposomes w/o Rnase inhibitor; Succesful transfection is highlighted in encircled areas in B and D, respectively).

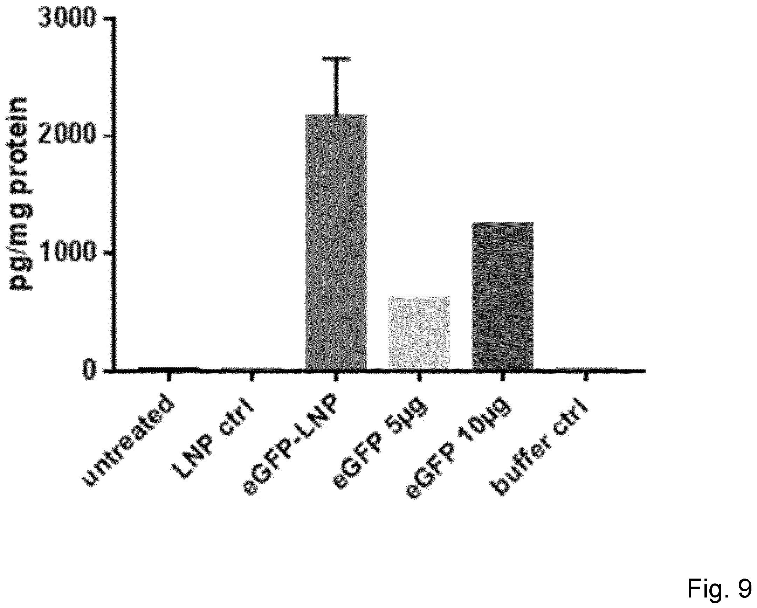

Figure 9 shows the detection of eGFP expression in porcine skin explants 24h after transfection with eGFP IVT mRNA (untreated: non-treated biopsy; LNP ctrl: porcine skin LNP control treated; eGFP-LNP: porcine skin transfected with mRNA-Lipid-Nano Particles (concentration shown: 2.4µg eGFP mRNA/dose); eGFP 5µg and eGFP 10µg: porcine skin transfected with non-complexed eGFP IVT-mRNA (concentrations shown: 5+10µg mRNA/dose); buffer ctrl porcine skin treated with buffer only).

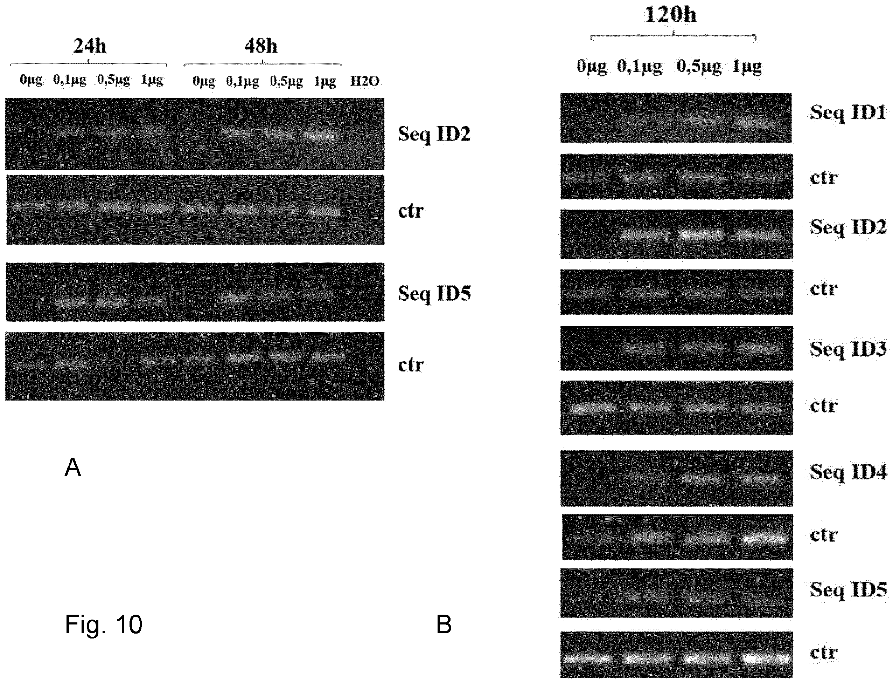

Figure 10 shows the detection of IVT mRNA 24-120h post transfection of murine 3T3 cells (24-120h: samples taken 24-120h post transfection; 0,1-1µg: mRNA doses used for transfection; 0µg: cells were treated with TransIT only and harvested 24h post transfection; H2O: negative control containing no cDNA.; ctr: RT PCR control using murine ACTB (muACTB) as control/reference gene).

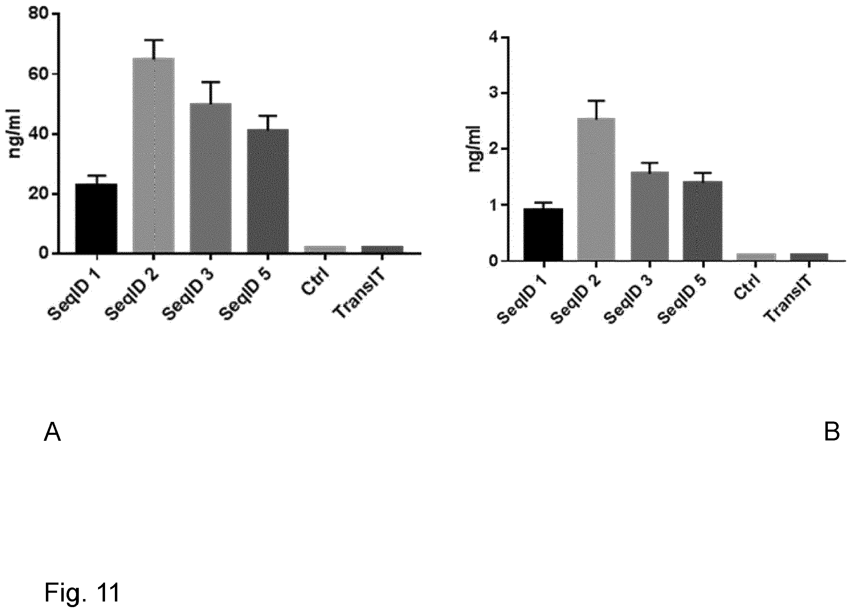

Figure 11 shows that IVT mRNA transfection of codon and GC content optimized mRNA variants induces increased human IFNa2a protein expression in murine 3T3 fibroblasts (SEQ ID NO: respective IFNa2 mRNA sequence complexed with TransIT; TransIT: TransIT mRNA transfection reagent only; ctrl: buffer only; A: analysis at 96h post transfection; B: analysis at 120h post transfection) .

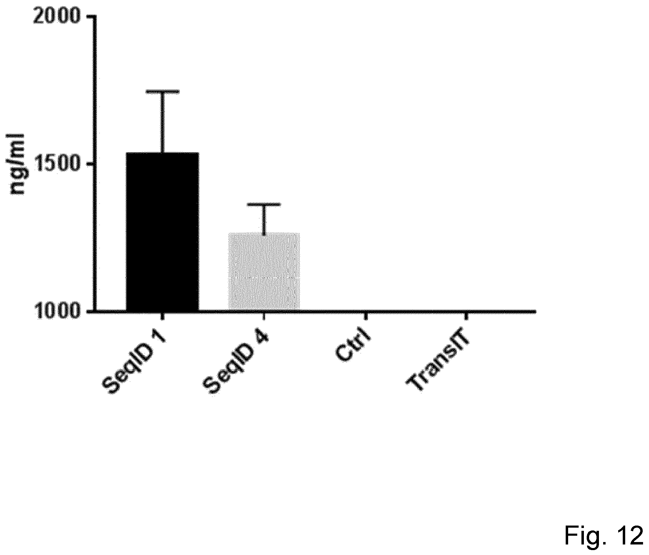

Figure 12 shows that IVT mRNA transfection of codon and AU content optimized mRNA variants induces low human IFNa2a protein expression in murine 3T3 fibroblasts at 24h post transfection (SeqID: respective IFNa2 mRNA sequence complexed with TransIT; TransIT: TransIT mRNA transfection reagent only; ctrl: buffer only).

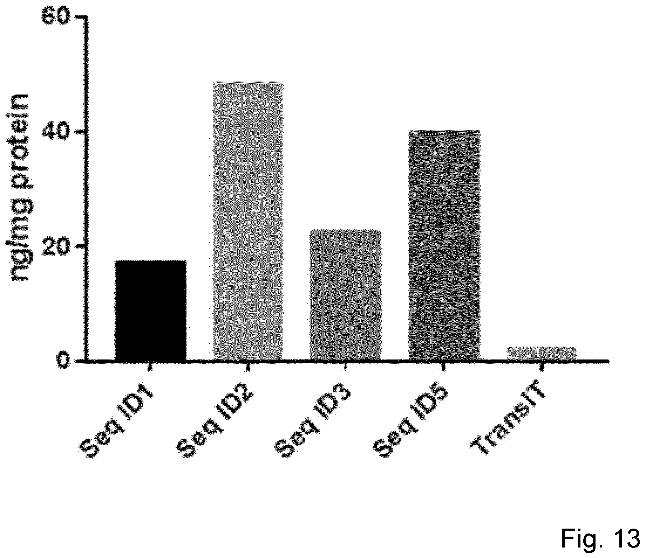

Figure 13 shows that IVT mRNA transfection of codon and GC content optimized mRNA variants induces increased human IFNa2a protein expression in porcine skin epithelial sheets 48h post transfection (SeqID: respective IFNa2 mRNA sequence complexed with TransIT; TransIT: TransIT mRNA transfection reagent only;).

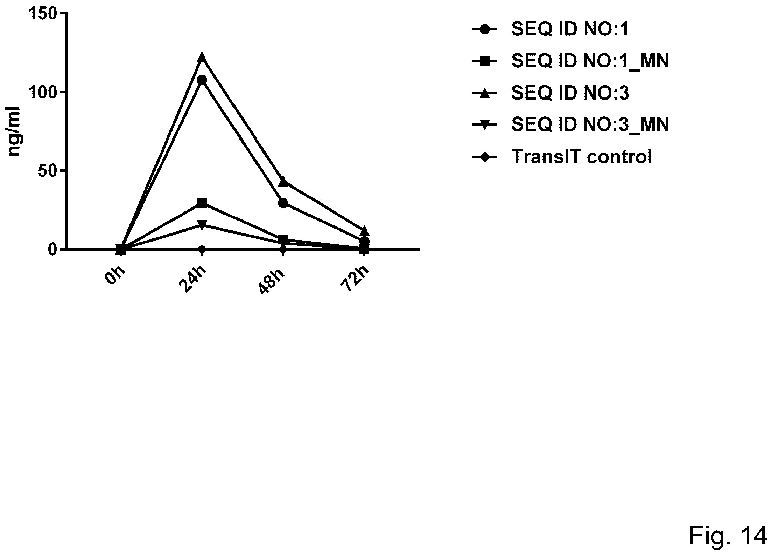

Figure 14 shows that IVT mRNAs which have 100% replacement of Pseudo-U for U and 5mC for C induce differential human IFNa2a protein expression in porcine epithelial sheets 24-72h post transfection (SEQ ID NO: respective IFNa2 mRNA sequence complexed with TransIT; only non-modified nucleotides used for in vitro transcription; SEQ ID NO1_MN/SEQ ID NO:3_MN: mRNA containing full replacement of Pseudo-U for U and 5mC for C).

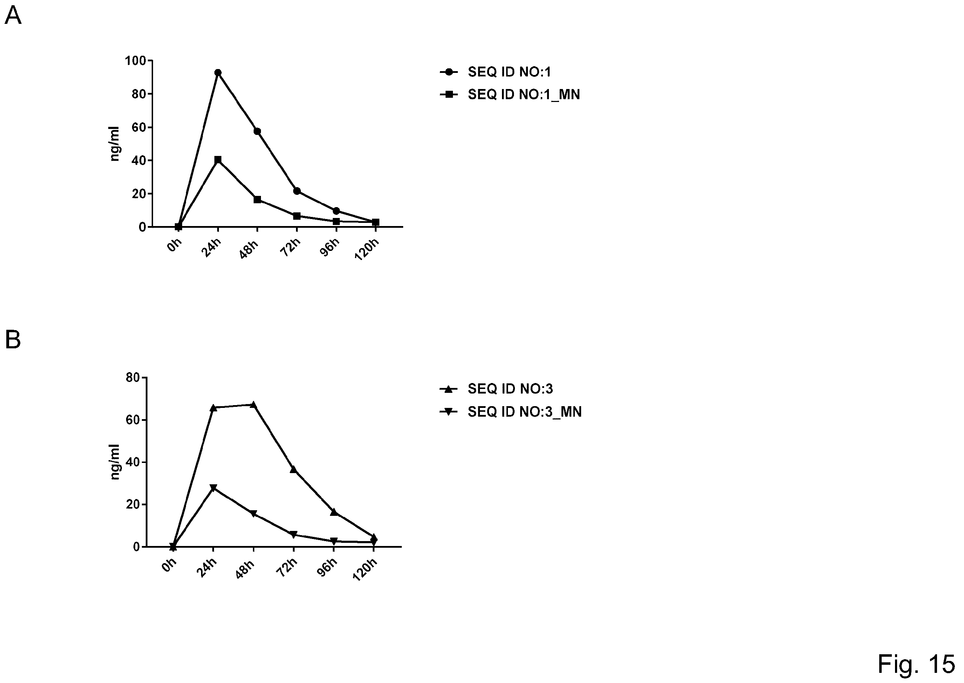

Figure 15 shows that IVT mRNAs which have 100% replacement of Pseudo-U for U and 5mC for C induce differential human IFNa2a protein expression in human BJ fibroblasts 24-120h post transfection (SEQ ID NO: respective IFNa2 mRNA sequence complexed with TransIT; only non-modified nucleotides used for in vitro transcription; SEQ ID NO1_MN/SEQ ID NO:3_MN: mRNA containing full replacement of Pseudo-U for U and 5mC for C). A) comparison of IVT mRNAs SEQ ID NO1 and SEQ ID NO1_MN; B) comparison of of IVT mRNAs SEQ ID NO3 and SEQ ID NO3_MN;

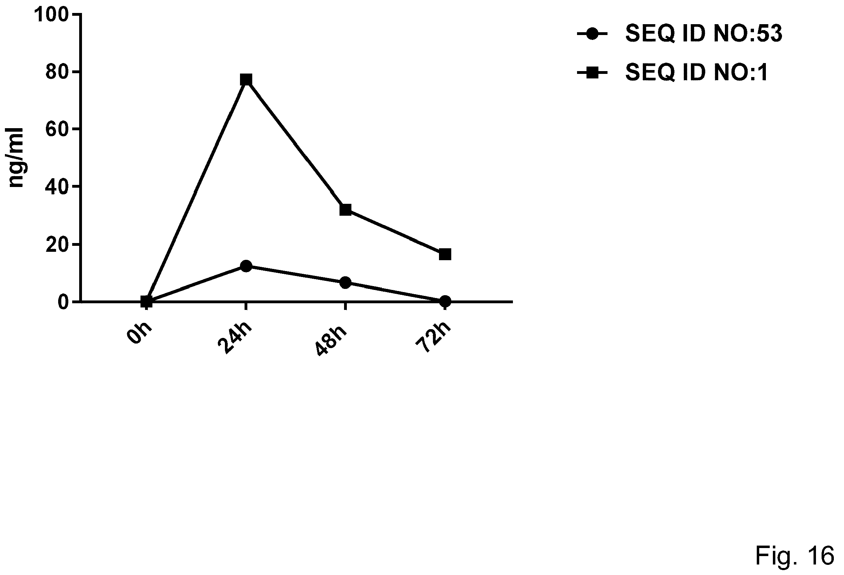

Figure 16 shows that IVT mRNA transfection of codon and GC content optimized mRNA variants induces increased human IFNa2a protein expression in human BJ cells 24h to 72h post transfection (SeqID: respective IFNa2 mRNA sequence complexed with TransIT).

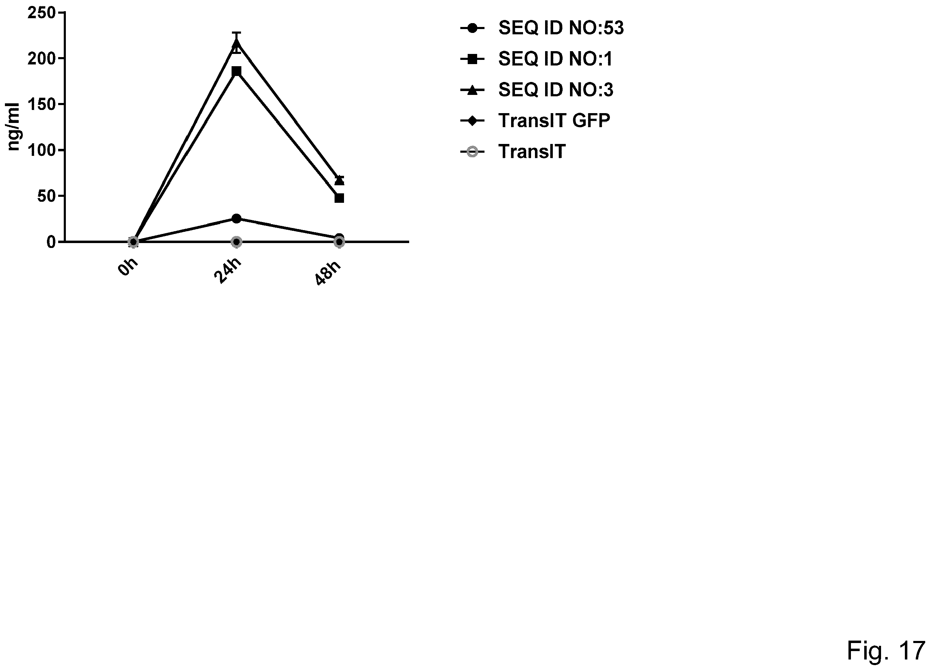

Figure 17 shows that IVT mRNA transfection of codon and GC content optimized mRNA variants induces increased human IFNa2a protein expression in porcine skin epithelial sheets 24h and 48h post transfection (SeqID: respective IFNa2 mRNA sequence complexed with TransIT; TransIT: TransIT mRNA transfection reagent only;).

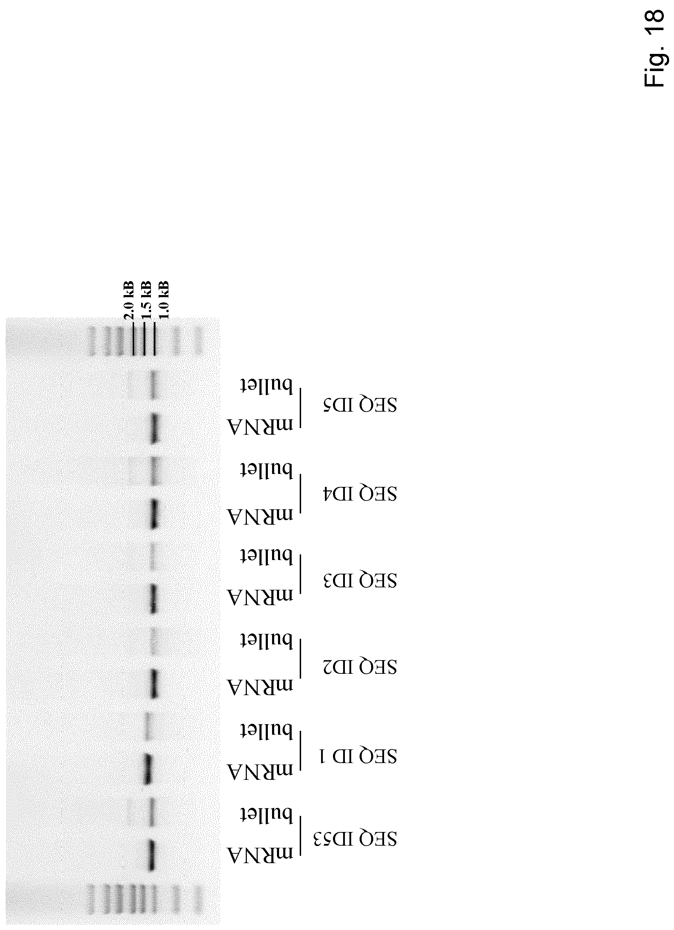

Figure 18 shows control of loading of with IVT mRNA coated gold particles (1.6µm gold microcarriers loaded with 1µg/µl IVT-mRNA) using conventional agarose gel electrophoresis. Comparable IVT mRNA amounts have been immobilized on gold particles.

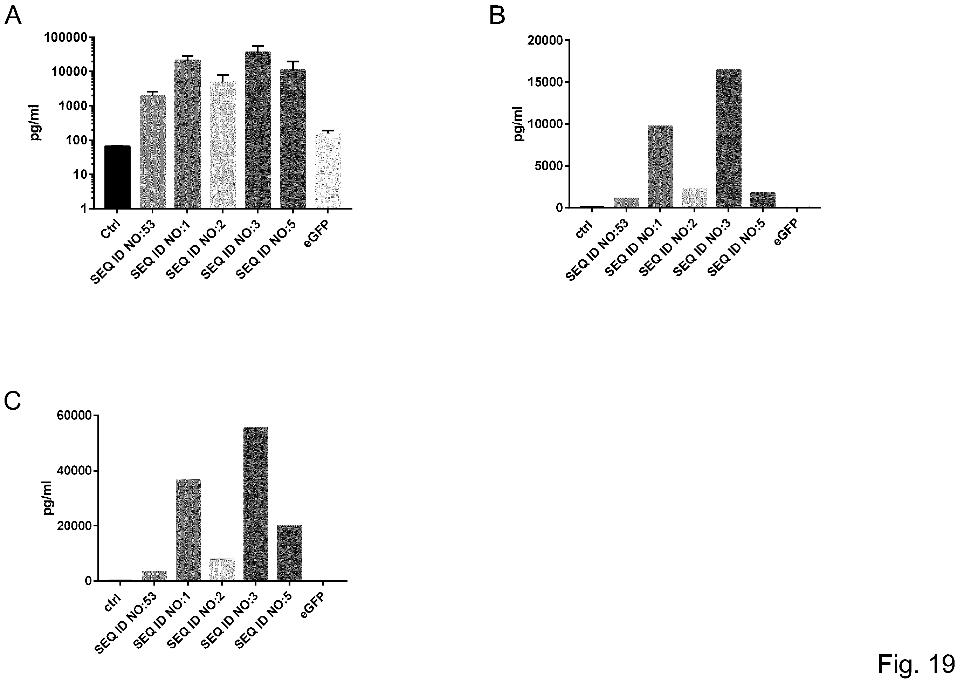

Figure 19 shows that biolistic IVT mRNA transfection of codon and GC content optimized mRNA variants induces increased human IFNa2a protein secretion from human skin 24h post transfection (SeqID: respective IFNa2 mRNA sequence coated gold particles; CTRL medium from untransfected skin; eGFP: medium from eGFP coated gold particle treated skin;). Results are shown as average +/- SEM; A) average of 5 five human donors; B) example of individual donor #1 (value: pooled supernatant from 3 biopsies); C) example of individual donor #2 (value: pooled supernatant from 3 biopsies)

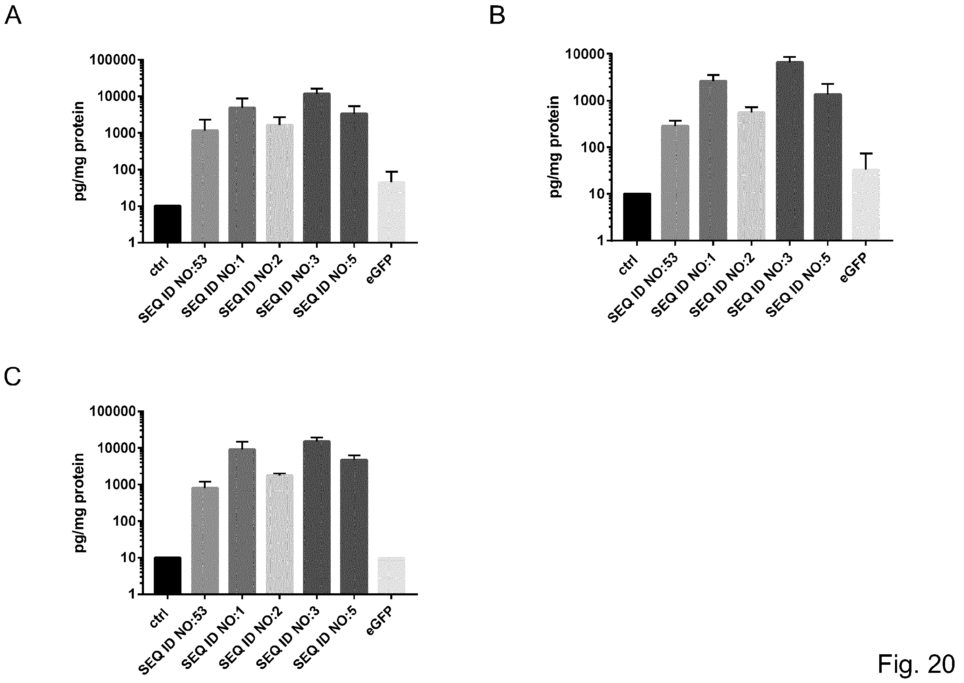

Figure 20 shows that biolistic IVT mRNA transfection of codon and GC content optimized mRNA variants induces increased human IFNa2a protein expression in human skin 24h post transfection (SeqID: respective IFNa2 mRNA sequence coated gold particles; CTRL extract from untransfected skin; eGFP: extract from eGFP coated gold particle treated skin;). Results are shown as average +/- SEM; A) average of 5 five human donors; B) example of individual donor #1 (average from 3 biopsies); C) example of individual donor #2 (average from 3 biopsies)

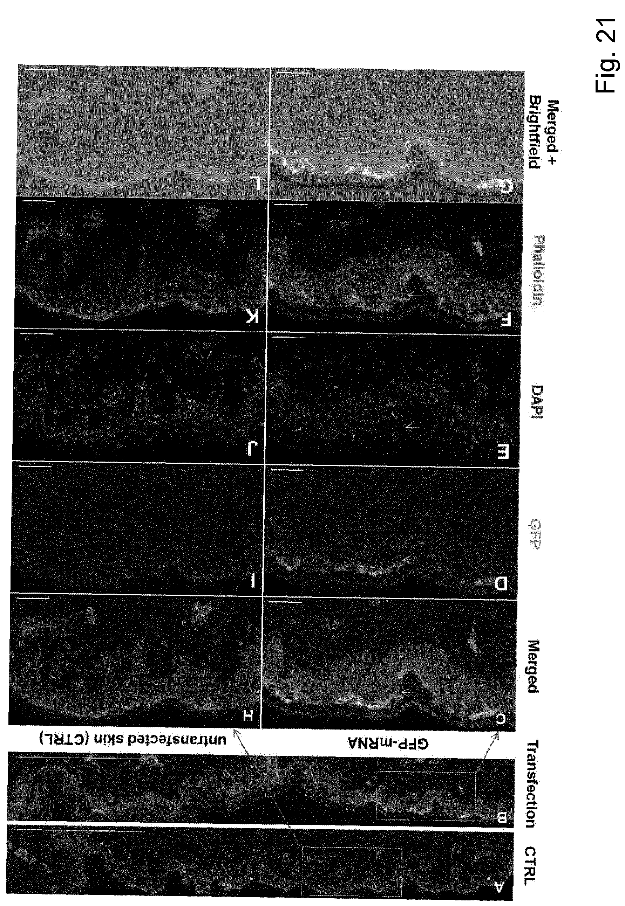

Figure 21 shows that biolistic IVT mRNA transfection leads to epidermal protein expression induced by the IVT mRNA used. eGFP expression can be detected by anti eGFP immunohistochemistry on cryosections. (A, H, I, J, K, L) Untransfected control biopsies. (B, C, D, E, F, G) Biopsies treated with 1µg/µl eGFP-mRNA.

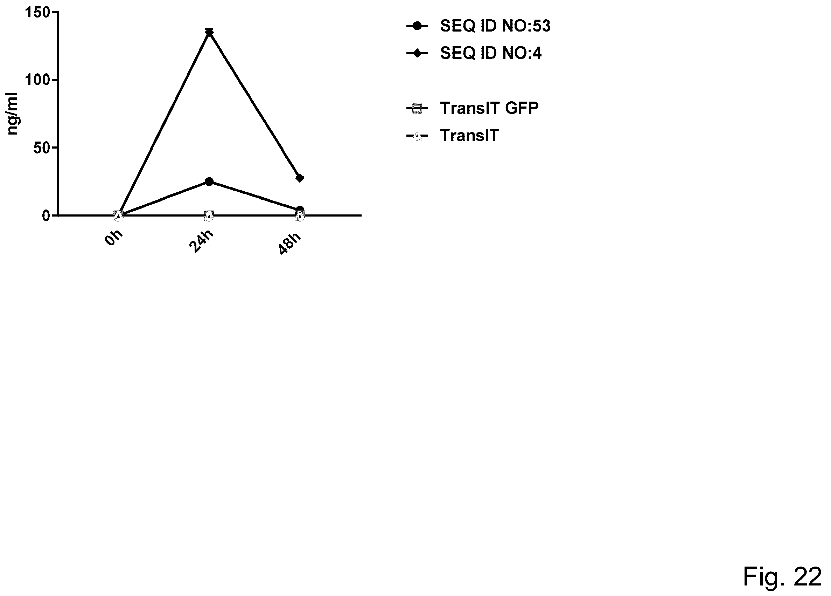

Figure 22 shows that IVT mRNA transfection of codon and AU content optimized mRNA variants induces increased human IFNa2a protein expression in porcine skin epithelial sheets 24h and 48h post transfection (SeqID: respective IFNa2 mRNA sequence complexed with TransIT; TransIT: TransIT mRNA transfection reagent only;).

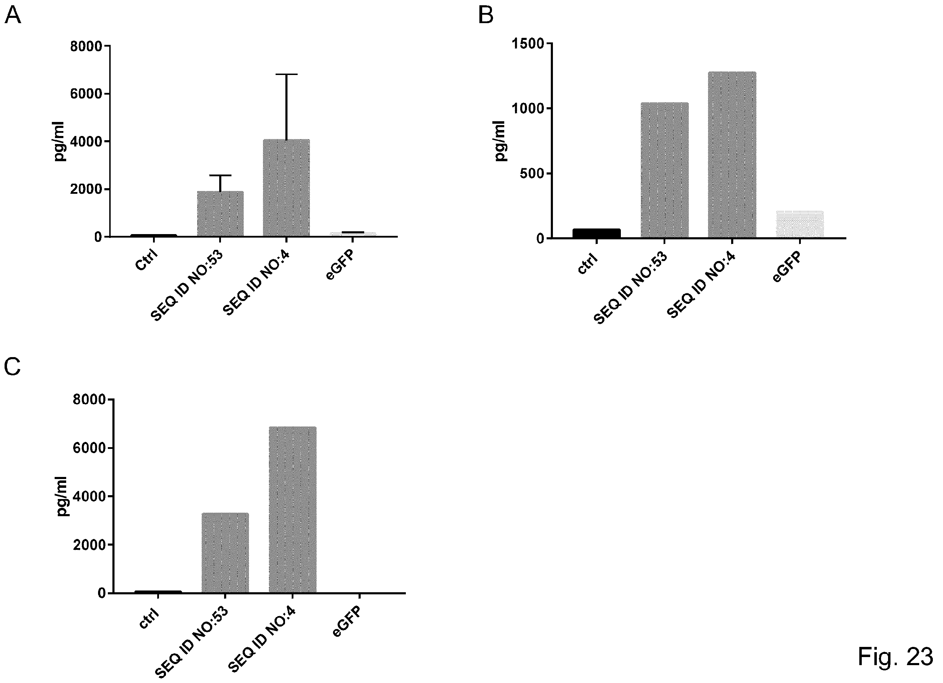

Figure 23 shows that biolistic IVT mRNA transfection of codon and AU content optimized mRNA variants induces increased human IFNa2a protein secretion from human skin 24h post transfection (SeqID: respective IFNa2 mRNA sequence coated gold particles; CTRL medium from untransfected skin; eGFP: medium from eGFP coated gold particle treated skin;). Results are shown as average +/- SEM; A) average of 5 five human donors; B) example of individual donor #1 (value: pooled supernatant from 3 biopsies); C) example of individual donor #2 (value: pooled supernatant from 3 biopsies)

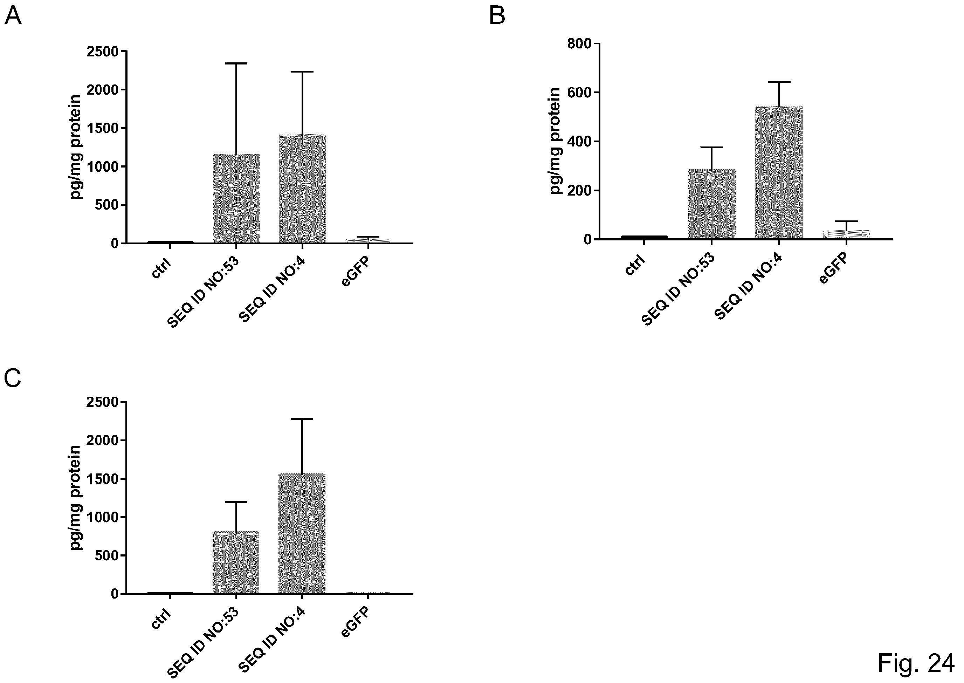

Figure 24 shows that biolistic IVT mRNA transfection of codon and AU content optimized mRNA variants induces increased human IFNa2a protein expression in human skin 24h post transfection (SeqID: respective IFNa2 mRNA sequence coated gold particles; CTRL extract from untransfected skin; eGFP: extract from eGFP coated gold particle treated skin;). Results are shown as average +/- SEM; A) average of 5 five human donors; B) example of individual donor #1 (average from 3 biopsies); C) example of individual donor #2 (average from 3 biopsies)

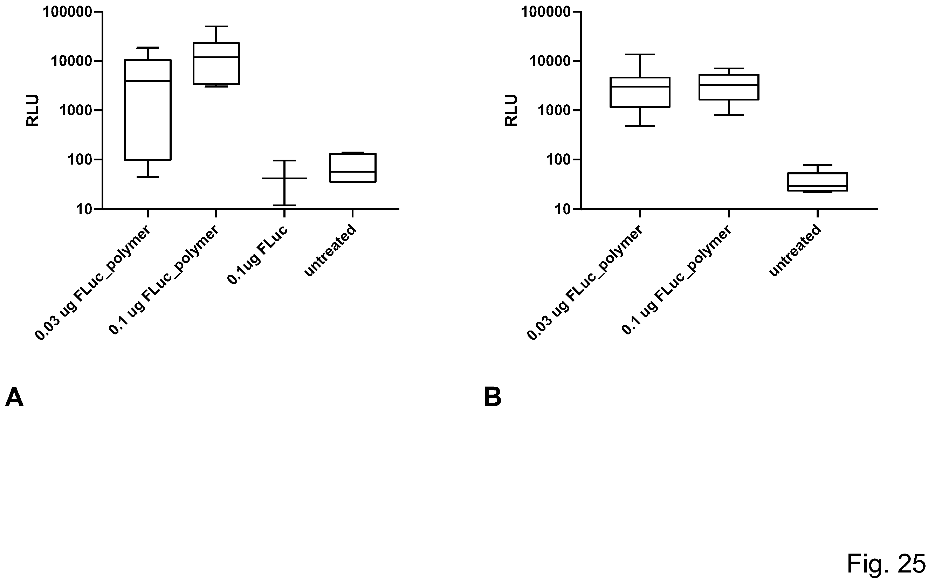

Figure 25 shows the detection of Firefly Luciferase (FLuc) expression in porcine skin biopsies 24h and 48h after intradermal injection with FLuc IVT mRNA complexed to cationic polymers (0,03-0,1µg mRNA/transfection) or non-complexed (0,1µg mRNA/transfection). Non-transfected porcine skin was used as control A: detection of relative luminescence units (RLU) 24h post transfection; B: detection of RLU 48h post transfection. 0,03µg FLuc _polymer and 0,1µg FLuc_polymer... IVT mRNA complexed to transfection reagent; 0,1µg FLuc... non-complexed mRNA, untreated... non-transfected skin

### EXAMPLES:

### Material and methods:

### Transfection of murine 3T3 fibroblasts and human B.J. skin fibroblasts

For transfection, murine 3T3 fibroblasts and human B.J. skin fibroblasts were seeded at 4-6 × 10⁴ cells/well in 12-well plates. After 24 hours incubation in full EMEM or DMEM medium (Gibco, Thermo Fisher, USA), culture medium was replaced. Different formulations of IVT mRNA complexed with TransIT mRNA transfection reagent (Mirus Bio; complex formation according to manufacturer instructions) were prepared and added to the cells. 24 hours after transfection, medium was replaced with complete DMEM. The cells were further cultured under standard conditions for up to 5 days with daily medium changes until results evaluation.

### Isolation and transfection of intact pig skin biopsies:

Full-thickness porcine skin flaps were isolated peri-mortally from pigs (samples are obtained under full compliance to current national legislation (i.e. Tierversuchsgesetz 2012, TVG 2012)) and disinfected using Octenisept^{®} disinfectant (Schuelke + Mayr GmbH, Germany).

Transfection of intact pig skin was done by direct, intradermal injection of the IVT-mRNA solution (1-10µg mRNA/dose). LacZ IVTmRNA (completely modified using 5-methylcytidine, pseu-douridine; Trilink Inc., USA) was formulated using either TransIT^{®}-mRNA Transfection kit (Mirus Bio^{™}) according to manufacturer instructions (with slight modification according to Kariko et al.; Mol. Ther. 2012. 20(5): 948-53) or DOTAP based liposomal formulations (Sigma Aldrich, USA). DOTAP based formulations were prepared using a lipid/RNA ratio of 5/1 (µg/µg). In addition, mRNA complexes were also supplemented with RNAse Inhibitor (5U/dose, RNasin, Promega, USA). Injection volume was ranging from 20µl to 30µL.

Alternatively, transfection of intact pig skin was done by direct, intradermal injection of eGFP IVT-mRNA solution (0,5-25µg mRNA/dose). eGFP IVTmRNA (AMPTec, Germany) was formulated using either TransIT^{®}-mRNA Transfection kit (Mirus Bio^{™}) according to manufacturer instructions (with slight modification according to Kariko et al., 2012, or DOTAP based liposomal formulations (Sigma Aldrich, USA), or Lipid-Nano-particle formulations (Polymun, Austria) or SAINT based liposomal formulations (Synvolux, Netherlands). DOTAP based liposomal formulations were prepared using a lipid/RNA ratio of 5/1 (µg/µg). SAINT lipid based formulations were prepared using a lipid/RNA ratio of 2.5-4/1 (µg/µg). In addition, also non-complexed mRNA in physiologic buffer was applied intradermally. Injection volume was ranging from 20µl to 30µL.

After injection punch biopsies of the injected areas (8mm, diameter) were taken, subcutaneous fat was removed and biopsies were transferred into standard complete culture medium in a petridish, epidermis up (5mL; containing: Dulbecco's Modified Eagle Medium with GlutaMAX (DMEM), 10% FCS, 1X Penicillin-Streptomycin-Fungizone; obtained from Gibco. Life Technologies). Subsequent culture was performed at 37°C/5% CO₂ for 24h. Harvest of biopsies was usually done 24 hours post transfection.

### Isolation and transfection of porcine epithelial sheets

Full-thickness porcine skin flaps were isolated peri-mortally from pigs (samples are obtained under full compliance to current national legislation (i.e. Tierversuchsgesetz 2012, TVG 2012)) and disinfected using Octenisept^{®} disinfectant (Schuelke + Mayr GmbH, Germany). Punch biopsies (6 or 8 mm, diameter) were taken from full-thickness skin flaps, subcutaneous fat was removed and biopsies were cut in two parts. Immediately afterwards cut biopsies were transferred, epidermis upside, to 9 cm (diameter) petri-dishes containing 5 mL Dispase II digestion solution (ca. 2.5 Units/mL; Dispase II; Sigma Aldrich, USA). Subsequent digestion was performed at 4°C overnight. Dispase II digestion solution was prepared by diluting Dispase II stock solution (10 mg/mL in 50mM HEPES/150 mM NaCl; pH-7.4) 1:2 with 1× DMEM (Gibco) and adding 1x Penicillin/Streptomycin. On the next day epidermal sheets were removed from the underlying dermis using forceps and transferred into DMEM for a short (5 min.) washing step. Subsequently sheets were put into complete DMEM culture medium and incubated at 37°C/5% CO₂ (6 to 8 hours) until transfection was performed in 24-well culture plates. Transfection of porcine epidermal sheets was performed using eGFP IVTmRNA (AmpTec, Germany) or IVT mRNA constructs for IFNa (e.g.: SEQ ID NOs:1-5 and NO:53). mRNA was formulated using either TransIT^{®}-mRNA Transfection kit (Mirus Bio^{™}) according to manufacturer instructions or liposomal formulations (Polymun, Austria). Liposomal formulations were prepared using a lipid/RNA ratio of 5/1 (µg/µg). All lipoplex solutions for transfection contained 0.1µg to 10µg mRNA/mL DMEM medium and epidermal sheets were cultured one to three days.

For analysis, tissue culture supernatants were collected for subsequent ELISA analysis. Sheets were harvested for RNA and protein extraction and subsequent analysis by qPCR and ELISA, respectively. In addition, eGFP transfected epidermal sheets were also analysed for eGFP expression by direct fluorescence microscopy and immunohistochemistry detecting eGFP in situ.

### RT-PCR analysis of cells transfected using IVT mRNA preparations

Human B.J. cells and murine 3T3 fibroblasts were transfected using 0.1 to 1µg IFNa2 IVT mRNAs complexed with TransIT mRNA transfection reagent. Total cellular RNAs were isolated from murine and human fibroblasts or porcine epithelial sheets at different time points post transfections using Tri-Reagent (Thermo Fisher, USA, manufacturer instructions) and mRNAs were reverse transcribed into cDNA by conventional RT-PCR (Protoscript First Strand cDNA synthesis kit, New England Biolabs, according to manufacturer instructions). cDNA samples were then subjected to conventional PCR and qPCR. Primers used were obtained from Invitrogen.

PCR analysis detecting IFNa2 variants was performed from cDNA obtained from cells/sheets transfected with different IFNa2 variants using Platinum Taq Polymerase (Invitrogen, USA) and IF-Na2 variant specific primers (Invitrogen, USA). Human RPL4 and murine ACTB (Eurofins Genomics) were used as positive controls. PCR products were analysed using conventional agarose gel electrophoresis.

**Table 4: PCR primers**

| **Primer (Producer, SEQ ID NO:)** | **Sequence** |
|---|---|
| huRPL4_fw (EG, 39) | 5'-AGC GTG GCT GTC TCC TCT C-3' |
| huRPL4_rev (EG, 40) | 5'-GAG CCT TGA ATA CAG CAG GC-3' |
| hu_IFNA2_v2_fw (IVG, 41) | 5'-GCT TGG GAT GAG ACC CTC CTA-3' |
| hu_IFNA2_v2_rev (IVG, 42) | 5'-CCC ACC CCC TGT ATC ACA C-3' |
| hu_IFNA2_ACC1_fw (IVG, 43) | 5'-CAC GAG ATG ATC CAG CAG AT-3' |
| hu_IFNA2_ACC1_rev (IVG, 44) | 5'-CTT GTC CAG CAG TGT CTC GT-3' |
| huIFNA2_AMP_humod_f (IVG, 45) | 5'-CTG CTC TGT TGG CTG TGA TT-3' |
| huIFNA2_AMP_humod_r (IVG, 46) | 5'-CAG GCA TGA GAA CAG GCT AA-3' |
| hu_IFNA2_AMP_AU_fw (IVG, 47) | 5'-TGA TGC TTC TTG CAC AAA TG-3' |
| hu_IFNA2_AMP_AU_rev (IVG, 48) | 5'-AGG ACA GGA ATG GTT TCA GC-3' |
| hu_IFNA2_AMP_GC_fw (IVG, 49) | 5'-CAA GGA GTC GGA GTG ACT GA-3' |
| hu_IFNA2_AMP_GC_rev (IVG, 50) | 5'-CAG GGT GAT CCT CTG GAA GT-3' |
| muACTB_fw (EG, 51) | 5'-GGC TGT ATT CCC CTC CAT CG-3' |
| muACTB_rev (EG, 52) | 5'-CCA GTT GGT AAC AAT GCC ATG T-3' |

| | |
|---|---|
| EG: Eurofins Genomics, IVG: Invitrogen | |

### Analysis of IVT mRNA induced human IFNa2 protein

Human B.J. cells and porcine epithelial sheets were transfected using 0.1-1µg IVT mRNA for different IFNa2 variants complexed with TransIT mRNA transfection reagent and cultured for up to 120h post transfection. Supernatant from transfected cells and epithelial sheets was obtained at several time points after transfection. Similarly, cells were harvested at the same time points and protein was extracted. Protein was extracted using cell extraction buffer (10mM HEPES, 10mM KCl, 0.1µM EDTA, 0.3% NP40 and Roche Protease Inhibitor, according to manufacturer's protocol). IFN-α determination in supernatants as well cellular extracts was performed using the human IFN-α (subtype 2; IFNa2) ELISA development kit (MABTECH AB, Sweden, according to manufacturer instructions), measurements were taken on an Infinite 200 PRO multimode reader (Tecan AG, Switzerland).

### Analysis of IVT mRNA induced eGFP protein

Intact porcine skin explants and porcine epithelial sheets were transfected using 0.1-10µg eGFP IVT mRNA complexed with TransIT mRNA transfection reagent or different liposomal carriers or uncomplexed ("naked" in physiologic buffer) and cultured for 24h post transfection. Samples were harvested and protein was extracted using cell extraction buffer (10mM HEPES, 10mM KCl, 0.1µM EDTA, 0.3% NP40 and Roche Protease Inhibitor, according to manufacturer's protocol). eGFP determination was performed using the GFP in vitro SimpleStep ELISA^{®} kit (Abcam Plc., UK, according to manufacturer instructions), measurements were taken on an Infinite 200 PRO multimode reader (Tecan AG, Switzerland) .

### Detection of beta-galactosidase activity in porcine tissue

Whole-mount beta-galactosidase (bGal) staining of biopsies was performed in 24 well culture plates for 24 or 48 h hours at 37°C. Positive staining controls were generated by injecting bGal enzyme (1U recombinant bGal protein/ injection) intradermally into pig skin. Before starting the staining procedure biopsies were mildly fixed in 4% formaldehyde solution (PBS) for 1 hour at room temperature. After fixation samples were washed in PBS (3x) and subsequently equilibrated in LacZ-washing buffer (2mM MgCl₂, 0.01% Na-deoxycholate and 0.02% NP-40 dissolved in PBS). After equilibration in LacZ buffer samples were stored overnight (4°C). Subsequently samples were incubated in staining solution at 37°C and colour reaction was monitored. The staining solution was freshly prepared (5mM K₄Fe(CN)₆ and 5mM K₃Fe(CN)₆ in LacZ buffer) and 1 mg/mL 5-Bromo-3-indolyl β-D-galactopyranoside, (Bluo-Gal) was added as colour substrate. If staining was performed for 48 hours the staining solution was substituted after 24 hours. Staining volume was generally 0.5 mL/well. Staining was stopped by washing in LacZ washing buffer and 3x PBS. Samples were subsequently either fixed overnight in buffered 4% formaldehyde solution and further processed for standard histology or were frozen in OCT for subsequent histologic analyses.

### Isolation and biolistic transfection of intact human skin biopsies:

Full-thickness human skin flaps were obtained from standard esthetic and reconstructive surgical procedures (samples are obtained under full compliance to current national legislation) and disinfected using Octenisept^{®} disinfectant (Schuelke + Mayr GmbH, Germany).

For biolistic mRNA transfection, the BioRad Helios gene gun system was used. The system was loaded with IVT mRNA coated gold particles (1.6µm gold microcarriers loaded with 1µg/µl IVT-mRNA; Biorad; according to manufacturer's protocols). Biolistic transfection was performed using helium gas pressure of 400 psi at a distance of 2.5 cm to human skin explants. Following transfection, punch biopsies of the transfected areas (8mm, diameter) were taken, subcutaneous fat was removed and biopsies were transferred into standard complete culture medium in a petri dish, epidermis up. Biopsies were maintained in αMEM + 10% pHPL media at an air-liquid interface at 5% CO₂ for 24 hours. Harvest of biopsies was usually performed 24 hours post transfection.

### Analysis of eGFP protein in situ in human skin

For biolistic transfection, the BioRad Helios Gene Gun System was used, loaded with eGFP-mRNA coated gold particles (1.6µm gold microcarriers loaded with 1µg/µl eGFP-mRNA) using a helium gas pressure of 400 psi at a distance of 2.5 cm to 8mm human skin explants. Explants were maintained in αMEM + 10% pHPL media at an air-liquid interface at 5% CO₂ for 24 hours. Biopsies were fixed in 4% Paraformaldehyde over night at 4°C and 10 µm cryo-sections were obtained. GFP antibody (Anti-GFP; chicken IgY) was used and detected by Alexa Fluor 488-conjugated donkey anti-chicken IgY antibody. The cytoskeleton was detected by staining F-actin using Alexa Fluor 568 Phalloidin and slides were mounted in Roti-mount Fluor Care DAPI containing DAPI (4',6-Diamidin-2-phenylindol) for counterstaining. Slides were scanned in 20× magnification by the Olympus Slidescanner VS-120-L 100-W system. Scale Bar in (A,B) = 500µm; Scale Bar in (C-L) = 50µm

### In vivo application of Firefly Luciferase IVT mRNA and analysis of IVT mRNA induced FLuc protein

All animal experiments were exclusively being carried out at the University Clinic for Swine (University of Veterinary Medicine Vienna) and were performed in accordance with the Austrian Animal Experiments Act (TVG2012) using pigs (mixed breed; Edelschwein x Pietrain). Experiments were approved by the Committee on the Ethics of Animal Experiments of the University of Veterinary Medicine Vienna and the and the Austrian Federal Ministry of Education, Science and Research and were performed under approval number: GZ 68.205/0192-WF/V/3b/2017. Pigs were between 30kg and 50kg at the time of the start of the experiment. On the day prior to the planned injections, the flanks of all pigs are carefully shaved in order to avoid skin irritations as much as possible. On the day of the injections, the pigs are anesthetized. Prior to the injections, the shaved skin areas are thoroughly cleaned with warm water and disinfected twice with Octenisept (Schuelke + Mayr GmbH, Germany). For the intradermal injections 30µL was applied using insulin syringes (BD Micro-FineTM+). The injection spots are distinctly marked with a suitable marker (Securline^{®} Laboratory Markers) and labelled according to the injections scheme. For sample analysis, pigs were eu-thanized under deep anaesthesia 24h and 48h post injection by trained personnel. Skin flaps containing all injection spots were resected and put on ice immediately. Porcine skin biopsies from labeled areas were harvested using 10mm punch biopsies. Samples were collected in 100uL of Dulbecco's Modified Eagle Medium, DMEM, High Glucose (Gibco) in a white 96 well plate (MicroWell^{™}, Nunc). Samples were subjected to direct Luciferase activity measurement. Measurements were performed using Firefly Luc One-Step Glow Assay Kit (Thermo Scientific, USA, according to manufacturer's instructions) and analysed on an Infinite 200 PRO multimode reader (Tecan AG, Switzerland).

**Example 1:** Detection of mRNA encoding different IFNa2 variants by IFNa2-variant specific PCR from cDNA obtained from human BJ fibroblast cells 24h-120h post transfection.

In order to assess whether IFNa2 mRNA variants are stable in human skin fibroblasts over prolonged time periods, different mRNAs encoding SEQ ID NOs:1-5 were used to transfect B.J. cells. RNA was isolated at different time points (24-120h post transfection) and the presence of mRNA in transfected cells was determined up to 120h post transfection by non-quantitative RT PCR.

Result: As shown in Figure 1 all IFNa2 mRNA variants can be detected following transfection using 1µg mRNA/12 well at all time points assessed, showing mRNA stability, irrespective of codon optimization or GC content in human cells for ≥120h.

Fig 1 shows BJ cells transfected using no, or 1µg mRNA complexed with TransIT mRNA transfection reagent. Total cellular RNAs were isolated at different time points after transfection (24h-120h) and mRNAs were reverse transcribed into cDNA by conventional RT-PCR. cDNA samples were then subjected to variant specific PCR using primers for SEQ ID NOs:1-5 for detection of transfected IFNa2 mRNAs and human RPL4 as PCR control (shown as ctr). Accordingly, all IFNa2a variants as present were stable over extended time periods in human cells (mRNA was also detectable for extended time in porcine epithelial sheets). It follows that there is differential expression/secretion of IFNa according to CAI and G+C content.

**Example 2:** Assessment of levels of human IFNa2a protein by protein ELISA from cell culture supernatants of human BJ fibroblast cells 24h and 72h post transfection with human IFNa IVT mRNA variants.

In order to assess whether codon optimization for optimal translation efficiency in the human system (as determined by the codon adaption index, CAI) and concomitant increase of GC content in the coding sequence (CDS) of IFNa2 mRNA variants changes expression of human IFNa2a protein in human skin fibroblasts, BJ fibroblasts were transfected with different IFNa2a mRNA variants. mRNAs encoding SEQ ID NOs:1, 2, 3 and 5 were used to transfect BJ cells. Subsequently, the level of secretion of human IFNa2a from transfected cells was determined for up to 120h post transfection.

Result: All 4 IFNa2 mRNA variants are inducing high levels of human IFNa2a protein using 1µg mRNA/well at all time points assessed (Figure 2). Comparative analysis of IVT mRNA encoding for native human IFNa2a and the three variants showed an unexpected combined effect of codon optimization (i.e. CAI levels ≥0.8) and an increase of G+C content (GC content ≥49.5%) in the CDS of the mRNA. Fig. 2 shows BJ cells (4*10⁴-5*10⁴/well) transfected using IVT mRNAs complexed with TransIT mRNA transfection reagent (1µg mRNA/well). Supernatants (≤n=6/condition) were obtained and subjected to human IFNa2 specific protein ELISA (MABTECH). Values depicted are measured as ng/ml IFNa2a protein.

Those sequences which were above the threshold of (CAI≥0.8 and GC content ≥49.50) were inducing significantly higher expression levels of IFNa2a at early and late stages indicating a surprisingly high benefit over wt, native sequences in the amplitude and longevity of expression.

**Example 3:** Assessment of levels of human IFNa2a protein by protein ELISA from cell culture supernatants of human BJ fibroblast cells 24h and 72h post transfection with human IFNa IVT mRNA variants.

In this example the effect of differential codon optimization for GC contents lower than the threshold (49.5%) and lower CAI levels (i.e. CAI<0.8) in the coding sequence (CDS) of IFNa2 mRNA variants was assessed. BJ fibroblasts were transfected with native (SEQ ID NO:1) as well as variants displaying CAIs<0.8 and/or GC contents <49.5% (e.g.: SEQ ID NO:4) as described above. Subsequently, the level of secretion of human IFNa2a from transfected cells was determined for up to 120h post transfection.

Result: Both IFNa2 mRNA variants are inducing high levels of human IFNa2a protein using 1µg mRNA/12 well at all time points assessed (Figure 3). Nevertheless, native, non-modified IFNa2a mRNA showed higher expression of IFNa2a protein as compared to SeqID4 mRNA at all time points assessed. Fig. 3 shows BJ cells (50.000/well) transfected using IVT mRNAs complexed with TransIT mRNA transfection reagent (1µg mRNA/well). Supernatants (≤n=6/condition) were obtained and subjected to human IFNa2 specific protein ELISA (MABTECH). Values depicted are measured as ng/ml IFNa2a protein.

Thus, sequences which underwent optimization but were below the threshold of (CAI≥0.8 and GC content ≥49.50) were less efficient in inducing IFNa2a in human cells (the amplitude and longevity of expression).

**Example 4:** Assessment of levels of human IFNa2a protein by protein ELISA from cell culture supernatants of porcine epithelial sheets 24h and 48h post transfection with human IFNa IVT mRNA variants.

In order to assess whether codon optimization for optimal translation efficiency in the human system (as determined by the codon adaption index, CAI) and concomitant increase of GC content in the coding sequence (CDS) of IFNa2 mRNA variants changes expression of human IFNa2a protein in porcine skin, epithelial sheets derived from porcine skin were transfected with different IFNa2a mRNA variants. mRNAs encoding SEQ ID NOs:1, 2, 3 and 5 were used to transfect epithelial sheets. TransIT alone as well as TransIT complexed to eGFP mRNA were used as controls. Subsequently, the level of secretion of human IFNa2a from transfected tissues was determined for up to 72h post transfection.

Result: All 4 IFNa2 mRNA variants are inducing high levels of human IFNa2a protein using 1µg mRNA/well at all time points assessed (Figure 4, see also Figure 13 for further data). Comparative analysis of IVT mRNA encoding for native human IFNa2a and the three variants showed an unexpected combined effect of codon optimization (i.e. CAI levels ≥0.8) and an increase of G+C content (GC content ≥49.50) in the CDS of the mRNA. Fig. 4 shows porcine epithelial sheets transfected using IVT mRNAs complexed with TransIT mRNA transfection reagent (1µg mRNA/well). Supernatants (≤n=6/condition) were obtained and subjected to human IF-Na2 specific protein ELISA (MABTECH). Values depicted are measured as ng/ml IFNa2a protein.

Those sequences which were above the threshold of (CAI≥0.8 and GC content ≥49.50) were inducing significantly higher expression levels of IFNa2a at early and late stages indicating a surprisingly high benefit over wt, native sequences in the amplitude and longevity of expression in an intact porcine tissue.

**Example 5:** Assessment of levels of human IFNa2a protein by protein ELISA from cell culture supernatants of porcine epithelial sheets 24h and 48h post transfection with human IFNa IVT mRNA variants.

In this example the effect of differential codon optimization for GC contents lower than the threshold (49.5%) and lower CAI levels (i.e. CAI<0.8) in the coding sequence (CDS) of IFNa2 mRNA variants was assessed. Porcine epithelial sheets were transfected with native (SEQ ID NO:1) as well as variants displaying CAIs<0,8 and/or GC contents <49,5% (e.g.: SEQ ID NO:4) as described above. Again, TransIT alone as well as TransIT complexed to eGFP mRNA were used as controls. Subsequently, the level of secretion of human IFNa2a from transfected tissue was determined for up to 72h post transfection.

Result: Both IFNa2 mRNA variants are inducing high levels of human IFNa2a protein using 1µg mRNA/well at all time points assessed (Figure 5). Nevertheless, native, non-modified IFNa2a mRNA showed higher expression of IFNa2a protein as compared to SeqID4 mRNA at all time points assessed. Fig. 5 shows porcine epithelial sheets transfected using IVT mRNAs complexed with TransIT mRNA transfection reagent (1µg mRNA/well). Supernatants (≤n=6/condition) were obtained and subjected to human IFNa2 specific protein ELISA (MABTECH). Values depicted are measured as ng/ml IFNa2a protein.

Thus, sequences which underwent optimization but were below the threshold of (CAI≥0.8 and GC content ≥49.50) were less efficient in inducing IFNa2a in porcine tissue (amplitude and longevity of expression).

**Example 6:** Detection of eGFP in porcine epithelial sheets 24h after transfection with eGFP IVT mRNA formulated using TransIT mRNA transfection reagent.

In this example eGFP expression was monitored over time as a positive control for transfection efficacy of experiments performed in example 4+5, respectively.

Porcine epithelial sheets were transfected with TransIT and TransIT complexed with eGFP mRNA as described above. In addition, also a second formulation of TransIT and eGFP mRNA (i.e. 0.5µg mRNA/well) was included as dosage control. Subsequently, eGFP protein expression in transfected tissue was monitored by direct fluorescence microscopy.

Result: similar levels of eGFP expression were detectable in both examples analysed indicating comparability of transfection efficacies in both experiments (Figure 6). Importantly, also a dose dependent effect of eGFP expression was detectable in this experiment.

Fig. 6 shows eGFP mRNA formulated in TransIT used at different concentrations: 0,5 and 1µg mRNA/ml. 24h post transfection native organ samples were mounted on Superfrost plus glass slides using Vectashield DAPI-Hard set embedding medium and subjected to direct fluorescence detection using a Zeiss AxioImage Z2 microscope with Apotome 2. Successful transfection was detectable by eGFP positive cells in the epithelial sheets as compared to liposome only treated sheets, respectively.

**Example 7:** Detection of eGFP in porcine epithelial sheets 24h after transfection with eGFP IVT mRNA formulated using a liposomal transfection reagent.

In this example eGFP expression induced by an alternative transfection formulation was monitored over time.

Porcine epithelial sheets were transfected with a liposome based formulation using two different eGFP mRNA concentrations (2µg/ml and 10µg/ml mRNA) as described above. Subsequently, eGFP protein expression in transfected tissue was monitored by direct fluorescence microscopy.

Result: eGFP expression was detectable in a dose dependent manner in this experiment (Figure 7 and Table 5). Overall transfection efficacy was comparable to results obtained in examples 4-6, respectively.

**Table 5: eGFP expression in porcine epithelial sheets 24h post transfection**

| Formulation of mRNA | Amount (mRNA/dose) | eGFP expression in epidermis (pg/mg total protein) |
|---|---|---|
| Cationic amphiphilic liposome | 5.4µg/ml | 775 |
| TransIT; lipoplex | 2µg/ml | 7677 |
| Cationic liposome | 2µg/ml | 8363 |
| Cationic liposome | 10µg/ml | 11180 |

Fig. 7 shows eGFP mRNA formulated in liposomes used at two concentrations: 2 and 10µg mRNA/ml. 24h post transfection native organ samples were mounted on Superfrost plus glass slides using Vectashield DAPI-Hard set embedding medium and subjected to direct fluorescence detection using a Zeiss AxioImage Z2 microscope with Apotome 2. Successful transfection was detectable by concentration dependent increase in eGFP positive cells in the epithelial sheets as compared to liposome only treated sheets, respectively.

**Example 8:** Detection of whole mount β-Galactosidase (bGal) activity in porcine skin explants 24h after transfection with LacZ IVT mRNA formulated using a DOTAP based liposomal transfection reagent.

In order to assess whether mRNA variants are able to transfect mammalian skin in situ, punch biopsies obtained from porcine skin were transfected with different LacZ mRNA formulations. In this example LacZ expression induced by intradermal transfection was monitored by whole mount β-Galactosidase staining 24h post transfection.

Transfection of intact pig skin was done by direct, intradermal injection of the IVT-mRNA solution (5µg mRNA/dose; +/-Rnase inhibitor). mRNA was formulated using DOTAP-liposomes. 24h post transfection organ samples were subjected to whole mount β-Galactosidase (bGal) staining. Succesful transfection is detectable by BluoGal staining in situ. Subsequently, punch biopsies of the injected areas (8mm, diameter) were taken, subcutaneous fat was removed and biopsies were cultured for 24h.

Result: LacZ expression was visualized by detection of bGal activity in transfected biopsies (Figure 8). bGal activity was comparable for different formulations of LacZ mRNA (+/- RNAse inhibitor) and expression was detectable as seen by blue staining in the upper dermal compartment of transfected biopsies.

**Example 9:** Detection of eGFP expression in porcine skin explants 24h after transfection with eGFP IVT mRNA formulated using various transfection reagents and non-complexed RNAs.

In order to assess whether mRNA variants are able to transfect mammalian skin in situ, punch biopsies obtained from porcine skin were transfected with different eGFP mRNA formulations. In this example eGFP expression induced by intradermal transfection was monitored by eGFP protein ELISA from tissue extracts obtained 24h post transfection. Along these lines, different formulations of eGFP mRNA were produced and injected (see Table 6 for details).

**Table 6: eGFP expression in porcine skin biopsies 24h post i.d. transfection**

| Formulation of mRNA | Amount (mRNA/dose) | eGFP expression in dermis |
|---|---|---|
| DOTAP based; liposomal | 1-10µg | + |
| SAINT lipid based; liposomal | 1-5µg | + |
| TransIT; lipoplex | 1-10µg | + |
| Lipid-Nano-particle | 1.2+2.4µg | + |
| Non-complexed | 1-25µg | + |

Formulations used included: eGFP mRNA complexed with TransIT mRNA transfection reagent, eGFP mRNA complexed with DOTAP based liposomal preparations, eGFP mRNA complexed with SAINT lipid based liposomal preparations, eGFP mRNA containing Lipid nano particles and as non-complexed eGFP mRNA formulated in physiologic buffer.

Transfection of intact pig skin was done by direct, intradermal injection of the IVT-mRNA solutions (the eGFP IVTm RNA (1-25µg mRNA/dose)). mRNA was formulated using TransIT mRNA transfection reagent, DOTAP based-liposomes, SAINT lipid based-liposomes, lipid nano particles or non-complexed mRNA in physiologic buffer. Subsequently, punch biopsies of the injected areas (8mm, diameter) were taken, subcutaneous fat was removed, biopsies were cultured for 24h and subsequently analysed for eGFP expression. 24h post transfection organ samples were subjected to protein extraction and subsequent eGFP protein ELISA.

Result: eGFP expression was detectable by eGFP protein ELISA 24h post injection. (Figure 9, Table 5). eGFP Lipoplexes (LNPs and liposomal complexes) as well as TransIT (used as standard) showed similar concentration dependent eGFP induction. Optimal expression was detectable between 2.4µg and 5µg mRNA/dose. Non-complexed mRNA also showed successful transfection. However, the minimal concentration required in this experimental setting was 5µg mRNA/dose in order to induce detectable eGFP expression in porcine dermis indicating less efficient transfection of mRNA in the absence of transfection reagents.

**Example 10:** Detection of mRNA encoding different IFNa2 variants by IFNa2-variant specific PCR from cDNA obtained from murine 3T3 fibroblast cells 24h-120h post transfection.

In order to assess whether IFNa2 mRNA variants are stable in murine fibroblasts over prolonged time periods, different mRNAs encoding SEQ ID NOs:1-5 were used to transfect 3T3 cells. RNA was isolated at different time points (24-120h post transfection) and the presence of mRNA in transfected cells was determined up to 120h post transfection by non-quantitative RT PCR.

Result: As shown in Figure 10 all IFNa2 mRNA variants can be detected following transfection using 1µg mRNA/12 well at all time points assessed, showing mRNA stability, irrespective of codon optimization or GC content in human cells for ≥120h.

Fig 10 shows 3T3 cells transfected using no, or 0.1-1µg mRNA complexed with TransIT mRNA transfection reagent. Total cellular RNAs were isolated at different time points after transfection (24h-120h) and mRNAs were reverse transcribed into cDNA by conventional RT-PCR. cDNA samples were then subjected to variant specific PCR using primers for SEQ ID NOs:1-5 for detection of transfected IFNa2 mRNAs and murine ACTB as PCR control (shown as ctr). Accordingly, all IFNa2a variants as present were stable over extended time periods in human cells. It follows that there is differential expression/secretion of IFNα according to CAI and G+C content.

**Example 11:** Assessment of levels of human IFNa2a protein by protein ELISA from cell culture supernatants of murine 3T3 fibroblast cells 96h and 120h post transfection with human IFNα IVT mRNA variants.

In order to assess whether codon optimization for optimal translation efficiency in the human system (as determined by the codon adaption index, CAI) and concomitant increase of GC content in the coding sequence (CDS) of IFNa2 mRNA variants changes expression of human IFNa2a protein in human skin fibroblasts, 3T3 fibroblasts were transfected with different IFNa2a mRNA variants. mRNAs encoding SEQ ID NOs:1, 2, 3 and 5 were used to transfect 3T3 cells. Subsequently, the level of secretion of human IFNa2a from transfected cells was determined for up to 120h post transfection.

Result: All 4 IFNa2 mRNA variants are inducing high levels of human IFNa2a protein using 1µg mRNA/well at all time points assessed (Figure 11). Comparative analysis of IVT mRNA encoding for native human IFNa2a and the three variants showed an unexpected combined effect of codon optimization (i.e. CAI levels ≥0.8) and an increase of G+C content (GC content ≥49.5%) in the CDS of the mRNA. Fig. 11 shows 3T3 cells (4*10⁴-5*10⁴/well) transfected using IVT mRNAs complexed with TransIT mRNA transfection reagent (1µg mRNA/well). Supernatants were obtained and subjected to human IFNa2 specific protein ELISA (MABTECH). Values depicted are measured as ng/ml IFNa2a protein.

Those sequences which were above the threshold of (CAI≥0.8 and GC content ≥49.50) were inducing significantly higher expression levels of IFNa2a at early and late stages indicating a surprisingly high benefit over wt, native sequences in the amplitude and longevity of expression.

**Example 12:** Assessment of levels of human IFNa2a protein by protein ELISA from cell culture supernatants of murine 3T3 fibroblast cells 24h post transfection with human IFNa IVT mRNA variants.

In this example the effect of differential codon optimization for GC contents lower than the threshold (49.5%) and lower CAI levels (i.e. CAI<0.8) in the coding sequence (CDS) of IFNa2 mRNA variants was assessed. 3T3 fibroblasts were transfected with native (SEQ ID NO:1) as well as variants displaying CAIs<0.8 and/or GC contents <49.5% (e.g.: SEQ ID NO:4) as described above. Subsequently, the level of secretion of human IF-Na2a from transfected cells was determined for up to 120h post transfection.

Result: Both IFNa2 mRNA variants are inducing high levels of human IFNa2a protein using 1µg mRNA/12 well at all time points assessed (Figure 12). Nevertheless, native, non-modified IFNa2a mRNA showed higher expression of IFNa2a protein as compared to SeqID4 mRNA at all time points assessed. Fig. 12 shows 3T3 cells (40-50.000/well) transfected using IVT mRNAs complexed with TransIT mRNA transfection reagent (1µg mRNA/well). Supernatants were obtained and subjected to human IFNa2 specific protein ELISA (MABTECH). Values depicted are measured as ng/ml IFNa2a protein.

Thus, sequences which underwent optimization but were below the threshold of (CAI≥0.8 and GC content ≥49.50) were less efficient in inducing IFNa2a in human cells (the amplitude and longevity of expression).

**Example 13:** Assessment of levels of human IFNa2a protein by protein ELISA from cell extracts of porcine epithelial sheets 48h post transfection with human IFNa IVT mRNA variants.

In order to assess whether codon optimization for optimal translation efficiency in the human system (as determined by the codon adaption index, CAI) and concomitant increase of GC content in the coding sequence (CDS) of IFNa2 mRNA variants changes expression of human IFNa2a protein in porcine skin, epithelial sheets derived from porcine skin were transfected with different IFNa2a mRNA variants. mRNAs encoding SEQ ID NOs:1, 2, 3 and 5 were used to transfect epithelial sheets. TransIT alone was used as control. Subsequently, the intracellular level of human IF-Na2a from transfected tissues was determined for up to 72h post transfection.

Result: All 4 IFNa2 mRNA variants are inducing high levels of human IFNa2a protein using 1µg mRNA/well at all time points assessed (Figure 13 shows an example at 48h post transfection). Comparative analysis of IVT mRNA encoding for native human IF-Na2a and the three variants showed an unexpected combined effect of codon optimization (i.e. CAI levels ≥0.8) and an increase of G+C content (GC content ≥49.50) in the CDS of the mRNA. Fig. 13 shows porcine epithelial sheets transfected using IVT mRNAs complexed with TransIT mRNA transfection reagent (1µg mRNA/well). Cell extracts were obtained and subjected to human IFNa2 specific protein ELISA (MABTECH). Values depicted are measured as ng human IFNa2 protein /mg total protein.

Those sequences which were above the threshold of (CAI≥0.8 and GC content ≥49.50) were inducing significantly higher expression levels of IFNa2a at early and late stages indicating a surprisingly high benefit over wt, native sequences in the amplitude and longevity of expression in an intact porcine tissue.

**Example 14:** Comparison of Seq ID NO:1 and SEQ ID NO:3 mRNAs to their variants which have 100% replacement of Pseudo-U for U and 5mC for C by Assessment of levels of human IFNa2a protein by protein ELISA from cell culture supernatants of porcine epithelial sheets 24h post transfection with human IFNa IVT mRNA variants.

In this example the effect of modified nucleotides (e.g.: pseudoU and m5C) on expression levels of IFNa2 mRNA variants is assessed. Porcine epithelial sheets are transfected with two forms of native (SEQ ID NO:1) IVT mRNA: one containing 100% replacement of Pseudo-U for U and 5mC for C and one w/o modified nucleotides as well as two forms of an IFNa variant (SEQ ID NO:3) displaying CAIs>0,8 and/or GC contents >49.5% (e.g.: one variant of SEQ ID NO:3 containing 100% replacement of Pseudo-U for U and 5mC for C and one w/o modified nucleotides) as described above. Again, TransIT alone as well as TransIT complexed to eGFP mRNA are used as controls. Subsequently, the level of secretion of human IFNa2a from transfected tissue is determined for 24h post transfection.

Result: As shown in figure 14, IFNa2 expression is visualized by detection of secreted IFNa2a in cell supernatants.

Those sequences which are above the threshold of (CAI≥0.8 and GC content ≥49.50) are inducing significantly higher expression levels of IFNa2a at early and late stages indicating a surprisingly high benefit over wt, native sequences in the amplitude and longevity of expression in an intact porcine tissue.

**Example 15:** Comparison of Seq ID NO:1 and SEQ ID NO:3 mRNAs to their variants which have 100% replacement of Pseudo-U for U and 5mC for C by Assessment of levels of human IFNa2a protein by protein ELISA from cell culture supernatants of human BJ fibroblasts 24-120h post transfection with human IFNa IVT mRNA variants.

In this example the effect of modified nucleotides (e.g.: pseudoU and m5C) on expression levels of IFNa2 mRNA variants is assessed. Human BJ fibroblasts are transfected with two forms of native (SEQ ID NO:1) IVT mRNA: one containing 100% replacement of Pseudo-U for U and 5mC for C and one w/o modified nucleotides as well as two forms of an IFNa variant (SEQ ID NO:3) displaying CAIs>0,8 and/or GC contents >49.5% (e.g.: one variant of SEQ ID NO:3 containing 100% replacement of Pseudo-U for U and 5mC for C and one w/o modified nucleotides) as described above. Again, TransIT alone as well as TransIT complexed to eGFP mRNA are used as controls. Subsequently, the level of secretion of human IF-Na2a from transfected cells is determined for 24-120h post transfection.

Result: As shown in figure 15, IFNa2 expression is visualized by detection of secreted IFNa2a in cell supernatants.

Those sequences which are above the threshold of (CAI≥0.8 and GC content ≥49.50) are inducing significantly higher expression levels of IFNa2a at early and late stages indicating a surprisingly high benefit over wt, native sequences in the amplitude and longevity of expression in an intact porcine tissue.

**Example 16:** Comparison of Seq ID NO:1 and SEQ ID NO:53 by assessment of levels of human IFNa2a protein by protein ELISA from cell culture supernatants of human BJ fibroblasts 24-120h post transfection with human IFNa IVT mRNA variants.

In this example the effect of UTR modification and differential codon optimization in the coding sequence (CDS) of IFNa2 mRNA variants was assessed.

Human BJ fibroblasts were transfected with fully human IFNa (SEQ ID NO:53), as well as IVT mRNAs containing the native IFNa coding sequence (CDS; SEQ ID NO:1). Again, TransIT alone as well as TransIT complexed to eGFP mRNA were used as controls (not shown). Subsequently, the level of secretion of human IFNa2a from transfected tissue was determined for up to 120h post transfection.

Result: all IFNa2 mRNA variants used are inducing high levels of human IFNa2a protein using 1µg mRNA/well at all time points assessed (Figure 16). Nevertheless, fully human IFNa (SEQ ID NO:53) showed lower expression of IFNa2a protein as compared to SEQ ID NO: 1 at all time points assessed. Figure 16 shows human BJ fibroblasts transfected using IVT mRNAs complexed with TransIT mRNA transfection reagent (1µg mRNA/well). Supernatants were obtained and subjected to human IFNa2 specific protein ELISA (MABTECH). Values depicted are measured as ng/ml IFNa2a protein.

Thus, sequences which underwent UTR and/or CDS optimization were more efficient in inducing IFNa2a in porcine tissue (amplitude and longevity of expression) than the fully human IFNa mRNA. The extent of increase for SEQ ID NO:1 compared to SEQ ID NO:53 is: 6,2 fold at 24h post transfection; 4,8 fold at 48h post transfection; and 92,5 fold 72h post transfection, respectively.

**Example 17:** Assessment of levels of human IFNa2a protein by protein ELISA from cell culture supernatants of porcine epithelial sheets 24h and 48h post transfection with human IFNa mRNA and human IVT mRNA variants.

In this example the effect of UTR modification and differential codon optimization in the coding sequence (CDS) of IFNa2 mRNA variants was assessed.

Porcine epithelial sheets were transfected with fully human IFNa (SEQ ID NO:53), as well as IVT mRNAs containing the native IFNa coding sequence (CDS; SEQ ID NO:1) as well as CDS variants as described above (SEQ ID NO:3). Again, TransIT alone as well as TransIT complexed to eGFP mRNA were used as controls. Subsequently, the level of secretion of human IFNa2a from transfected tissue was determined for up to 48h post transfection.

Result: all IFNa2 mRNA variants used are inducing high levels of human IFNa2a protein using 1µg mRNA/well at all time points assessed (Figure 17). Nevertheless, fully human IFNa (SEQ ID NO:53) showed lower expression of IFNa2a protein as compared to SEQ ID NO: 1-3 at all time points assessed. Fig. 17 shows porcine epithelial sheets transfected using IVT mRNAs complexed with TransIT mRNA transfection reagent (1µg mRNA/well). Supernatants were obtained and subjected to human IFNa2 specific protein ELISA (MABTECH). Values depicted are measured as ng/ml IF-Na2a protein.

Thus, sequences which underwent UTR and/or CDS optimization were more efficient in inducing IFNa2a in porcine tissue (amplitude and longevity of expression) than the fully human IFNa mRNA. The extent of increase compared to SEQ ID NO:53 is: 7.4 fold for SEQ ID NO:1; and 8.6 fold for SEQ ID NO:3, respectively.

**Example 18:** Assessment of levels of human IFNa2a protein by protein ELISA from cell culture supernatants and tissue extracts of biolistically transfected human skin biopsies 24h post transfection with human IFNa mRNA and human IVT mRNA variants.

In this example the effect of UTR modification and differential codon optimization in the coding sequence (CDS) of IFNa2 mRNA variants was assessed. Human skin biopsies from n=5 different donors were biolistically transfected using the Helios gene gun system with fully human IFNa (SEQ ID NO:53), as well as IVT mRNAs containing the native IFNa coding sequence (CDS; SEQ ID NO:1) or CDS variants as described above (SEQ ID NO:2,3,5). Non-transfected skin as well as skin transfected with eGFP IVT mRNA were used as controls. Subsequently, the level of secreted human IFNa2a from transfected tissue and the level of IFNa2a in tissue was determined for up to 24h post transfection.

Result: Fig. 18 shows similar efficacy in coating of gold particles irrespective of the mRNA used. Figure 19 shows secreted IFNa2a from human skin biopsies transfected using IVT mRNAs particles. Supernatants were obtained and subjected to human IF-Na2 specific protein ELISA (MABTECH). Values depicted are measured as ng/ml IFNa2a protein. Figure 20 shows levels of human IFNa2a protein in skin tissue from human skin biopsies transfected using IVT mRNAs particles. Cell extracts were obtained and subjected to human IFNa2 specific protein ELISA (MABTECH). Figure 21 shows epidermal transfection using biolistic eGFP particle transfection.

eGFP mRNA is inducing a very low level secretion of human IFNa2a from human skin following transfection (157 pg/ml; 12 fold lower than SEQ ID NO:53 (1873 pg/ml)). All IFNa2 mRNA variants used are inducing high levels of human IFNa2a protein secretion using 1µg mRNA/µl loaded particles (Figure 18-20). Nevertheless, fully human IFNa (SEQ ID NO:53) showed lower secretion of IFNa2a protein as compared to SEQ ID NO: 1-5. Thus, sequences which underwent UTR and/or CDS optimization were more efficient in inducing IFNa2a secretion in human tissue (amplitude and longevity of expression) than the fully human IFNa mRNA. The extent of increase compared to SEQ ID NO:53 based on all 5 donors is: 10.9 fold for SEQ ID NO:1; 2.7 fold for SEQ ID NO:2; 19.2 fold for SEQ ID NO:3; and 5.8 fold for SEQ ID NO:5 in this experiment, respectively. Individual donors as presented in Figure 19 show different amplitudes supporting the teaching mentioned above.

eGFP mRNA is inducing a very low level of human IFNa2a in human skin following transfection (44pg/mg tissue; 26 fold lower than SEQ ID NO:53 (1150pg/mg tissue)). All IFNa2 mRNA variants used are inducing high levels of human IFNa2a protein using 1µg mRNA/µl loaded particles (Figure 18-20). Nevertheless, fully human IFNa (SEQ ID NO:53) showed lower expression of IFNa2a protein as compared to SEQ ID NO: 1-5. Thus, sequences which underwent UTR and/or CDS optimization were more efficient in inducing IFNa2a in human tissue (amplitude and longevity of expression) than the fully human IFNa mRNA. The extent of increase compared to SEQ ID NO:53 based on all 5 donors is: 4.2 fold for SEQ ID NO:1; 1.4 fold for SEQ ID NO:2; 10.1 fold for SEQ ID NO:3; and 3 fold for SEQ ID NO:5 in this experiment, respectively. Individual donors as presented in Figure 20 show different amplitudes supporting the teaching mentioned above.

Example 19: Assessment of eGFP protein expression by immunofluorescence analysis from biolistically transfected human skin biopsies 24h post transfection with eGFP mRNA.

In this example the expression and localization of eGFP protein in human skin was assessed. Human skin biopsies from different donors were biolistically transfected using the Helios gene gun system with eGFP IVT mRNA. Skin treated with empty gold particles was used as control. Subsequently, biopsies were fixed 24h post transfection in 4% Paraformaldehyde, and eGFP specific immunofluorescence analysis was performed on 10µm cryosections.

Result: as shown in Figure 21, widespread expression of eGFP can be detected in biolistically transfected human skin 24h post transfection. Interestingly, biolistic transfection is limited to the epidermal compartment as marked by eGFP positive, green cells detectable in the F-actin positive epidermal compartment (red) but absent from the underlying dermal compartment). This epidermal targeting is strongly supporting feasibility and applicability of the previously impossible, novel concept of directly targeting affected cells (e.g.: keratinocytes) in the epidermis and thus excerting a direct effect on affected cells by IFNa mRNA mediated protein expression in addition to potential indirect effects also addressable by state of the art topical application of immune modulators like Imiquimod.

**Example 20:** Assessment of levels of human IFNa2a protein by protein ELISA from cell culture supernatants of porcine epithelial sheets 24h and 48h post transfection with human IFNa mRNA and human IVT mRNA variants.

In this example the effect of UTR modification and differential codon optimization in the coding sequence (CDS) of IFNa2 mRNA variants was assessed.

Porcine epithelial sheets were transfected with fully human IFNa (SEQ ID NO: 53), as well as CDS variants as described above (SEQ ID NO: 4). Again, TransIT alone as well as TransIT complexed to eGFP mRNA were used as controls. Subsequently, the level of secreted human IFNa2a from transfected tissue was determined for up to 48h post transfection.

Result: all IFNa2 mRNA variants used are inducing high levels of human IFNa2a protein using 1µg mRNA/well at all time points assessed (Figure 22). Nevertheless, fully human IFNa (SEQ ID NO:53) showed lower expression of IFNa2a protein as compared to SEQ ID NO: 4 at all time points assessed. Fig. 20 shows porcine epithelial sheets transfected using IVT mRNAs complexed with TransIT mRNA transfection reagent (1µg mRNA/well). Supernatants were obtained and subjected to human IFNa2 specific protein ELISA (MABTECH). Values depicted are measured as ng/ml IF-Na2a protein.

Thus, sequences which underwent UTR and/or CDS optimization were more efficient in inducing IFNa2a in porcine tissue (amplitude and longevity of expression) than the fully human IFNa mRNA.

**Example 21:** Assessment of levels of human IFNa2a protein by protein ELISA from cell culture supernatants and tissue extracts of biolistically transfected human skin biopsies 24h post transfection with human IFNa mRNA and human IVT mRNA variants.

In this example the effect of UTR modification and differential codon optimization in the coding sequence (CDS) of IFNa2 mRNA variants was assessed. Human skin biopsies from n=5 different donors were biolistically transfected using the Helios gene gun system with fully human IFNa (SEQ ID NO:53), as well as IVT mRNAs containing CDS variants as described above (SEQ ID NO:4). Non-transfected skin as well as skin transfected with eGFP IVT mRNA were used as controls. Subsequently, the level of secreted human IFNa2a from transfected tissue and the level of IFNa2a in tissue was determined for up to 24h post transfection.

Result: Figure 23 shows secretion of IFNa2a from human skin biopsies transfected using IVT mRNAs particles. Supernatants were obtained and subjected to human IFNa2 specific protein ELISA (MABTECH). Values depicted are measured as pg/ml IFNa2a protein. Figure 24 shows levels of human IFNa2a protein in skin tissue from human skin biopsies transfected using IVT mRNAs particles. Cell extracts were obtained and subjected to human IFNa2 specific protein ELISA (MABTECH).

eGFP mRNA is inducing a very low level secretion of human IFNa2a from human skin following transfection (157 pg/ml; 12 fold lower than SEQ ID NO:53 (1873 pg/ml)). All IFNa2 mRNA variants used are inducing high levels of human IFNa2a protein secretion using 1µg mRNA/µl loaded particles (Figure 23). Nevertheless, fully human IFNa (SEQ ID NO:53) showed lower secretion of IFNa2a protein as compared to SEQ ID NO: 4. Thus, sequences which underwent UTR and/or CDS optimization were more efficient in inducing IFNa2a secretion in human tissue (amplitude and longevity of expression) than the fully human IFNa mRNA. Individual donors as presented in Figure 23 show different amplitudes supporting the teaching mentioned above.

eGFP mRNA is inducing a very low level of human IFNa2a in human skin following transfection (44pg/mg tissue; 26 fold lower than SEQ ID NO:53 (1150pg/mg tissue)). All IFNa2 mRNA variants used are inducing high levels of human IFNa2a protein using 1µg mRNA/µl loaded particles (Figure 24). Nevertheless, fully human IFNa (SEQ ID NO:53) showed lower expression of IFNa2a protein as compared to SEQ ID NO: 4. Thus, sequences which underwent UTR and/or CDS optimization were more efficient in inducing IFNa2a in human tissue (amplitude and longevity of expression) than the fully human IFNa mRNA. Individual donors as presented in Figure 24 show different amplitudes supporting the teaching mentioned above.

**Example 22:** In vivo detection of Firefly Luciferase activity in porcine skin 24h an 48h after transfection with Firefly Luciferase IVT mRNA formulated using a cationic polymer-based transfection reagent.

In this example, in vivo Firefly Luciferase (FLuc) expression induced by cationic polymer-based transfection formulations was monitored over time. For detection of Luciferase activity, pigs (ca. 45kg, mixed breed; Edelschwein x Pietrain) were injected intradermally with Polymer based formulations using 2 different doses of mRNA: 1ng/µl (i.e.: 0,03µg/dose) and 3,3ng/µl (i.e.: 0,1µg/dose) mRNA as well as with non-complexed mRNA (3,3ng/µl; i.e.: 0,1µg/dose) as described above. Native, non-transfected organ samples (i.e. skin biopsies) were used as background controls. Subsequently, Luciferase activity transfected tissue was monitored 24h and 48h post transfection by direct activity measurement. For analysis, injection sites were isolated and resulting biopsies were subjected to Firefly Luc One-Step Glow Assay Kit. Successful transfection was detectable by Bioluminescence (detection: relative luminescence units (RLU)).

Result: Luciferase activity was detectable using both doses of complexed FLuc mRNA following transfection (Figure 25). In this experiment, Luciferase activity was detectable 24h (Fig. 25A; n=6 samples for 0,03µg/dose and n=9 for 0,1µg/dose; non complexed mRNA: n=3; untreated biopsies: n=6) and 48h (Fig. 25B n=9 samples for 0,03µg/dose and n=9 for 0,1µg/dose; untreated biopsies: n=6) post in vivo, intradermal injection in a mRNA dose dependent manner (0,1µg FLuc mRNA complex induced RLU > 0,03µg FLuc mRNA complex induced RLU). Non-complexed FLuc mRNA induced RLUs similar to background signals also detectable in untreated skin samples, respectively.

Accordingly, the present invention relates to the following preferred embodiments:
1. Interferon alpha (IFN-α) messenger-RNA (mRNA), wherein the mRNA has a 5' CAP region, a 5' untranslated region (5'-UTR), a coding region encoding human IFN-α, a 3' untranslated region (3'-UTR) and a poly-adenosine Tail (poly-A tail), for use in the prevention and treatment of non-melanoma skin cancer (NMSC) in a human patient.
2. IFN-α mRNA for use according to embodiment 1, wherein the NMSC is actinic keratosis (AK), basal cell carcinoma (BCC) and squamous cell carcinoma (SCC), especially AK.
3. IFN-α mRNA for use according to embodiment 1 or 2, wherein the IFN-α mRNA is selected from IFN-α type 1 mRNA (IFNal), IFN-α type 2a mRNA (IFNa2a), and IFN-α type 2b mRNA (IFNa2b).
4. IFN-α mRNA for use according to any one of embodiments 1 to 3, wherein the poly-A tail comprises at least 100 adenosine monophosphates, preferably at least 120 adenosine monophosphates.
5. IFN-α mRNA for use according to any one of embodiments 1 to 4, wherein the 5'-UTR or 3'-UTR or the 5'-UTR and the 3'-UTR are different from the native IFN-α mRNA, preferably wherein the 5'-UTR or 3'-UTR or the 5'-UTR and the 3'-UTR contain at least one a stabilisation sequence, preferably a stabilisation sequence with the general formula (C/U)CCANₓCCC(U/A)PyₓUC(C/U)CC (SEQ ID NO:38), wherein "x" is, independently in Nₓ and Pyₓ, an integer of 0 to 10, preferably of 0 to 5, especially 0, 1, 2, 4 and/or 5) .
6. IFN-α mRNA for use according to any one of embodiments 1 to 5, wherein the 5'-UTR or 3'-UTR or the 5'-UTR and the 3'-UTR are different from the native IFN-α mRNA, and contain at least one destabilisation sequence element (DSE), preferably AU-rich elements (AREs) and/or U-rich elements (UREs), especially a single, tandem or multiple or overlapping copies of the nonamer UUAUUUA(U/A) (U/A.
7. IFN-α mRNA for use according to any one of embodiments 1 to 6, wherein the 5'-UTR or 3'-UTR or the 5'-UTR and the 3'-UTR are different from the native IFN-α mRNA, and wherein the 5'-UTR and/or 3'-UTR are the 5'-UTR and/or 3'-UTR of a different human mRNA than IFN-α, preferably selected from alpha Globin, beta Globin, Albumin, Lipoxygenase, ALOX15, alpha(1) Collagen, Tyrosine Hydroxylase, ribosomal protein 32L, eukaryotic elongation factor 1a (EEF1A1), 5'-UTR element present in orthopoxvirus, and mixtures thereof, especially selected from alpha Globin, beta Globin, alpha(1) Collagen, and mixtures thereof.
8. IFN-α mRNA for use according to any one of embodiments 1 to 7, wherein in the IFN-α mRNA, at least 5%, preferably at least 10%, preferably at least 30%, especially at least 50% of all
   - cytidine residues are replaced by 5-methyl-cytidine residues, and/or
   - cytidine residues are replaced by 2-amino-2-deoxy-cytidine residues, and/or
   - cytidine residues are replaced by 2-fluoro-2-deoxy-cytidine residues, and/or
   - cytidine residues are replaced by 2-thio-cytidine residues, and/or
   - cytidine residues are replaced by 5-iodo-cytidine residues, and/or
   - uridine residues are replaced by pseudo-uridine residues, and/or
   - uridine residues are replaced by 1-methyl-pseudo-uridine residues, and/or
   - uridine residues are replaced by 2-thio-uridine residues, and/or
   - uridine residues are replaced by 5-methyl-uridine residues, and/or
   - adenosine residues are replaced by N6-methyl-adenosine residues.
9. IFN-α mRNA for use according to any one of embodiments 1 to 8, wherein in the IFN-α mRNA, at least 5%, preferably at least 10%, preferably at least 30%, especially at least 50% of all
   - cytidine residues are replaced by 5-methyl-cytidine residues, and/or
   - uridine residues are replaced by pseudo-uridine residues, and/or
   - uridine residues are replaced by 2-thio-uridine residues.
10. IFN-α mRNA for use according to any one of embodiments 1 to 9, wherein the IFN-α mRNA has a GC to AU ratio of at least 49.5%, preferably of at least 49.6, more preferred 50%, even more preferred, at least 55%, especially at least 60%.
11. IFN-α mRNA for use according to any one of embodiments 1 to 10, wherein the IFN-α mRNA has a codon adaption index (CAI) of at least 0.8, preferably at least 0.9.
12. IFN-α mRNA for use according to any one of embodiments 1 to 11, wherein the coding region of the IFN-α mRNA encodes human IFNa2a and is preferably SEQ ID NO:12, especially SEQ ID NOs: 2, 3, 5, 6, 7, 8, 9, 10, or 11.
13. IFN-α mRNA for use according to any one of embodiments 1 to 10, wherein the coding region of the IFN-α mRNA encodes human IFNa2b and is preferably SEQ ID NO:26, especially SEQ ID NOs: 19, 20, 22, or 25.
14. IFN-α mRNA for use according to any one of embodiments 1 to 10, wherein the coding region of the IFN-α mRNA encodes human IFNa1 and is preferably SEQ ID NO:36, especially SEQ ID NOs: 29, 30, 31, 32, 34, or 35.
15. IFN-α mRNA for use according to any one of embodiments 1 to 11, wherein the IFN-α mRNA is administered subcutaneously, intradermally, transdermally, epidermally, or topically, especially epidermally.
16. IFN-α mRNA for use according to any one of embodiments 1 to 15, wherein the IFN-α mRNA is administered at least twice within one month, preferably weekly.
17. IFN-α mRNA for use according to any one of embodiments 1 to 16, wherein the IFN-α mRNA is administered in an amount of 0.001µg to 100 mg per dose, preferably of 0.01µg to 100 mg per dose, more preferably of 0.1µg to 10mg per dose, especially of 1µg to 1mg per dose.
18. IFN-α mRNA for use according to any one of embodiments 1 to 17, wherein the IFN-α mRNA has a CAI of 0.8 or more and a GC content of 48.7% or more.
19. IFN-α mRNA for use according to any one of embodiments 1 to 18, wherein the IFN-α mRNA has a CAI of 0.8 to 0.99, preferably of 0.81 to 0.97, especially of 0.83 to 0.85.
20. IFN-α mRNA for use according to any one of embodiments 1 to 19, wherein the IFN-α mRNA has a GC content of 48.7% to 63.7%, preferably of 48.7% to 60%, especially of 48.7% to 58.0%.
21. IFN-α mRNA for use according to any one of embodiments 1 to 20, wherein the IFN-α mRNA has a CAI of 0.80 to 0.97 and a GC content of 48.7% to 63.7%.
22. IFN-α mRNA for use according to any one of embodiments 1 to 9 and 12 to 17, wherein the IFN-α mRNA has a CAI of 0.6 to 0.8 and an AU content of 48.7% to 65.0%.
22. IFN-α mRNA for use according to any one of embodiments 1 to 10 and 12 to 17, wherein the IFN-α mRNA has a CAI of 0.6 to 0.8, preferably of 0.6 to 0.7, especially of 0.65 to 0.75.
23. IFN-α mRNA for use according to any one of embodiments 1 to 9 and 11 to 17, wherein the IFN-α mRNA has an AU content of 48.7% to 65.0%, preferably of 51.3 to 60.0%.
24. Pharmaceutical formulation for use in the prevention and treatment of NMSC, preferably AK, BCC and SCC, especially AK, comprising the IFN-α mRNA as defined in any one of embodiments 1 to 23.
25. Pharmaceutical formulation for use according to embodiment 24, comprising a pharmaceutically acceptable carrier, preferably polymer based carriers, especially cationic polymers, including linear and branched PEI and viromers; lipid nanoparticles and liposomes, nanoliposomes, ceramide-containing nanoliposomes, proteoliposomes, cationic amphiphilic lipids e.g.: SAINT-Lipids, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, dry powders, poly(D-arginine), nanodendrimers, starch-based delivery systems, micelles, emulsions, sol-gels, niosomes, plasmids, viruses, calcium phosphate nucleotides, aptamers, peptides, peptide conjugates, vectorial tags, preferably small-molecule targeted conjugates, or viral capsid proteins, preferably bionanocapsules.
26. Pharmaceutical formulation for use according to embodiment 24 or 25, comprising cationic polymers including linear and branched PEI and viromers, lipid nanoparticles and liposomes, transfersomes, and nanoparticulates, preferably calcium phosphate nanoparticulates.
27. Pharmaceutical formulation for use according to embodiment 24, wherein the mRNA is non-complexed mRNA, and wherein preferably the non-complexed mRNA is contained in a suitable aqueous buffer solution, especially a physiological glucose buffered aqueous solution.
28. Pharmaceutical formulation for use according to any one of embodiments 24 to 27, wherein the formulation comprises a 1xHEPES buffered solution; a 1xPhosphate buffered solution, a Na-Citrate buffered solution; a Na-Acetate buffered solution; preferably additionally comprising glucose, especially 5% Glucose.
29. Kit for administering the IFN-α mRNA for use according to any one of embodiments 1 to 23 to a patient comprising
   - the IFN-α mRNA as defined in any one of embodiments 1 to 23, and
   - a skin delivery device.
30. Kit according to embodiment 29, wherein the skin delivery device is an intradermal delivery device, preferably selected from the group consisting of needle based injection systems, and needle-free injecton systems.
31. Kit according to embodiment 29, wherein the skin delivery device is a transdermal delivery device, preferably selected from the group consisting of transdermal patches, hollow and solid microneedle systems, microstructured transdermal systems, electrophoresis systems, and iontophoresis systems.
32. Kit according to embodiment 29, wherein the skin delivery device is an epidermal delivery device, preferably selected from the group consisting of needle free injection systems, laser based systems, especially Erbium YAG laser systems, and gene gun systems.
33. Method for treating and preventing NMSC, preferably AK, BCC and SCC, wherein the IFN-α mRNA as defined in any one of embodiments 1 to 23 is administered in an effective amount to a patient in need thereof.
34. m-RNA molecule comprising the nucleotide sequence as defined in any one of claims 1 to 23, except the native IFN-α mRNA sequences.

## Claims

1. Interferon alpha (IFN-α) messenger-RNA (mRNA), wherein the mRNA has a 5' CAP region, a 5' untranslated region (5'-UTR), a coding region encoding human IFN-α, a 3' untranslated region (3'-UTR) and a poly-adenosine Tail (poly-A tail), wherein the 5'-UTR or 3'-UTR or the 5'-UTR and the 3'-UTR are different from the native IFN-α mRNA.

2. IFN-α mRNA according to claim 1, wherein the IFN-α mRNA is selected from IFN-α type 1 mRNA (IFNa1), IFN-α type 2a mRNA (IF-Na2a), and IFN-α type 2b mRNA (IFNa2b).

3. IFN-α mRNA according to claim 1 or 2, wherein the poly-A tail comprises at least 100 adenosine monophosphates, preferably at least 120 adenosine monophosphates.

4. IFN-α mRNA according to any one of claims 1 to 3, wherein the 5'-UTR or 3'-UTR or the 5'-UTR and the 3'-UTR are different from the native IFN-α mRNA, preferably wherein the 5'-UTR or 3'-UTR or the 5'-UTR and the 3'-UTR contain at least one a stabilisation sequence, preferably a stabilisation sequence with the general formula (C/U)CCAN_{X}CCC(U/A)PyₓUC(C/U)CC (SEQ ID NO:38), wherein "x" is, independently in Nₓ and Pyₓ, an integer of 0 to 10, preferably of 0 to 5, especially 0, 1, 2, 4 and/or 5).

5. IFN-α mRNA according to any one of claims 1 to 4, wherein the 5'-UTR and/or 3'-UTR are the 5'-UTR and/or 3'-UTR of a different human mRNA than IFN-α, preferably selected from alpha Globin, beta Globin, Albumin, Lipoxygenase, ALOX15, alpha(1) Collagen, Tyrosine Hydroxylase, ribosomal protein 32L, eukaryotic elongation factor 1a (EEF1A1), 5'-UTR element present in orthopoxvirus, and mixtures thereof, especially selected from alpha Globin, beta Globin, alpha(1) Collagen, and mixtures thereof.

6. IFN-α mRNA according to any one of claims 1 to 5, wherein in the IFN-α mRNA, at least 5%, preferably at least 10%, preferably at least 30%, especially at least 50% of all
- cytidine residues are replaced by 5-methyl-cytidine residues, and/or
- cytidine residues are replaced by 2-amino-2-deoxy-cytidine residues, and/or
- cytidine residues are replaced by 2-fluoro-2-deoxy-cytidine residues, and/or
- cytidine residues are replaced by 2-thio-cytidine residues, and/or
- cytidine residues are replaced by 5-iodo-cytidine residues, and/or
- uridine residues are replaced by pseudo-uridine residues, and/or
- uridine residues are replaced by 1-methyl-pseudo-uridine residues, and/or
- uridine residues are replaced by 2-thio-uridine residues, and/or
- uridine residues are replaced by 5-methyl-uridine residues, and/or
- adenosine residues are replaced by N6-methyl-adenosine residues.

7. IFN-α mRNA according to any one of claims 1 to 6, wherein the IFN-α mRNA has a GC to AU ratio of at least 49.5%, preferably of at least 49.6, more preferred 50%, even more preferred, at least 55%, especially at least 60%.

8. IFN-α mRNA according to any one of claims 1 to 7, wherein the IFN-α mRNA has a codon adaption index (CAI) of at least 0.8, preferably at least 0.9.

9. IFN-α mRNA according to any one of claims 1 to 8, wherein the coding region of the IFN-α mRNA encodes
- human IFNa2a and is preferably SEQ ID NO:12, especially SEQ ID NOs: 2, 3, 5, 6, 7, 8, 9, 10, or 11; and/or
- human IFNa2b and is preferably SEQ ID NO:26, especially SEQ ID NOs: 19, 20, 22, or 25, and/or
- human IFNa1 and is preferably SEQ ID NO:36, especially SEQ ID NOs: 29, 30, 31, 32, 34, or 35.

10. IFN-α mRNA according to any one of claims 1 to 9, wherein the IFN-α mRNA is administered subcutaneously, intradermally, transdermally, epidermally, or topically, especially epidermally.

11. Pharmaceutical formulation comprising the IFN-α mRNA according to any one of claims 1 to 10.

12. Pharmaceutical formulation according to claim 11, comprising a pharmaceutically acceptable carrier, preferably polymer based carriers, especially cationic polymers, including linear and branched PEI and viromers; lipid nanoparticles and liposomes, nanoliposomes, ceramide-containing nanoliposomes, proteoliposomes, cationic amphiphilic lipids e.g.: SAINT-Lipids, both natural and synthetically-derived exosomes, natural, synthetic and semi-synthetic lamellar bodies, nanoparticulates, calcium phosphor-silicate nanoparticulates, calcium phosphate nanoparticulates, silicon dioxide nanoparticulates, nanocrystalline particulates, semiconductor nanoparticulates, dry powders, poly(D-arginine), nanodendrimers, starch-based delivery systems, micelles, emulsions, sol-gels, niosomes, plasmids, viruses, calcium phosphate nucleotides, aptamers, peptides, peptide conjugates, vectorial tags, preferably small-molecule targeted conjugates, or viral capsid proteins, preferably bionanocapsules.

13. Kit for administering the IFN-α mRNA according to any one of claims 1 to 10 to a patient comprising
- the IFN-α mRNA according to any one of claims 1 to 10, and
- a skin delivery device.

14. Kit according to claim 13, wherein the skin delivery device is
- an intradermal delivery device, preferably selected from the group consisting of needle based injection systems and needle-free injecton systems, or
- a transdermal delivery device, preferably selected from the group consisting of transdermal patches, hollow and solid microneedle systems, microstructured transdermal systems, electrophoresis systems, and iontophoresis systems, or
- an epidermal delivery device, preferably selected from the group consisting of needle free injection systems, laser based systems, especially Erbium YAG laser systems, and gene gun systems.


SEQUENCE LISTING

<110> Accanis Biotech F&E GmbH & Co KG

<120> Prevention and treatment of non-melanoma skin cancer (NMSC)

<130> R 72204

<140> PCT/EP2018/058036
<141> 2018-03-29

<150> EP 17164257.2
<151> 2017-03-31

<150> EP 17200162.0
<151> 2017-11-06

<160> 53

<170> BiSSAP 1.3

<210> 1
<211> 567
<212> RNA
<213> Homo sapiens

<400> 1
auggccuuga ccuuugcuuu acugguggcc cuccuggugc ucagcugcaa gucaagcugc 60

ucugugggcu gugaucugcc ucaaacccac agccugggua gcaggaggac cuugaugcuc 120

cuggcacaga ugaggaaaau cucucuuuuc uccugcuuga aggacagaca ugacuuugga 180

uuuccccagg aggaguuugg caaccaguuc caaaaggcug aaaccauccc uguccuccau 240

gagaugaucc agcagaucuu caaucucuuc agcacaaagg acucaucugc ugcuugggau 300

gagacccucc uagacaaauu cuacacugaa cucuaccagc agcugaauga ccuggaagcc 360

ugugugauac aggggguggg ggugacagag acuccccuga ugaaggagga cuccauucug 420

gcugugagga aauacuucca aagaaucacu cucuaucuga aagagaagaa auacagcccu 480

ugugccuggg agguugucag agcagaaauc augagaucuu uuucuuuguc aacaaacuug 540

caagaaaguu uaagaaguaa ggaauga 567

<210> 2
<211> 567
<212> RNA
<213> Homo sapiens

<400> 2
auggcacuga cauuugcccu gcucguugcu cuuuuggucc uuuccugcaa gaguagcugc 60

ucuguuggcu gugauuugcc ccaaacccac ucucucgguu caaggagaac ucugaugcug 120

cuugcccaaa ugcggaagau uagccuguuc ucaugccuga aagaccggca ugauuucggc 180

uuuccucagg aggaauuugg gaaccaguuc cagaaagcgg aaaccauucc cguccuucac 240

gagaugaucc agcagaucuu caaccuguuu ucuaccaagg auuccagugc ugcuugggau 300

gagacacugc uggacaaguu cuacacugag cucuaucagc agcugaauga ccuggaagcc      360

ugugugaucc aaggaguagg agugacugag acaccacuca ugaaagagga cuccauacuc      420

gcagugcgca aguacuucca gaggauuacc cuguaucuga aggagaagaa auacaguccg      480

ugugcauggg aaguggugag agccgagauc augcguagcu uuucccuguc aacgaaucug      540

caggaaagcu ugcgaagcaa agaauga                                          567


<210>  3
<211>  567
<212>  RNA
<213>  Homo sapiens

<400>  3
auggcacuga cauucgcccu gcucgucgcu cuccucgucc ucuccugcaa gaguagcugc       60

ucugucggcu gugauuugcc ccaaacccac ucccucgguu ccaggcgcac ucugaugcug      120

cucgcccaga ugcggaagau uagccuguuc ucaugccuga aggaccggca ugauuucggc      180

uucccucagg aggaauucgg gaaccaguuc cagaaggcgg agaccauccc cguccuccac      240

gagaugaucc agcagaucuu caaccuguuc ucuaccaagg acuccagugc ugcuugggac      300

gagacccugc ucgacaaguu cuacacugag cucuaccagc agcugaacga ccuggaggcc      360

ugugugaucc aaggagucgg agugacugag acaccacuca ugaaggagga cuccauccuc      420

gcaguccgca aguacuucca gaggaucacc cuguaccuga aggagaagaa guacaguccg      480

ugugcauggg agguggugag agccgagauc augcguagcu ucucccuguc aacgaaccug      540

caggagagcc uccgaagcaa ggaguga                                          567


<210>  4
<211>  567
<212>  RNA
<213>  Homo sapiens

<400>  4
auggcacuga cauuugcccu gcucguugcu cuuuuggucc uuucuugcaa gaguagcugu       60

ucuguuggau gugauuugcc ucaaacucau ucuuuggguu caagaagaac uuugaugcuu      120

cuugcacaaa ugagaaagau uagccuuuuc ucauguuuga aagaucgaca ugauuucgga      180

uuuccucaag aagaauuugg uaaccaauuc caaaaagcug aaaccauucc uguccuucau      240

gaaaugaucc aacaaaucuu caaucuuuuu ucuacuaagg auucuagugc ugcuugggau      300

gaaacacuuc uugauaaguu cuacacugaa cucuaucaac agcugaauga cuuggaagcc      360

uguguuaucc aaggaguugg aguuacugag acaccacuca ugaaagaaga uuccauacuc      420

gcaguucgca aguacuucca aaggauuacc uuguaucuga aggaaaagaa auacaguccu      480

ugugcauggg aagugguuag agcugaaauc augcguagcu uuucccuguc aacgaauuug      540

caggaaagcu ugcgaagcaa agaauga 567

<210> 5
<211> 567
<212> RNA
<213> Homo sapiens

<400> 5
auggcccuga cauucgcucu gcugguggcc cugcuggugc ugagcugcaa gagcagcugu 60

agcgugggcu gcgaccugcc ucagacacac agccugggca gcagacggac ccugaugcug 120

cuggcccaga ugcggaagau cagccuguuc agcugccuga aggaccggca cgacuucggc 180

uucccucagg aagaguucgg caaccaguuc cagaaggccg agacaauccc cgugcugcac 240

gagaugaucc agcagaucuu caaccuguuc uccaccaagg acagcagcgc cgccugggac 300

gagacacugc uggacaaguu cuacaccgag cuguaccagc agcugaauga ccuggaagcc 360

ugcgugaucc agggcguggg cgugacagag acaccccuga ugaaggaaga uagcauccug 420

gccgugcgca aguacuucca gcggaucacc cuguaccuga aagagaagaa guacagcccc 480

ugcgccuggg aggucgugcg ggccgagauc augagaagcu ucagccugag caccaaccug 540

caggaaagcc ugcggagcaa agaguga 567

<210> 6
<211> 567
<212> RNA
<213> Homo sapiens

<400> 6
auggcucuca ccuucgcccu gcugguggca cuucuugucc ucucauguaa aucuagcugu 60

agcguuggcu gcgaucugcc ucagacucau agucugggau cucggaggac gcuuaugcug 120

uuggcccaga ugaggaagau cucccuguuc uccugucuca aagaccggca cgauuuuggc 180

uucccacagg aggaguuugg gaaccaguuc cagaaagcug agaccauccc ggugcuucau 240

gaaaugaucc agcagaucuu caaucuguuc aguacaaagg auaguucugc ugcuugggac 300

gagacacucc ucgacaaauu uuacacugaa cuguaucagc aguugaacga ccuugaggcu 360

ugcguuauuc agggagucgg ugugacagaa acuccccuca ugaaggagga cagcauccuc 420

gccguucgaa aguauuucca acgaaucaca cuguaucuua aggagaaaaa guacagccca 480

ugugccuggg agguuguccg ggcugagaua augcgaaguu ucucacugag uacaaacuug 540

caggagagcc uccgaucaaa ggaguga 567

<210> 7
<211> 567
<212> RNA
<213> Homo sapiens

<400> 7

auggcucuua cguucgcucu uuugguugcc cucuuggugc ugaguuguaa auccucaugu 60

uccgugggau gcgaucugcc gcagacucac ucucuuggca guagaaggac ccugaugcug 120

cuggcucaaa ugcggaagau uagucuguuc uccugccuga aggaccggca ugacuucggu 180

uuuccacagg aggaauucgg aaaccaguuu cagaaggcug agacaauccc ugugcugcac 240

gaaaugaucc aacagauuuu caaccuuuuc ucaaccaagg acuccucagc cgccugggac 300

gaaacacugc uggauaaauu cuauaccgag cucuaccaac agcugaacga uuuggaggca 360

ugugucaucc agggggucgg ggucacugag acuccacuga ugaaggaaga cuccauucuc 420

gcgguaagga aauacuucca gaggaucacg cuguaccuga aggaaaagaa auacagcccu 480

ugcgcauggg aggugguucg cgcugagauc augcggagcu ucagucugag cacuaaucug 540

caggaaagcc ucaggucaaa ggaauag 567

<210> 8
<211> 567
<212> RNA
<213> Homo sapiens

<400> 8
auggcuuuga ccuucgcccu ccugguggcc cuguuggugc ucucaugcaa auccagcugc 60

ucagugggcu gcgauuugcc ccagacccac ucacugggaa guaggaggac uuugaugcug 120

uuggcucaga ugcggaaaau cucucuguuc uccugccuga aggauaggca ugacuuuggu 180

uuuccucaag aggaguuugg aaaccaguuc caaaaggccg aaaccauccc ugugcuccac 240

gaaaugaucc agcagauauu uaaccuuuuc ucuacuaaag auuccagcgc cgcaugggac 300

gagacccugc uugacaaguu cuacacugag cucuaccagc agcucaacga ccuggaggcg 360

ugugugaucc agggugucgg ugugacggag acuccccuua ugaaagagga uucuauccug 420

gcagugcgga aauauuuuca gagaaucacg cuguauuuga aggagaagaa guauagcccc 480

ugcgccuggg aaguggugag ggcugaaauu augcgcaguu ucagcuugag caccaaccug 540

caggaauccc uucgguccaa ggaauaa 567

<210> 9
<211> 567
<212> RNA
<213> Homo sapiens

<400> 9
auggcccuga ccuucgcacu gcugguggca cugcugguuc uuuccuguaa gagcaguugc 60

agcguuggau gugaccugcc acagacucau ucucugggua gccggcgcac ucuuaugcuc 120

cucgcacaaa ugaggaagau uagucuguuc agcugucuca aagauagaca cgauuuuggc 180

uucccucagg aagaauucgg aaaccaguuu cagaaggccg agaccauccc cguguugcau 240

gagaugauuc agcagaucuu caaccuguuu ucuaccaagg acagcuccgc ugcuugggac 300

gagacucugc uggacaaguu cuacacugag cucuaccagc agcugaacga ccuugaagcc 360

ugcguuauuc agggcgucgg cgugacagag acuccacuga ugaaagagga caguauccug 420

gccgugcgga aguauuuuca gaggauuaca cuuuauuuga aggaaaagaa guacuccccc 480

ugcgcauggg aagugguaag ggcugaaauc augcggagcu uuucccuguc uaccaaccug 540

caggaauccc ugagauccaa agaauaa 567

<210> 10
<211> 567
<212> RNA
<213> Homo sapiens

<400> 10
auggcacuga cauuugccuu gcucgucgcc cugcuuguuc uguccuguaa gagcuccugc 60

ucaguuggcu gcgaucuucc ucaaacccau agccucggua gccgccgaac ccugaugcug 120

cuggcccaga ugcgcaagau uagucuguuc ucuugucuga aagacaggca cgauuucgga 180

uucccacagg aggaguucgg caaucaauuc cagaaagcgg agaccauccc cgugcuucac 240

gagaugauuc agcaaauuuu caaccuguuc aguacuaaag auucuagcgc ugcgugggac 300

gagacccugc uggacaaauu cuauacagaa cucuaucagc agcugaacga ucuggaggcc 360

uguguuaucc aggggguagg ugugaccgaa accccucuua ugaaggaaga uuccauccug 420

gccguuagga aauacuucca gcgcaucaca uuguaccuga aggagaaaaa guacagcccu 480

ugcgcauggg agguggucag agcugaaauc augcgaucau uuagucucag uacuaaucuc 540

caagagucac ugcgcuccaa ggaguaa 567

<210> 11
<211> 567
<212> RNA
<213> Homo sapiens

<400> 11
auggcccuga ccuucgcccu gcugguggcc cugcuggugc ugagcugcaa gagcagcugc 60

agcgugggcu gcgaccugcc ccagacccac agccugggca gccgccgcac ccugaugcug 120

cuggcccaga ugcgcaagau cagccuguuc agcugccuga aggaccgcca cgacuucggc 180

uucccccagg aggaguucgg caaccaguuc cagaaggccg agaccauccc cgugcugcac 240

gagaugaucc agcagaucuu caaccuguuc agcaccaagg acagcagcgc cgccugggac 300

gagacccugc uggacaaguu cuacaccgag cuguaccagc agcugaacga ccuggaggcc 360

ugcgugaucc agggcguggg cgugaccgag accccccuga ugaaggagga cagcauccug 420

gccgugcgca aguacuucca gcgcaucacc cuguaccuga aggagaagaa guacagcccc 480

ugcgccuggg agguggugcg cgccgagauc augcgcagcu ucagccugag caccaaccug 540

caggagagcc ugcgcagcaa ggaguaa 567

<210> 12
<211> 567
<212> RNA
<213> Homo sapiens

<220>
<221> variation
<222> 81,294,360,375,381,387,426,480,498,516,534
<223> any nucleotide

<400> 12
auggchyuba cvuuygchyu byusgubgch cubyubgubc ubwshugyaa rwsywshugy 60

wshgubggmu gygayyukcc ncarachcay wshcubgghw shmgvmgvac byukaugcus 120

yubgchcara ugmgsaarau ywsycuguuc wshugycusa argaymgvca ygayuuyggh 180

uuycchcarg argaruuygg vaaycaruuy caraargcbg aracmauycc bgusyubcay 240

garaugauyc arcarauhuu yaaycukuuy wshachaarg aywsywshgc ygcnugggay 300

garachcusc ubgayaaruu yuayachgar cusuaycarc agyusaayga yyukgargcn 360

ugygubauyc arggnguvgg ngusacngar achcchcuba ugaargarga ywsyauhcus 420

gcvgunmgva aruayuuyca rmgvauyacv yukuayyuka argaraaraa ruaywsyccn 480

ugygcmuggg argubgunmg vgcygarauh augmgnwshu uywshyusws hacnaayyus 540

cargarwsmy ubmgvwsmaa rgarurr 567

<210> 13
<211> 567
<212> RNA
<213> Homo sapiens

<220>
<221> variation
<222> 81,201,294,360,375,378,381,387,426,480,498,516,534
<223> any nucleotide

<400> 13
auggchyuba cvuuygchyu byusgubgch cubyubgubc ubwshugyaa rwsywshugy 60

wshgubggmu gygayyukcc ncarachcay wshyubgghw shmgvmgvac byukaugcub 120

yubgchcara ugmgvaarau ywsycukuuc wshugyyusa argaymgvca ygayuuyggh 180

uuycchcarg argaruuygg naaycaruuy caraargcbg aracmauycc bgusyubcay 240

garaugauyc arcarauhuu yaaycukuuy wshachaarg aywsywshgc ygcnugggay 300

garachcubc ubgayaaruu yuayachgar cusuaycarc agyusaayga yyukgargcn 360

ugygubauyc arggngungg ngubacngar achcchcuba ugaargarga ywsyauhcus 420

gcvgunmgva aruayuuyca rmgvauyacv yukuayyuka argaraaraa ruaywsyccn 480

ugygcmuggg argubgunmg vgcygarauh augmgnwshu uywshyusws hacnaayyus 540

cargarwsmy ubmgvwsmaa rgarurr                                    567

<210> 14
<211> 567
<212> RNA
<213> Homo sapiens

<220>
<221> variation
<222> 21,81,201,294,312,360,375,378,381,387,423,426,450,480,498,516,534,552
<223> any nucleotide

<400> 14
auggchyuba cvuuygchyu nyusgubgch cubyubgubc ubwshugyaa rwshwshugy      60

wshgubggmu gygayyukcc ncarachcay wshyubgghw shmgvmgvac byukaugcub     120

yubgchcara ugmgvaarau ywsycukuuc wshugyyusa argaymgvca ygayuuyggh     180

uuycchcarg argaruuygg naaycaruuy caraargcbg aracmauycc bgusyubcay     240

garaugauyc arcarauhuu yaaycubuuy wshachaarg aywshwshgc ygcnugggay     300

garachcubc ungayaaruu yuayachgar cusuaycarc agyusaayga yyukgargcn     360

ugygubauhc arggngungg ngubacngar achcchcuba ugaargarga ywsyauhcus     420

gcngunmgva aruayuuyca rmgvauyacn yubuayyuka argaraaraa ruaywsyccn     480

ugygcmuggg argubgunmg vgchgarauh augmgnwshu uywshyusws hacnaayyus     540

cargarwshy unmgvwshaa rgarurr                                    567

<210> 15
<211> 188
<212> PRT
<213> Homo sapiens

<400> 15

<210> 16
<211> 72
<212> RNA
<213> Homo sapiens

<220>
<221> 5'UTR
<222> 1..72
<223> /standard_name="alpha-Globin"

<400> 16
gggagacata aaccctggcg cgctcgcggc ccggcactct tctggtcccc acagactcag      60

agagaaccca cc                                                          72


<210> 17
<211> 230
<212> RNA
<213> Homo sapiens

<220>
<221> 3'UTR
<222> 1..230
<223> /standard_name="alpha-Globin"

<220>
<221> polyA_signal
<222> 110..230

<400> 17
gctggagcct cggtggccat gcttcttgcc ccttgggcct ccccccagcc cctcctcccc      60

ttcctgcacc cgtacccccg tggtctttga ataaagtctg agtgggcggc aaaaaaaaaa     120

aaaaaaaaaa aaaaaaaaaa aaaaaaaaaa aaaaaaaaaa aaaaaaaaaa aaaaaaaaaa     180

aaaaaaaaaa aaaaaaaaaa aaaaaaaaaa aaaaaaaaaa aaaaaaaaaa               230


<210> 18
<211> 567
<212> RNA
<213> Homo sapiens

<400> 18
auggccuuga ccuuugcuuu acugguggcc cuccuggugc ucagcugcaa gucaagcugc      60

ucugugggcu gugaucugcc ucaaacccac agccugggua gcaggaggac cuugaugcuc     120

cuggcacaga ugaggagaau cucucuuuuc uccugcuuga aggacagaca ugacuuugga     180

uuuccccagg aggaguuugg caaccaguuc caaaaggcug aaaccauccc uguccuccau     240

gagaugaucc agcagaucuu caaucucuuc agcacaaagg acucaucugc ugcuugggau     300

gagacccucc uagacaaauu cuacacugaa cucuaccagc agcugaauga ccuggaagcc     360

ugugugauac aggggguggg ggugacagag acuccccuga ugaaggagga cuccauucug     420

gcugugagga aauacuucca aagaaucacu cucuaucuga aagagaagaa auacagcccu 480

ugugccuggg agguugucag agcagaaauc augagaucuu uuucuuuguc aacaaacuug 540

caagaaaguu uaagaaguaa ggaauga 567

<210> 19
<211> 567
<212> RNA
<213> Homo sapiens

<400> 19
auggcccuga ccuucgcccu gcugguggcc cugcuggugc ugagcugcaa gagcagcugc 60

agcgugggcu gcgaccugcc ccagacccac agccugggca gccgccgcac ccugaugcug 120

cuggcccaga ugcgccgcau cagccuguuc agcugccuga aggaccgcca cgacuucggc 180

uucccccagg aggaguucgg caaccaguuc cagaaggccg agaccauccc cgugcugcac 240

gagaugaucc agcagaucuu caaccuguuc agcaccaagg acagcagcgc cgccugggac 300

gagacccugc uggacaaguu cuacaccgag cuguaccagc agcugaacga ccuggaggcc 360

ugcgugaucc agggcguggg cgugaccgag acccccugaa ugaaggagga cagcauccug 420

gccgugcgca aguacuucca gcgcaucacc cuguaccuga aggagaagaa guacagcccc 480

ugcgccuggg agguggugcg cgccgagauc augcgcagcu ucagccugag caccaaccug 540

caggagagcc ugcgcagcaa ggaguaa 567

<210> 20
<211> 567
<212> RNA
<213> Homo sapiens

<400> 20
auggcccuga cauucgcucu gcugguggcc cugcuggugc ugagcugcaa gagcagcugu 60

agcgugggcu gcgaccugcc ucagacacac agccugggca gcagacggac ccugaugcug 120

cuggcccaga ugcggagaau cagccuguuc agcugccuga aggaccggca cgacuucggc 180

uucccucagg aagaguucgg caaccaguuc cagaaggccg agacaauccc cgugcugcac 240

gagaugaucc agcagaucuu caaccuguuc uccaccaagg acagcagcgc cgccugggac 300

gagacacugc uggacaaguu cuacaccgag cuguaccagc agcugaauga ccuggaagcc 360

ugcgugaucc agggcguggg cgugacagag acaccccuga ugaaggaaga uagcauccug 420

gccgugcgca aguacuucca gcggaucacc cuguaccuga aagagaagaa guacagcccc 480

ugcgccuggg aggucgugcg ggccgagauc augagaagcu ucagccugag caccaaccug 540

caggaaagcc ugcggagcaa agaguga 567

<210> 21

<211> 567
<212> RNA
<213> Homo sapiens

<400> 21
auggcuuuga cauucgcacu guuggucgcc cugcuugugc ucucaugcaa aagcaguugu      60

uccguggguu gcgauuugcc acagacucac agucugggau cucgccgcac ucugaugcuu      120

cucgcgcaaa ugcgccggau uagucuuuuc uccugucuga aggauagaca cgacuuuggg      180

uuuccccagg aggaguuugg gaaucaguuc caaaaggcgg aaacuauucc uguccuucac      240

gaaaugaucc agcagauauu uaauuuguuc ucaacaaaag auucaucagc ugcaugggac      300

gaaacccugc uggauaaguu cuacacggag cucuaccagc agcugaauga ucucgaagcc      360

ugugucaucc agggcguagg agugacagaa accccacuga ugaaggagga uucaauccug      420

gcggugagga aguauuucca gcggaucacc cuguaucuga aggaaaagaa guauucccca      480

ugugcuuggg aggugguccg agcagagauc augaggagcu ucucucucuc aacaaaucug      540

caggagaguc uuagguccaa ggaguga                                          567

<210> 22
<211> 567
<212> RNA
<213> Homo sapiens

<400> 22
auggcucuca cuuucgcacu ccucguggca cugcuugugc uguccugcaa aaguucuugc      60

agcguggggu gcgaccuccc acagacccac ucacuggggu caaggcgcac ccugaugcug      120

cuggcccaga ugagacgaau uucccuguuu uccugccuca aggaucggca ugacuucggc      180

uuuccccaag aggaguucgg caaccaguuc cagaaagccg agaccauccc ugugcugcau      240

gagaugaucc agcaaauauu caaucucuuu uccaccaagg acagcuccgc cgccugggau      300

gagacauugc ucgauaaguu uuauacugaa cuguaccagc agcugaacga ucuugaggcc      360

uguguaauac aggguguagg cgugacagag accccccuua ugaaggagga cucaauucug      420

gcagucagga aauauuucca gcggauaacu cuguaccuga aggagaaaaa guauagucca      480

ugugcuuggg aaguggugcg ggccgagauc augcgcagcu uuucacuuag uacuaaucuc      540

caggagucuu ugaggucaaa ggaguga                                          567

<210> 23
<211> 567
<212> RNA
<213> Homo sapiens

<400> 23
auggcacuua ccuucgcccu uuugguggca cugcugguac ucucaugcaa gaguaguugc      60

aguguggggu gcgaucuccc acagacucac uccuugggau caaggcggac gcucaugcuc      120

cuggcucaaa ugagaagaau uuccuuguuc ucaugcuuga aagauagaca cgacuuuggc 180

uucccacagg aggaauuugg gaaccaguuc caaaaagcug agacgauccc aguccugcac 240

gagaugauac agcagaucuu uaaucuuuuu uccaccaagg acagcagugc cgccugggac 300

gagacucucc uugauaaauu cuacacugaa cucuaucaac aguugaauga cuuggaagcc 360

ugugugaucc aaggagucgg cgugaccgaa acaccacuua ugaaggagga cagcauccuu 420

gccguuagaa aguacuuuca acggaucacu cucuaucuca aagagaaaaa guacaguccc 480

ugugcuuggg aggugguccg cgccgaaauc augaggaguu ucagccucuc cacuaaucuu 540

caagaauccc uccgaagcaa agaauga 567

<210> 24
<211> 567
<212> RNA
<213> Homo sapiens

<400> 24
auggcucuca ccuucgcacu guugguggcu cuccuugugc ugagcuguaa aucuuccugu 60

ucugucgguu gcgauuugcc gcagacacac agccugggga gucggagaac ccugauguug 120

cuggcucaga ugcggagaau uucucuguuc aguugccuua aggaccgcca ugauuuuggg 180

uucccccagg aggaauuugg aaaucaguuu cagaaggcgg agacgauccc gguucugcac 240

gaaaugaucc agcagaucuu caauuuguuu ucaaccaagg acuccucugc ugccugggau 300

gaaacacugc uggacaaguu cuauaccgag cuguaccaac agcugaacga ucuugaggca 360

ugugugauuc aaggagucgg ggucaccgaa accccacuga ugaaagaaga uucuauucug 420

gcugugagaa aauauuucca aagaauaacu cucuaccuga aggagaagaa auauucacca 480

ugugccuggg aagucgugcg cgcugagaua augcgcagcu uuagucuuag uacaaaccug 540

caggagucuc ugcgcucuaa ggaguga 567

<210> 25
<211> 567
<212> RNA
<213> Homo sapiens

<400> 25
auggcccuua cuuuugcccu gcugguggca cuucuggucc ucucuugcaa gucaagcugc 60

aguguuggau gcgaucuucc ucagacacac ucccugggga guagaagaac ucugauguug 120

cucgcucaga ugcgcagaau aagucuguuu aguugucuga aggaucgcca cgauuucggg 180

uuuccacagg aagaguucgg caaccaguuc cagaaagcug agacuauccc uguacuucac 240

gaaaugauuc agcagaucuu uaaucuguuc uccaccaagg acuccucugc cgcuugggau 300

gagacucucc uggacaaauu uuacacugag cuguaucaac agcugaauga ucuggaagcc 360

ugcguaaucc aggggguggg ugugacagaa acaccguuga ugaaggaaga cuccauacuu 420

gcugugcgca aguacuuuca gcggaucacu cuguaucuga aagagaagaa auauucuccu     480

ugcgcuuggg aggucgucag ggcggaaaua augcggucuu ucagccucuc uaccaaucug     540

caggaguccc ugagaucuaa ggaauga                                         567

<210> 26
<211> 567
<212> RNA
<213> Homo sapiens

<400> 26
auggcycuba chuuygchcu scusguggcm cukcukgusc uswsyugcaa rwshwsyugy      60

agygukggvu gcgaycubcc hcagacmcac wsmcugggsw shmgvmgvac ycugaugyug     120

cusgcycaga ugmgvmgmau hwsycuguuy wsyugycusa aggaycgsca ygayuucggs     180

uuycchcarg argaguucgg caaccaguuc cagaargcyg agachauccc ygurcukcay     240

garaugauyc agcaraumuu yaaycusuuy wscaccaagg acwscwsygc cgcyugggay     300

gagachyusc usgayaaruu yuayacygar cuguaycarc agcugaayga ycukgargcc     360

ugyguraumc agggbgurgg ygugacmgar acmccsyuka ugaaggarga ywsmauhcuk     420

gchgusmgsa aruayuuyca gcgsaumacy cuguaycuga argagaaraa ruaywsycch     480

ugygcyuggg argusgusmg sgcsgaraum augmgvwsyu uywsmcubws yacyaaycus     540

caggarwsyy ugmgvwshaa rgarura                                         567

<210> 27
<211> 188
<212> PRT
<213> Homo sapiens

<400> 27
Met Ala Leu Thr Phe Ala Leu Leu Val Ala Leu Leu Val Leu Ser Cys
1               5                   10                  15
Lys Ser Ser Cys Ser Val Gly Cys Asp Leu Pro Gln Thr His Ser Leu
            20                  25                  30
Gly Ser Arg Arg Thr Leu Met Leu Leu Ala Gln Met Arg Arg Ile Ser
        35                  40                  45
Leu Phe Ser Cys Leu Lys Asp Arg His Asp Phe Gly Phe Pro Gln Glu
    50                  55                  60
Glu Phe Gly Asn Gln Phe Gln Lys Ala Glu Thr Ile Pro Val Leu His
65                  70                  75                  80
Glu Met Ile Gln Gln Ile Phe Asn Leu Phe Ser Thr Lys Asp Ser Ser
                85                  90                  95
Ala Ala Trp Asp Glu Thr Leu Leu Asp Lys Phe Tyr Thr Glu Leu Tyr
            100                 105                 110
Gln Gln Leu Asn Asp Leu Glu Ala Cys Val Ile Gln Gly Val Gly Val
        115                 120                 125
Thr Glu Thr Pro Leu Met Lys Glu Asp Ser Ile Leu Ala Val Arg Lys
    130                 135                 140
Tyr Phe Gln Arg Ile Thr Leu Tyr Leu Lys Glu Lys Lys Tyr Ser Pro
145                 150                 155                 160
Cys Ala Trp Glu Val Val Arg Ala Glu Ile Met Arg Ser Phe Ser Leu
                165                 170                 175

Ser Thr Asn Leu Gln Glu Ser Leu Arg Ser Lys Glu
            180                 185

<210> 28
<211> 570
<212> RNA
<213> Homo sapiens

<400> 28
auggccucgc ccuuugcuuu acugaugguc cugguggugc ucagcugcaa gucaagcugc      60

ucucugggcu gugaucuccc ugagacccac agccuggaua acaggaggac cuugaugcuc     120

cuggcacaaa ugagcagaau cucuccuucc uccugucuga uggacagaca ugacuuugga     180

uuuccccagg aggaguuuga uggcaaccag uuccagaagg cuccagccau cucuguccuc     240

caugagcuga uccagcagau cuucaaccuc uuuaccacaa aagauucauc ugcugcuugg     300

gaugaggacc uccuagacaa auucugcacc gaacucuacc agcagcugaa ugacuuggaa     360

gccuguguga ugcaggagga gaggguggga gaaacucccc ugaugaaugc ggacuccauc     420

uuggcuguga agaaauacuu ccgaagaauc acucucuauc ugacagagaa gaaauacagc     480

ccuugugccu gggagguugu cagagcagaa aucaugagau cccucucuuu aucaacaaac     540

uugcaagaaa gauuaaggag gaaggaauaa                                      570


<210> 29
<211> 570
<212> RNA
<213> Homo sapiens

<400> 29
auggccucuc cauucgcccu gcugauggug cugguggugc ugagcugcaa gagcagcugc      60

agccugggcu gcgaccugcc ugagacacac agccuggaca accggcggac ccugaugcug     120

cuggcccaga ugagcagaau cagccccagc agcugccuga uggaccggca cgauuucggc     180

uucccucagg aagaguucga cggcaaccag uuccagaagg ccccugccau cagcgugcug     240

cacgagcuga uccagcagau cuucaaccug uucaccacca aggacagcag cgccgccugg     300

gacgaggacc ugcuggauaa guucugcacc gaacuguauc agcagcugaa cgaccuggaa     360

gccugcguga ugcaggaaga gagagugggc gagacacccc ugaugaacgc cgacucuauc     420

cuggccguga agaaguacuu ucggcggauc acccuguacc ugaccgagaa aaaguacagc     480

cccugcgccu gggaagucgu gcgggccgag aucaugagaa gccugagccu gagcaccaac     540

cugcaggaac ggcugcggcg gaaagaguaa                                      570


<210> 30
<211> 570
<212> RNA
<213> Homo sapiens

<400> 30

auggccagcc ccuucgcccu gcugauggug cugguggugc ugagcugcaa gagcagcugc 60

agccugggcu gcgaccugcc cgagacccac agccuggaca accgccgcac ccugaugcug 120

cuggcccaga ugagccgcau cagccccagc agcugccuga uggaccgcca cgacuucggc 180

uucccccagg aggaguucga cggcaaccag uuccagaagg cccccgccau cagcgugcug 240

cacgagcuga uccagcagau cuucaaccug uucaccacca aggacagcag cgccgccugg 300

gacgaggacc ugcuggacaa guucugcacc gagcuguacc agcagcugaa cgaccuggag 360

gccugcguga ugcaggagga gcgcgugggc gagacccccc ugaugaacgc cgacagcauc 420

cuggccguga agaaguacuu ccgccgcauc acccuguacc ugaccgagaa gaaguacagc 480

cccugcgccu gggagguggu gcgcgccgag aucaugcgca gccugagccu gagcaccaac 540

cugcaggagc gccugcgccg caaggaguaa 570

<210> 31
<211> 570
<212> RNA
<213> Homo sapiens

<400> 31
auggccucuc ccuucgcuuu gcugaugguu cucgugguuc ucagcugcaa guccuccugu 60

agucuggggu gugaccuucc cgagacucac agccuugaca accgacggac ucugaugcug 120

cuggcccaga ugagucgcau auccccuucu ucuugcuuga uggauagaca cgauuucggc 180

uucccucagg aggaguucga ugggaaccaa uuccagaaag cgccugcgau cagcguacuc 240

caugagcuga uccagcagau cuuuaauuug uucacaacga aggacagcag ugcugcuugg 300

gacgaagacc ugcuggacaa guucuguaca gaauuguacc agcagcugaa ugaccucgag 360

gccuguguga ugcaggagga aagagucggc gagacuccuc ucaugaacgc cgacagcauc 420

cucgccguga agaaguauuu ccggcggauc acccucuauc ugacagagaa gaaguacucc 480

cccugcgccu gggagguggu gcgagccgaa auaaugcgca gccucucucu gucaacuaau 540

cuccaggagc ggcuucggcg aaaggaguga 570

<210> 32
<211> 570
<212> RNA
<213> Homo sapiens

<400> 32
auggcaucuc cauucgcucu gcugauggug cugguggucc ugucauguaa gagcagcugc 60

ucccuggggu gcgaucugcc agagacccac agccuugaca acagaagaac cuugaugcuc 120

uuggcccaaa ugucaagaau aagcccuagc ucaugucuga uggaccggca ugauuucggc 180

uuuccgcagg aggaguucga cggaaaucag uuucagaaag caccagcaau aagcgugcuc 240

cacgaacuca uucagcagau uuucaaucug uuuacuacaa aagauucauc cgcugcuugg 300

gaugaagacu ugcuugacaa guucugcacu gagcucuacc aacaacugaa cgaucuggag 360

gcaugcguca ugcaagagga gagagugggg gaaacccccc ugaugaacgc ugauuccaua 420

cuggccguga agaaauauuu ccgcagaauc acucuguacu ugacugagaa gaaguauucu 480

cccugcgcuu gggagguggu gcgcgcugag auaaugcggu ccuugucacu cagcaccaau 540

cuucaagagc ggcugaggcg caaggaauga 570

<210> 33
<211> 570
<212> RNA
<213> Homo sapiens

<400> 33
auggccucuc ccuucgcucu ucucaugguc cuugucguuc ugaguuguaa gagcaguugu 60

uccuugggcu gugacuugcc cgaaacucac agucuggaca accgccgcac gcucaugcuc 120

cucgcccaga ugucacggau cagcccguca aguugccuga uggacaggca cgauuucggg 180

uucccacagg aagaguucga ugguaaucaa uuccaaaaag cuccugcaau uucuguacug 240

caugaacuua uucagcagau cuucaaccuc uuuaccacua aagauuccuc ugccgccugg 300

gaugaggauu ugcucgauaa guucugcacc gaacucuacc agcagcugaa cgaccucgag 360

gcuuguguua ugcaggaaga gcgggugggg gagacacccc ucaugaacgc cgacagcauu 420

cuggccguga aaaaguacuu uagaagaaua acgcuguacc ucaccgaaaa gaaauacucc 480

cccugcgcuu gggaggucgu gcgcgccgag auaaugcgcu cacucucuuu gagcacaaau 540

cuccaagaac ggcugaggag aaaagaguga 570

<210> 34
<211> 570
<212> RNA
<213> Homo sapiens

<400> 34
auggcaucuc cuuucgcucu gcuuauggug cugguugugc ucucuugcaa guccagcugu 60

agccuuggau gcgaccuucc ugagacccau ucucuggaua accggaggac gcucaugcug 120

cuggcacaga ugagucggau cagcccgucc agcugucuca uggacaggca cgacuuuggu 180

uuuccccagg aggaguucga ugguaaccag uuccagaagg cgccagcaau aagcgugcug 240

cacgagcuga uucagcagau cuuuaaccuc uucacuacua aggacaguag cgcugcaugg 300

gacgaggauc uguuggauaa guucuguacg gaacuguauc agcagcucaa ugaucucgaa 360

gcauguguga ugcaagaaga gcgcgucggu gaaacuccac ugaugaacgc ugauagcauc 420

cuggccguua agaaauacuu uaggcgcauu acucuguauc ugacugagaa aaaguauagc 480

ccaugcgcau gggagguagu gcgagccgag aucaugcgca gccugucauu gucaacuaac 540

cuccaggaac gccuccgacg aaaggaguga                                    570


<210> 35
<211> 570
<212> RNA
<213> Homo sapiens

<400> 35
auggcaucac ccuucgcucu gcugaugguc cugguggugc ugucauguaa auccagcugc      60

agcuugggau gcgaccugcc ggaaacacau ucccucgaca auaggcggac gcugaugcug     120

cucgcucaga ugucccgcau aagcccauca agcugccuca uggaccggca cgauuuuggu     180

uucccacagg aggaguucga uggaaaccag uuccagaagg cucccgcuau cuccguucug     240

caugagcuua uucagcagau uuucaaccuc uuuacgacaa aggauucauc cgccgccugg     300

gaugaagacc ugcuggauaa guucuguacc gaguuguacc aacagcugaa cgaucucgaa     360

gccuguguca ugcaggagga gcgcgugggg gagacucccu ugaugaacgc agacucaauu     420

cuugcaguga agaaauacuu ucgccggauu acucuuuauc ucaccgagaa gaaguacagu     480

cccugcgcau gggaagucgu ucgggccgaa aucaugcggu cccugucccu uuccaccaac     540

cugcaggaac gccuucgcag aaaagaguaa                                    570


<210> 36
<211> 570
<212> RNA
<213> Homo sapiens

<220>
<221> variation
<222> 81,147,186,204,222,228,276,279,330
<223> any nucleotide

<400> 36
auggcmwshc chuucgcyyu gcukauggub cusgukgubc uswshugyaa rwscwscugy      60

wsyyukggvu gygaycukcc ngarachcay wsycubgaya aymgvmgvac byusaugcus     120

yusgchcara ugwshmgvau mwscccnwsh wshugyyusa uggaymgvca ygayuuyggy     180

uuyccncagg argaguucga yggnaaycar uuycagaarg cncchgcnau mwscgudcus     240

caygarcuba uycagcagau yuuyaayyus uuyacnacna argaywshws ygcygchugg     300

gaygargayy ugyukgayaa guucugyacn garyusuayc arcarcusaa ygaycusgar     360

gcmugygusa ugcargarga rmgmgusggb garachcchy usaugaacgc hgaywshauh     420

cubgcmguka agaaruayuu ymgsmgvauy acycubuayy usachgagaa raaguaywsy     480

ccmugcgchu gggarguvgu kcgvgcygar aumaugmgvw scyuswshyu bwsmacyaay     540

cubcargarc gscubmgvmg vaargarura                                    570


<210> 37

<211> 189
<212> PRT
<213> Homo sapiens

<400> 37
Met Ala Ser Pro Phe Ala Leu Leu Met Val Leu Val Val Leu Ser Cys
1               5                   10                  15
Lys Ser Ser Cys Ser Leu Gly Cys Asp Leu Pro Glu Thr His Ser Leu
            20                  25                  30
Asp Asn Arg Arg Thr Leu Met Leu Leu Ala Gln Met Ser Arg Ile Ser
        35                  40                  45
Pro Ser Ser Cys Leu Met Asp Arg His Asp Phe Gly Phe Pro Gln Glu
    50                  55                  60
Glu Phe Asp Gly Asn Gln Phe Gln Lys Ala Pro Ala Ile Ser Val Leu
65                  70                  75                  80
His Glu Leu Ile Gln Gln Ile Phe Asn Leu Phe Thr Thr Lys Asp Ser
                85                  90                  95
Ser Ala Ala Trp Asp Glu Asp Leu Leu Asp Lys Phe Cys Thr Glu Leu
            100                 105                 110
Tyr Gln Gln Leu Asn Asp Leu Glu Ala Cys Val Met Gln Glu Glu Arg
        115                 120                 125
Val Gly Glu Thr Pro Leu Met Asn Ala Asp Ser Ile Leu Ala Val Lys
    130                 135                 140
Lys Tyr Phe Arg Arg Ile Thr Leu Tyr Leu Thr Glu Lys Lys Tyr Ser
145                 150                 155                 160
Pro Cys Ala Trp Glu Val Val Arg Ala Glu Ile Met Arg Ser Leu Ser
                165                 170                 175
Leu Ser Thr Asn Leu Gln Glu Arg Leu Arg Arg Lys Glu
            180                 185

<210> 38
<211> 15
<212> RNA
<213> Homo sapiens

<220>
<221> variation
<222> 5,10
<223> any nucleotide

<400> 38
yccancccwn ucycc                                                        15


<210> 39
<211> 19
<212> DNA
<213> Homo sapiens

<220>
<223> /PCR_primers="fwd_huRPL4"

<400> 39
agcgtggctg tctcctctc                                                    19


<210> 40
<211> 20
<212> DNA
<213> Homo sapiens

<220>
<223> /PCR_primers="rev_huRPL4"

<400> 40
gagccttgaa tacagcaggc                                                    20


<210> 41
<211> 21
<212> DNA
<213> Homo sapiens

<220>
<223> /PCR_primers="fwd_hu_IFNA2_v2"

<400> 41
gcttgggatg agaccctcct a                                                  21


<210> 42
<211> 19
<212> DNA
<213> Homo sapiens

<220>
<223> /PCR_primers="rev_huIFNA2_v2"

<400> 42
   cccaccccct gtatcacac                                                    19


<210> 43
<211> 20
<212> DNA
<213> Homo sapiens

<220>
<223> /PCR_primers="fwd_huIFNA2_ACC1"

<400> 43
cacgagatga tccagcagat                                                    20


<210> 44
<211> 20
<212> DNA
<213> Homo sapiens

<220>
<223> /PCR_primers="rev_huIFNA2_ACC1"

<400> 44
cttgtccagc agtgtctcgt                                                    20


<210> 45
<211> 20
<212> DNA
<213> Homo sapiens

<220>
<223> /PCR_primers="fwd_huIFNA2_AMP_humod"

<400> 45
ctgctctgtt ggctgtgatt                                                    20

<210> 46
<211> 20
<212> DNA
<213> Homo sapiens

<220>
<223> /PCR_primers="rev_huIFNA2_AMP_humod"

<400> 46
caggcatgag aacaggctaa                                                    20


<210> 47
<211> 20
<212> DNA
<213> Homo sapiens

<220>
<223> /PCR_primers="fwd_huIFNA2_AMP_AU"

<400> 47
tgatgcttct tgcacaaatg                                                    20


<210> 48
<211> 20
<212> DNA
<213> Homo sapiens

<220>
<223> /PCR_primers="rev_huIFNA2_AMP_AU"

<400> 48
aggacaggaa tggtttcagc                                                    20


<210> 49
<211> 20
<212> DNA
<213> Homo sapiens

<220>
<223> /PCR_primers="fwd_huIFNA2_AMP_GC"

<400> 49
caaggagtcg gagtgactga                                                    20


<210> 50
<211> 20
<212> DNA
<213> Homo sapiens

<220>
<223> /PCR_primers="rev_huIFNA2_AMP_GC"

<400> 50
cagggtgatc ctctggaagt                                                    20


<210> 51

<211> 20
<212> DNA
<213> Mus musculus

<220>
<223> /PCR_primers="fwd_muACTB"

<400> 51
ggctgtattc ccctccatcg                                                    20


<210> 52
<211> 22
<212> DNA
<213> Mus musculus

<220>
<223> /PCR_primers="rev_muACTB"

<400> 52
ccagttggta acaatgccat gt                                                 22


<210> 53
<211> 948
<212> RNA
<213> Homo sapiens

<400> 53
gggagaugag ccuaaaccuu aggcucaccc auuucaacca gucuagcagc aucugcaaca      60

ucuacaaugg ccuugaccuu ugcuuuacug guggcccucc uggugcucag cugcaaguca     120

agcugcucug ugggcuguga ucugccucaa acccacagcc uggguagcag gaggaccuug     180

augcuccugg cacagaugag gaaaaucucu cuuuucuccu gcuugaagga cagacaugac     240

uuuggauuuc cccaggagga guuuggcaac caguuccaaa aggcugaaac caucccuguc     300

cuccaugaga ugauccagca gaucuucaau cucuucagca caaaggacuc aucugcugcu     360

ugggaugaga cccuccuaga caaauucuac acugaacucu accagcagcu gaaugaccug     420

gaagccugug ugauacaggg ggugggggug acagagacuc cccugaugaa ggaggacucc     480

auucuggcug ugaggaaaua cuuccaaaga aucacucucu aucugaaaga gaagaaauac     540

agcccuugug ccugggaggu ugucagagca gaaaucauga gaucuuuuuc uuugucaaca     600

aacuugcaag aaaguuuaag aaguaaggaa ugaaaacugg uucaacaugg aaaugauuuu     660

cauuaauucg uaugccagcu caccuuuuua ugaucugcca uuucaaagac ucauguuucu     720

gcuaugacca ugacacgauu uaaaucuuuu ucaaauguuu uuaggaguau uaaucaacau     780

uguauucagc ucuuaaggca cuagucccuu acagaggacc augcugacug auccauuauc     840

uauuuaaaua uuuuuaaaau auuauuuauu uaacuauuua uaaaacaacu uauuuuuguu     900

cauauuacgu caugugcacc uuugcacagu gguuaaugua auaaaaua                  948